(19) **Europäisches Patentamt / European Patent Office / Office européen des brevets**

(11) **EP 4 238 647 A1**

(12) **EUROPEAN PATENT APPLICATION**
published in accordance with Art. 153(4) EPC

(43) Date of publication:
**06.09.2023 Bulletin 2023/36**

(21) Application number: **21885131.9**

(22) Date of filing: **26.10.2021**

(51) International Patent Classification (IPC):
*B01J 23/75* (2006.01)     *B01J 23/755* (2006.01)
*B01J 23/78* (2006.01)     *B01J 23/80* (2006.01)
*B01J 23/83* (2006.01)     *C07C 211/03* (2006.01)
*C07C 209/04* (2006.01)

(52) Cooperative Patent Classification (CPC):
**B01J 23/75; B01J 23/755; B01J 23/78;
B01J 23/80; B01J 23/83; C07C 209/04;
C07C 211/03**

(86) International application number:
**PCT/CN2021/126326**

(87) International publication number:
**WO 2022/089404 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  30.10.2020  CN 202011187657
         30.10.2020  CN 202011192987
         30.10.2020  CN 202011187667
         30.10.2020  CN 202011188178

(71) Applicants:
• **China Petroleum & Chemical Corporation
  Beijing 100728 (CN)**
• **BEIJING RESEARCH INSTITUTE OF CHEMICAL
  INDUSTRY,
  CHINA PETROLEUM & CHEMICAL
  CORPORATION
  Chaoyang District
  Beijing 100013 (CN)**

(72) Inventors:
• **TIAN, Baoliang
  Beijing 100013 (CN)**

• **WANG, Guoqing
  Beijing 100013 (CN)**
• **PENG, Hui
  Beijing 100013 (CN)**
• **TANG, Guoqi
  Beijing 100013 (CN)**
• **XIANG, Liangyu
  Beijing 100013 (CN)**
• **YANG, Yi
  Beijing 100013 (CN)**
• **ZHANG, Lijun
  Beijing 100013 (CN)**
• **SONG, Chao
  Beijing 100013 (CN)**
• **ZHANG, Xiaorong
  Beijing 100013 (CN)**
• **CHEN, Song
  Beijing 100013 (CN)**
• **LIU, Jing
  Beijing 100013 (CN)**

(74) Representative: **Hoffmann Eitle
  Patent- und Rechtsanwälte PartmbB
  Arabellastraße 30
  81925 München (DE)**

(54) **AMINATION CATALYST AND PREPARATION AND USE THEREOF**

(57)     Disclosed are a catalyst useful for producing organic amines by catalytic amination its preparation and application thereof, which catalyst comprising an inorganic porous carrier containing aluminum and/or silicon, and an active metal component supported on the carrier, the active metal component comprising at least one metal selected from Group VIII and Group IB metals, wherein the carrier has an L acid content of 85% or more relative to the total of the L acid and B acid contents. The catalyst shows an improved catalytic performance when used for producing organic amines by catalytic amination.

## Description

## Technical Field

The present application relates to the field of amination reaction, particularly to a catalyst useful for producing organic amines by catalytic amination, its preparation and application thereof.

## Background Art

Amines are very important industrial organic compounds and are widely used in various fields, for example, as solvents, medical intermediates, resin raw materials, textile additives, insecticides, rubber stabilizers, resists, and also in cleaning and plastics processing. The three main processes for producing amines are the hydroamination of carbonyl compounds, the hydroamination of alcohols and the hydrogenation of nitriles. The hydroamination of carbonyl compounds is, for example, the reaction of acetone, hydrogen and ammonia to form isopropylamine. The hydroamination of alcohols includes, for example, the hydroamination of ethanol with ammonia in the presence of hydrogen to form ethylamine, the hydroamination of isopropanol with ammonia in the presence of hydrogen to form isopropylamine, the hydroamination of butanol with ammonia in the presence of hydrogen to form butylamine, and the hydroamination of hexanediol with ammonia in the presence of hydrogen to form hexanediamine, etc. Nitrile hydrogenation is, for example, the hydrogenation of acetonitrile to ethylamine and the hydrogenation of adiponitrile to hexanediamine.

Chinese patent application No. CN102658162A discloses a catalyst for synthesizing ethyleneamine and a process for producing ethyleneamine. The catalyst consists of three parts, namely a main active component, an auxiliary agent and an aminated carrier, wherein the main active component is one or more selected from Ni and Co and accounts for 1-40% of the total weight of the catalyst, and the auxiliary agent is one or more selected from the group consisting of Fe, Cu, Ru, Re, K, Zn and B and oxides thereof, and accounts for 0.1-20% of the total weight of the catalyst; the aminated carrier is obtained by amination of one or more carriers selected from the group consisting of $SiO_2$ and $Al_2O_3$, and the amination treatment comprises: contacting the carrier with an ammonia source at a temperature of 150 to 400 °C for 0.5 to 15 hours. The inventors of the present application have found that the carrier material has a close relationship with the activity of the catalyst, since a large amount of hydroxyl groups exist on the surface of the carrier $SiO_2$ or $Al_2O_3$, the surface of the carrier is present in an acidic environment, which promotes the polymerization of the intermediate product imine, where the carrier in the catalyst is aminated, a large amount of hydroxyl groups on the surface of the carrier will be converted into amine groups, so that the carrier shows an alkaline environment, the possibility of imine polymerization is reduced, and the activity, selectivity and stability of the catalyst are improved.

It is typically acknowledged in prior arts that, where the catalyst for producing amine by amination of alcohols has alkalinity, it is more favorable for improving the activity and selectivity of the catalyst, and the activity of existing catalysts for amination reaction has great promotion space.

## Summary of the Invention

It is an object of the present application to provide a catalyst useful for producing organic amines by catalytic amination, its preparation and application thereof, which catalyst shows improved performance when used for amination reaction, such as at least one of improved catalytic activity, improved reaction conversion, improved product selectivity and improved catalyst stability.

To achieve the above object, in one aspect, the present application provides a catalyst useful for producing organic amines by catalytic amination, comprising an inorganic porous carrier containing aluminum and/or silicon, and an active metal component supported on the carrier, the active metal component comprising at least one metal selected from Group VIII and Group IB metals, wherein the carrier has an L acid content of 85% or more relative to the total of the L acid and B acid contents.

Preferably, the carrier comprises a matrix and a doping element, wherein the matrix is selected from alumina, silica, molecular sieves, diatomite, aluminosilicates, or combinations thereof, and the doping element is a non-metallic element.

Preferably, the catalyst further comprises a metal promoter supported on the carrier, and the metal promoter comprises at least one metal selected from the group consisting of Group VIB, Group VIIB, Group IB, Group IIB and lanthanide series elements, or a combination of at least one Group IIA metal, at least one Group IIB metal and at least one Group VA metal.

In another aspect, there is provided a method for producing the catalyst of the present application, comprising the steps of:

1) providing an inorganic porous carrier containing aluminium and/or silicon, which carrier has an L acid content of 85% or more relative to the total of the L acid and B acid contents;

2) loading the active metal component and optionally the metal promoter on the carrier; and

3) carrying out a heat treatment and optionally a reduction treatment on the material obtained in step 2) to obtain the catalyst.

In yet another aspect, the present application provides a process for producing organic amines, comprising: contacting an amination raw material and an amination reagent with the catalyst according to the present application for amination reaction in the presence of hydrogen to obtain an organic amine, wherein the amination raw material is selected from the group consisting of alcohols, ketones, alcohol amines, aldehydes and combinations thereof; and the amination reagent is selected from the group consisting of ammonia, primary amines, secondary amines, and combinations thereof.

The catalyst of the present application shows an improved performance, particularly improved catalytic activity, reaction conversion, product selectivity and/or catalyst stability, when used for producing organic amines by catalytic amination.

Other characteristics and advantages of the present application will be described in detail in the detailed description hereinbelow.

## Detailed Description of the Invention

The present application will be further described hereinafter in detail with reference to specific embodiments thereof. It should be noted that the specific embodiments of the present application are provided for illustration purpose only, and are not intended to be limiting in any manner.

Any specific numerical value, including the endpoints of a numerical range, described in the context of the present application is not restricted to the exact value thereof, but should be interpreted to further encompass all values close to said exact value, for example all values within $\pm 5\%$ of said exact value. Moreover, regarding any numerical range described herein, arbitrary combinations can be made between the endpoints of the range, between each endpoint and any specific value within the range, or between any two specific values within the range, to provide one or more new numerical range(s), where said new numerical range(s) should also be deemed to have been specifically described in the present application.

Unless otherwise stated, the terms used herein have the same meaning as commonly understood by one skilled in the art; and if the terms are defined herein and their definitions are different from the ordinary understanding in the art, the definition provided herein shall prevail.

In the present application, the ratio of the L acid content of the catalyst carrier to the total of the L acid and B acid contents is measured by pyridine probe adsorption spectrometry.

In the present application, the ammonia adsorption capacity of the carrier and the catalyst is measured by $NH_3$-TPD test, wherein the ammonia adsorption capacity is expressed as the measured ammonia desorption amount.

In the present application, the specific surface area, pore volume and proportion of pores having different pore diameters of the carrier are measured by a nitrogen adsorption-desorption method according to GB/T6609.35-2009.

In the present application, the expression "C2-20" means having 2 to 20 carbon atoms, for example having 2, 3, 4, 5, 6, 7, 8, 9, 10, 11, 12, 13, 14, 15, 16, 17, 18, 19 or 20 carbon atoms. Similarly, the expression "C1-12" means having 1-12 carbon atoms.

In the present application, the grain sizes of the active metal component and the metal promoter are measured by XRD analysis.

In the present application, the isoelectric point of the carrier is measured by means of a particle size potentiometer.

In the present application, unless otherwise indicated, the pressures given are gauge pressures.

In the context of the present application, in addition to those matters explicitly stated, any matter or matters not mentioned are considered to be the same as those known in the art without any change. Moreover, any of the embodiments described herein can be freely combined with another one or more embodiments described herein, and the technical solutions or ideas thus obtained are considered as part of the original disclosure or original description of the present application, and should not be considered to be a new matter that has not been disclosed or anticipated herein, unless it is clear to one skilled in the art that such a combination is obviously unreasonable.

All of the patent and non-patent documents cited herein, including but not limited to textbooks and journal articles, are hereby incorporated by reference in their entirety.

As mentioned above, in a first aspect, the present application provides a catalyst useful for producing organic amines by catalytic amination, comprising an inorganic porous carrier containing aluminium and/or silicon and an active metal component supported on the carrier, the active metal component comprising at least one metal selected from Group VIII and Group IB metals, wherein the carrier has an L acid content of 85% or more relative to the total of the L and B acid contents.

According to the present application, the Group VIII metal may be, for example, cobalt, nickel or palladium, and the Group IB metal may be, for example, copper. In a preferred embodiment, the metal in the active metal component is selected from cobalt, nickel, palladium, copper or a combination thereof, more preferably selected from cobalt, nickel or a combination thereof.

In the catalyst of the present application, the Group IB metal, such as copper, can be used alone as the active metal component, in which case it is typically used in a relatively larger amount; it may also be used in combination with a Group VIII metal, in which case it is typically used in a relatively smaller amount. When used in combination with a Group VIII metal, such as cobalt, nickel and palladium, the Group IB metal is normally referred to herein as a metal promoter.

In a preferred embodiment, the L acid content of the carrier is 88% or more, more preferably 90% or more, and particularly preferably 92% or more, relative to the total of the L acid and B acid contents.

In a preferred embodiment, the carrier comprises a matrix and a doping element, wherein the matrix is selected from alumina, silica, molecular sieves, diatomite, aluminosilicates or combinations thereof, and the doping element is a non-metallic element, preferably at least one selected from the group consisting of Group IIIA, Group VA, Group VIA and Group VIIA non-metallic elements, excluding chlorine, more preferably at least one selected from the group consisting of boron, fluorine, phosphorus, sulfur and selenium. Further preferably, the doping element in the carrier is derived from a non-metallic acid radical ion, and the non-metallic acid radical ion is preferably at least one selected from the group consisting of borate ion, fluoride ion, phosphate ion, sulfate ion and selenate ion.

In a preferred embodiment, the carrier has at least one of the following characteristics:

the proportion of the pore volume of pores having a pore diameter in a range of 7-27 nm to the pore volume of the carrier is greater than 65%, preferably 70-90%, and the proportion of the pore volume of pores having a pore diameter of less than 7 nm to the pore volume of the carrier is 0-10%, preferably 0-8%, and where the carrier has the above-described pore diameter distribution, it is favorable to increase the surface diffusivity of the catalyst and improve the activity and the product selectivity of the catalyst;

the proportion of the pore volume of pores having a pore diameter of less than 7.5nm to the pore volume of the carrier is less than 20%, preferably 5-17%, the proportion of the pore volume of pores having a pore diameter of less than 9 nm to the pore volume of the carrier is less than 40%, the proportion of the pore volume of pores having a pore diameter of greater than 27nm to the pore volume of the carrier is less than 5%, preferably 0.5-5%, preferably, the proportion of the pore volume of pores having a pore diameter of more than or equal to 7.5nm and less than 9nm to the pore volume of the carrier is 5-17%, and the proportion of the pore volume of pores having a pore diameter of more than or equal to 9nm and less than or equal to 27nm to the pore volume of the carrier is 61-89.5%, and where the carrier has the above-described pore diameter distribution, it is favorable to increase the surface diffusivity of the catalyst and improve the activity and the product selectivity of the catalyst;

the carrier has an ammonia adsorption capacity of 0.25-0.65 mmol/g, preferably 0.3-0.6 mmol/g, and more preferably 0.3-0.5 mmol/g;

the content of alumina in the carrier is 65 wt% or more, preferably 70 wt% or more, more preferably 75 wt% or more, based on the total amount of the matrix;

the doping element is present in an amount of 0.05 to 6 wt%, preferably 0.05 to 5 wt%, more preferably 0.05 to 4.5 wt%, particularly preferably 0.07 to 4 wt%, for example 0.08 to 4 wt% or 0.1 to 3 wt% (e.g., may be 0.1 wt%, 0.5 wt%, 1 wt%, 1.5 wt%, 2 wt%, 2.5 wt%, 3 wt%, or a value between any two of them), relative to the total weight of the matrix;

the carrier has a specific surface area of 100-220 $m^2/g$, preferably 105-210 $m^2/g$, more preferably 110-210 $m^2/g$, and particularly preferably 120-210 $m^2/g$;

the carrier has a pore volume of 0.4-1.1 ml/g, preferably 0.43-1.1 ml/g, more preferably 0.45-1.1 ml/g, and particularly preferably 0.45-1 ml/g; and

the isoelectric point of the carrier is 3-6, preferably 3.5-5.5.

In a preferred embodiment of the catalyst of the present application, the active metal component is present in an amount of 5 to 45 g, preferably 8 to 44 g, more preferably 10 to 38 g, and particularly preferably 15 to 37 g, per 100g of the matrix.

In a preferred embodiment, the active metal component has a grain size of less than 10nm, more preferably from 3 to 8nm, which can be well matched to the properties of the carrier, so that a better catalytic activity and product selectivity can be achieved.

In a preferred embodiment, the catalyst further comprises a metal promoter supported on the carrier, and the metal promoter comprises at least one metal selected from the group consisting of Group VIB, Group VIIB, Group IB, Group IIB and lanthanide series elements, preferably at least one metal selected from the group consisting of Cr, Mo, W, Mn, Re, Cu, Ag, Au, Zn, La and Ce; further preferably, the metal promoter is present in an amount of 0 to 10g, preferably 0.1 to 10g, more preferably 0.5 to 8 g, per 100 g of the matrix.

In some further preferred embodiments, the metal promoter comprises a combination of at least one Group VIIB metal and at least one Group IB metal, wherein the weight ratio of the Group VIIB metal to the Group IB metal, calculated as metal element, is 0.05-15 : 1, preferably 0.1-12 : 1; or the metal promoter comprises a combination of at least one Group VIIB metal and at least one Group IIB metal, wherein the weight ratio of the Group VIIB metal to the Group IIB metal is 0.2-20 : 1, preferably 0.3-6 : 1, calculated as metal element; or the metal promoter comprises a combination of at least one Group VIB metal, at least one Group IB metal and at least one Group IIB metal, wherein the weight ratio of the Group VIB metal, the Group IB metal and the Group IIB metal is 0.1-10 : 0.1-10 : 1, preferably 0.2-8 : 0.2-8 : 1, calculated as metal element. Particularly preferably, the Group VIIB metal is selected from manganese and/or rhenium, the Group IB metal is at least one selected from the group consisting of copper, silver and gold, the Group IIB metal is selected from zinc, and the Group VIB metal is selected from molybdenum and/or tungsten.

In other preferred embodiments, the catalyst further comprises a metal promoter supported on the carrier, the metal promoter is a combination of at least one Group IIA metal, at least one Group IIB metal, and at least one Group VA metal, further preferably the metal promoter is present in an amount of 0.1 to 10g, preferably 0.5 to 6g, per 100 g of the matrix. Still more preferably, the weight ratio of the Group IIA metal, the Group IIB metal and the Group VA metal in the metal promoter is 0.1-10 : 0.1-10 : 1, preferably 0.2-8 : 0.2-8 : 1. Particularly preferably, the Group IIA metal is at least one selected from the group consisting of magnesium, calcium and barium, the Group IIB metal is selected from zinc, and/or the Group VA metal is selected from bismuth.

According to the present application, the carrier for the catalyst can be obtained by methods known in the art useful for producing carriers having the above-mentioned properties, to which there is no particular limitation in the present application. Preferably, the carrier can be prepared by a method comprising the following steps: sequentially shaping, drying and calcining a mixture comprising the doping element and a matrix or a precursor thereof to obtain the carrier, wherein the matrix is selected from alumina, silica, molecular sieves, diatomite, aluminosilicates or combinations thereof. The molecular sieve may be, for example, a ZSM-5 or ZSM-11 molecular sieve. Where a precursor of the matrix is used, the precursor of alumina may be pseudo-boehmite, and the precursor of silica may be silicic acid, orthosilicic acid, or silica gel.

In the above method for producing the carrier, the precursor of the matrix is preferably pseudo-boehmite. The pseudo-boehmite may be prepared by at least one of carbonization method, organoaluminum hydrolysis method, aluminum sulfate method, and nitric acid method. The specific surface area of the pseudo-boehmite is preferably 250-400 $m^2/g$, preferably 255-360 $m^2/g$, more preferably 255-340 $m^2/g$, and particularly preferably 260-330 $m^2/g$; the pseudo-boehmite preferably has a pore volume of 0.5 to 1.3 ml, preferably 0.75 to 1.25 ml/g, more preferably 0.78 to 1.2 ml/g, particularly preferably 0.78 to 1.1 ml/g. A catalyst with better performance can be obtained by using the pseudo-boehmite with a specific pore structure.

In the above method for producing the carrier, where the raw material providing the precursor of the matrix already contains a desired amount of the doping element, the shaping may be simply performed using such raw material, and where the raw material providing the precursor of the matrix does not contain the doping element or the content of the doping element is low (insufficient), an introduction of additional doping element may be performed.

In the above method for producing the carrier, the doping element may be provided using a carrier modifier comprising at least one compound capable of providing a non-metallic acid radical ion, for example, an inorganic acid and/or an inorganic salt comprising a non-metallic acid radical, the non-metallic acid radical ion is preferably at least one selected from borate ion, fluoride ion, phosphate ion, sulfate ion, and selenate ion. Further preferably, the carrier modifier is at least one selected from the group consisting of boric acid, nickel borate, cobalt borate, potassium borate, ammonium borate, magnesium borate, potassium fluoride, magnesium fluoride, cobalt fluoride, nickel fluoride, hydrofluoric acid, ammonium fluoride, phosphoric acid, aluminum phosphate, tripotassium phosphate, potassium dihydrogen phosphate, potassium hydrogen phosphate, magnesium phosphate, calcium phosphate, ammonium phosphate, sulfuric acid, cobalt sulfate, nickel sulfate, aluminum sulfate, calcium sulfate, potassium sulfate, magnesium sulfate, strontium phosphate, strontium sulfate, and selenic acid.

In the above method for producing the carrier, the method for shaping may be selected from kneading, rolling, flaking, or the like.

In the above method for producing the carrier, the carrier modifier is used in such an amount that the content of the doping element is 0.05 to 6 wt%, preferably 0.05 to 5 wt%, more preferably 0.05 to 4.5 wt%, particularly preferably 0.07 to 4 wt%, for example 0.08 to 4 wt%, relative to the total weight of the matrix. One skilled in the art can determine the amount of the raw material (e.g., the carrier modifier) for a component based on the amount of said component in the final carrier, and therefore, the amounts of some raw materials are not given herein.

In the above method for producing the carrier, the drying conditions may include: a temperature of 80-150 °C (e.g., 80 °C, 85 °C, 90 °C, 95 °C, 100 °C, 110 °C, 115 °C, 120 °C, 125 °C, 130 °C, 140 °C, 150 °C, or a value between any two of them), preferably 85-130 °C, and a time of 6-20h (e.g., 6h, 7 h, 7.5 h, 8 h, 8.5 h, 9 h, 10 h, 12 h, 15 h, 18 h, 20h, or a value between any two of them), preferably 10-20 h.

In the above method for producing the carrier, the calcining conditions may include: a temperature of 500-1120 °C, such as 500-650 °C, preferably 700-1100 °C, more preferably 800-1050 °C (e.g. 800 °C, 850 °C, 860 °C, 870 °C, 880 °C, 890 °C, 900 °C, 920 °C, 950 °C, 960 °C, 980 °C, 1000 °C, 1050 °C, or a value between any two of them), and a time of 2-20h (e.g. 2 h, 3 h, 3.5 h, 4 h, 4.5 h, 5 h, 6 h, 7 h, 8 h, 10 h, 15 h, 20h, or a value of any two of them).

The catalyst of the present application can be used after reduction, for example, it can be reduced by using a hydrogen-containing gas at 350-500 °C, preferably at 350-450 °C. The hydrogen-containing gas may be pure hydrogen or hydrogen diluted with an inert gas, such as a mixture of nitrogen and hydrogen. The reduction temperature is gradually increased during the reduction, and the temperature rise is preferably not too fast, for example, not more than 20 °C per hour. The reduction time can be determined by monitoring the generation of $H_2O$ in the reduction system, that is when no new $H_2O$ is generated in the reduction system, the reduction is terminated, and one skilled in the art can select the reduction time accordingly, of which the detailed description is omitted herein for brevity, for example, the reduction time can be 2-5h at the highest temperature. The reduction may be carried out directly in the reactor, followed by the catalytic reaction. It is also possible to carry out the reduction in a separate reactor, also referred to as out-of-reactor reduction, and a passivation may be carried out after the reduction with a gas mixture comprising oxygen before the catalyst being discharged from the reactor, the passivation temperature being, for example, from 10 to 60 °C and particularly from 20 to 40 °C. The catalyst undergone the out-of-reactor reduction and passivation can be activated before use using hydrogen or a mixture of hydrogen and nitrogen at, for example, 150 °C to 250 °C, preferably 170 °C to 200 °C. The activation time can be determined by monitoring the generation of $H_2O$ in the activation system, that is when no new $H_2O$ is generated in the activation system, the activation is terminated and one skilled in the art can select the activation time accordingly, of which the detailed description is omitted herein for brevity. For example, the activation time at the highest temperature may be, for example, 1 to 5 hours, preferably 2 to 3 hours, or the catalyst can be used without activation, depending on the extent to which the active metal component and metal promoter of the catalyst have been oxidized.

In a second aspect, there is provided a method for producing the catalyst of the present application, comprising the steps of:

1) providing an inorganic porous carrier containing aluminium and/or silicon, which carrier has an L acid content of 85% or more relative to the total of the L acid and B acid contents;
2) loading the active metal component and optionally the metal promoter on the carrier; and
3) carrying out a heat treatment and optionally a reduction treatment on the material obtained in step 2) to obtain the catalyst,

In a preferred embodiment, the L acid content of the carrier is 88% or more, more preferably 90% or more, and particularly preferably 92% or more, relative to the total of the L acid and B acid contents.

In a preferred embodiment, said "providing an inorganic porous carrier containing aluminum and/or silicon" of step 1) comprises subjecting a mixture comprising a doping element and a matrix or a precursor thereof to shaping, drying and calcining sequentially to obtain the carrier, wherein the matrix is selected from alumina, silica, molecular sieves, diatomite, aluminosilicates or combinations thereof, preferably the alumina precursor is pseudo-boehmite having a specific surface area of 250-400 $m^2$/g, preferably 255-360 $m^2$/g, more preferably 255-340 $m^2$/g, particularly preferably 260-330 $m^2$/g, and a pore volume of 0.5-1.3 ml, preferably 0.75-1.25 ml/g, more preferably 0.78-1.2 ml/g, particularly preferably 0.78-1.1 ml/g; the doping element is a non-metallic element, preferably at least one selected from the group consisting of Group IIIA non-metallic elements, Group VA non-metallic elements, Group VIA non-metallic elements and Group VIIA non-metallic elements, excluding chlorine, preferably at least one selected from the group consisting of boron, fluorine, phosphorus, sulfur and selenium.

In a further preferred embodiment, the doping element is provided using a carrier modifier, and the doping element and the carrier modifier may be selected as described above in the first aspect, of which the detailed description is omitted herein for brevity. Still more preferably, the carrier modifier is used in such an amount that the resulting carrier has a doping element content of 0.05 to 6 wt%, preferably 0.05 to 5 wt%, more preferably 0.05 to 4.5 wt%, particularly preferably 0.07 to 4 wt%, e.g. 0.08 to 4 wt% or 0.1 to 3 wt% (e.g., may be 0.1 wt%, 0.5 wt%, 1 wt%, 1.5 wt%, 2 wt%, 2.5 wt%, 3 wt%, or a value between any two of them), relative to the total weight of the matrix.

In a further preferred embodiment, the method for shaping may be selected from kneading, rolling, flaking, or the like.

In a further preferred embodiment, the drying conditions of step 1) include: a temperature of 80-150 °C, and a drying time of 6-20 h; and/or the calcining conditions include: a temperature of 500-1120 °C, such as 500-650 °C, preferably 700-1100 °C, more preferably 800-1050 °C, and a calcining time of 2-20 h.

In a further preferred embodiment, the step 1) has the characteristics as described above for the method for producing the carrier in the first aspect, of which the detailed description is omitted herein for brevity.

In a preferred embodiment, said loading of step 2) comprises impregnating the carrier with a solution comprising a precursor of said active metal component and optionally a precursor of said metal promoter, preferably the impregnation solution has a pH in a range of 3.5-5.5. Controlling the pH of the impregnation solution within the above range can further improve the dispersibility of the active metal component.

According to the present application, the impregnation is carried out by immersing the carrier in an appropriate solution comprising precursors of said active metal component and metal promoter, so that the precursor is loaded onto the carrier by adsorption. The method for impregnation may be classified as dry impregnation, wet impregnation, multiple impregnation, mixed impregnation, spray impregnation and the like. The dry impregnation and wet impregnation respectively mean impregnating a carrier that is in a dry state or wetted with water in advance with the precursor of the active metal component. The multiple impregnation means impregnating with a mixed solution of the precursor(s) of one or more components in multiple times or impregnating in multiple times with different precursors, and in the multiple impregnation, drying and calcining need to be performed after each time of impregnation to "anchor" the impregnated components. The mixed impregnation means impregnating with the precursors of the active metal component and the metal promoter together, where no precipitation reaction would occur between those precursors. The spray impregnation means spraying an impregnation solution onto a continuously rotating carrier by a spray gun so that the impregnation solution just fills the pore volume of the carrier to saturation. These impregnation methods can be appropriately selected according to the actual conditions for the production of the catalyst of the present application.

In a preferred embodiment, the precursors of the active metal component and the metal promoter are soluble salts of the corresponding metals, such as nitrates, formates, oxalates, lactates, and the like. The solvent used to form the metal salt solution for impregnating the carrier is preferably water, although some organic solvents may be used, such as ethanol. The impregnation of the carrier with the metal salt solution can be carried out in any desired sequence, or continuously with a plurality of solutions containing one or more metal salts. All impregnation steps or a single impregnation step may be carried out in several stages, and the order of impregnation may also be varied. The concentration of the solution is selected so that a desired amount of metal is loaded onto the carrier.

According to the present application, the carrier loaded with the active metal component and optionally the metal promoter is subjected to a heat treatment in step 3), said heat treatment preferably comprising calcining or a combination of drying and calcining. For example, the heat treatment may comprise drying the impregnated carrier at 80 to 150 °C, more preferably 80 to 120 °C. The drying time can be appropriately selected according to the drying temperature, the amount of the material to be dried, the drying equipment and the like, and can be, for example, 6 to 20 hours, as long as the moisture content after drying does not affect the subsequent calcining. Further, the drying may be followed by calcining at 150-500 °C to remove the water of crystallization in the salt or to decompose the salt into oxides, preferably at 300-500 °C for 1-6 h. In the case of multiple impregnations, it is preferable to carry out drying and calcining after each impregnation.

In the present application, the loading of the active metal component and the metal promoter has no substantial impact on the microstructure of the catalyst, and therefore, the catalyst obtained has a similar pore structure to that of the carrier.

In a third aspect, the present application provides a carrier which is an inorganic porous material containing aluminium and/or silicon, wherein the carrier has an L acid content of 85% or more relative to the total of the L acid and B acid contents.

In a preferred embodiment, the L acid content of the carrier is 88% or more, more preferably 90% or more, and particularly preferably 92% or more, relative to the total of the L acid and B acid contents.

In a preferred embodiment, the carrier comprises a matrix and a doping element, wherein the matrix is selected from alumina, silica, molecular sieves, diatomite, aluminosilicates or combinations thereof, and the doping element is a non-metallic element, preferably at least one selected from the group consisting of Group IIIA, Group VA, Group VIA and Group VIIA non-metallic elements, excluding chlorine, more preferably at least one selected from the group consisting of boron, fluorine, phosphorus, sulfur and selenium. Further preferably, the doping element in the carrier is derived from a non-metallic acid radical ion, and the non-metallic acid radical ion is preferably at least one selected from the group consisting of borate ion, fluoride ion, phosphate ion, sulfate ion and selenate ion.

In a preferred embodiment, the carrier has at least one of the following characteristics:

the proportion of the pore volume of pores having a pore diameter in a range of 7-27 nm to the pore volume of the carrier is greater than 65%, preferably 70 to 90%, and the proportion of the pore volume of pores having a pore diameter less than 7 nm to the pore volume of the carrier is 0 to 10%, preferably 0 to 8%;
the proportion of the pore volume of pores having a pore diameter of less than 7.5nm to the pore volume of the carrier is less than 20%, preferably 5-17%, the proportion of the pore volume of pores having a pore diameter of less than 9nm to the pore volume of the carrier is less than 40%, the proportion of the pore volume of pores having a pore diameter of greater than 27nm to the pore volume of the carrier is less than 5%, preferably 0.5-5%, preferably, the proportion of the pore volume of pores having a pore diameter of more than or equal to 7.5nm and less than 9nm to the pore volume of the carrier is 5-17%, and the proportion of the pore volume of pores having a pore diameter of more than or equal to 9nm and less than or equal to 27nm to the pore volume of the carrier is 61-89.5%;
the carrier has an ammonia adsorption capacity of 0.25-0.65 mmol/g, preferably 0.3-0.6 mmol/g, and more preferably 0.3-0.5 mmol/g;
the content of alumina in the carrier is 65 wt% or more, preferably 70 wt% or more, more preferably 75 wt% or more, based on the total amount of the matrix;
the content of the doping element is 0.05 to 6 wt%, preferably 0.05 to 5 wt%, more preferably 0.05 to 4.5 wt%, particularly preferably 0.07 to 4 wt%, relative to the total amount of the matrix;
the carrier has a specific surface area of 100-220 m$^2$/g, preferably 105-210 m$^2$/g, more preferably 110-210 m$^2$/g, and particularly preferably 120-210 m$^2$/g;
the carrier has a pore volume of 0.4-1.1 ml/g, preferably 0.43-1.1 ml/g, more preferably 0.45-1.1 ml/g, and particularly preferably 0.45-1 ml/g; and
the isoelectric point of the carrier is 3-6, preferably 3.5-5.5.

In a fourth aspect, the present application provides the use of the catalyst according to the present application or the carrier according to the present application for producing organic amines by catalytic amination.

In a fifth aspect, the present application provides a process for producing organic amines, comprising: in the presence of hydrogen, contacting an amination raw material and an amination reagent with the catalyst according to the present application for amination reaction to obtain an organic amine.

In a preferred embodiment, the amination raw material is selected from the group consisting of alcohols, ketones, alcohol amines, aldehydes or combinations thereof, preferably selected from the group consisting of C2-20 alcohols, C3-20 ketones, C2-20 alcohol amines, C2-20 aldehydes and mixtures thereof. Further preferably, the amination raw material is selected from the group consisting of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, 1,12-dodecanediol, 1,12-dodecanedialdehyde, ethanolamine, propanolamine, isopropanolamine, 6-aminohexanol, diethanolamine, diisopropanolamine, dimethylethanolamine, acetone, ethylene glycol, 1,3-propanediol, and mixtures thereof.

In the present application, the amination reagent refers to a reactant capable of providing an amino group and/or an amine group. Preferably, the amination reagent is selected from the group consisting of ammonia, primary amines, secondary amines, and combinations thereof, preferably selected from the group consisting of ammonia, C1-12 primary amines, C2-12 secondary amines, and mixtures thereof, such as at least one of alkyl amine, cycloalkyl amine, and aralkyl amine, more preferably a C1-4 alkyl amine. Further preferably, the amination reagent is selected from the group consisting of ammonia, monomethylamine, dimethylamine, methyl ethyl amine, monoethylamine, diethylamine and mixtures thereof.

In a preferred embodiment, the amination conditions include: a molar ratio of hydrogen to the amination reagent and to the amination raw material of 1-6 : 2-35 : 1, preferably 1-6 : 2-33 : 1, more preferably 1-5 : 3-33 : 1, a temperature of 105-230 °C, preferably 110-220 °C, more preferably 110-210 °C, a pressure of 0.7-25 MPa, preferably 1-25 MPa, more preferably 1-22 MPa, particularly preferably 1-17 MPa, and a liquid phase volume space velocity of the amination raw material of 0.06-1 m$^3$/(m$^3$·h).

In some preferred embodiments, the amination raw material is a monohydric alcohol and the amination conditions include: a molar ratio of hydrogen to the amination reagent and to the amination raw material of preferably 1-4 : 2-9 : 1, more preferably 1-4 : 2-8 : 1, a temperature of 130-210 °C, preferably 130-208 °C, more preferably 130-200 °C, a pressure of 0.8-3.5 MPa, preferably 1-2.5 MPa, and a liquid phase volume space velocity of the amination raw material of 0.1-0.8 m$^3$/(m$^3$·h).

In some preferred embodiments, the amination raw material is a ketone or an aldehyde and the amination conditions include: a molar ratio of hydrogen to the amination reagent and to the amination raw material of 1-4 : 2-6 : 1, preferably 1-4 : 2-5 : 1, a temperature of 105-180 °C, preferably 110-170 °C, more preferably 110-160 °C, a pressure of 0.7-2.5 MPa, preferably 1-2.5 MPa, more preferably 1-2 MPa, and a liquid phase volume space velocity of the amination raw material of 0.1-1 m$^3$/(m$^3$ h), preferably 0.1-0.8 m$^3$/(m$^3$ h).

In some preferred embodiments, the amination raw material is an alcohol amine and the amination conditions include: a molar ratio of hydrogen to the amination reagent and to the amination raw material of 1-4 : 3-23 : 1, preferably 1-4 : 3-20 : 1, more preferably 1-4 : 3-10 : 1, a temperature of 130-200 °C, a pressure of 1-16 MPa, preferably 1-13 MPa, more preferably 1-11 MPa, and a liquid phase volume space velocity of the amination raw material of 0.06-0.8 m$^3$/(m$^3$·h).

In some preferred embodiments, the amination raw material is a dihydric alcohol and the amination conditions include: a molar ratio of hydrogen to the amination reagent and to the amination raw material of 0.3-5 : 2-35 : 1, preferably 1-4 : 3-35 : 1, more preferably 1-4 : 3-33 : 1, particularly preferably 1-4 : 3-32 : 1, a temperature of 130-230 °C, preferably 130-220 °C, more preferably 130-210 °C, a pressure of 1-25 MPa, preferably 1-22 MPa, more preferably 1-17 MPa, and a liquid phase volume space velocity of the amination raw material of 0.1-0.9 m$^3$/(m$^3$·h), preferably 0.1-0.8 m$^3$/(m$^3$·h).

In some preferred embodiments, the amination raw material is a mixture of 1,6-hexanediol, cycloheximide, and 6-amino-1-hexanol, and the amination conditions include: a molar ratio of hydrogen to the amination reagent and to the amination raw material of 0.3-4 : 3-35 : 1, preferably 1-4 : 3-33 : 1, more preferably 1-4 : 3-32 : 1, a temperature of 130-230 °C, preferably 130-220 °C, more preferably 130-210 °C, a pressure of 1-22 MPa, preferably 1-17 MPa, and a liquid phase volume space velocity of the amination raw material of 0.1-0.9 m$^3$/(m$^3$·h), preferably 0.1-0.8 m$^3$/(m$^3$·h).

First type of embodiments

In the first type of embodiments of the present application, there is provided a catalyst having a function of catalyzing the hydroamination of an alcohol to produce an organic amine, the catalyst comprising a carrier and an active metal component and optionally a metal promoter supported on the carrier, wherein the carrier comprises a matrix and a doping element, the matrix comprises alumina and optionally an additional carrier, wherein said additional carrier is at least one selected from the group consisting of silica, molecular sieve and diatomite; the proportion of the pore volume of pores having a pore diameter of less than 7.5 nm to the pore volume of the carrier is less than 20%, the proportion of the pore volume of pores having a pore diameter of less than 9 nm to the pore volume of the carrier is less than 40%, and the proportion of the pore volume of pores having a pore diameter of greater than 27nm to the pore volume of the carrier is less than 5%; the carrier has an ammonia adsorption capacity of 0.3-0.6 mmol/g; the carrier has an L acid content of 90% or more relative to the total of the L acid and B acid contents; the active metal component is cobalt and/or nickel.

The catalyst of the first type of embodiments of the present application has specific acidity and pore structure, and when used for alcohol hydroamination, the catalyst not only exhibits a high catalytic activity, but also has an excellent selectivity. When the catalyst is used for the hydroamination of 1,3-propanediol, the production of 3-aminopropanol and other impurities is less; when the catalyst is used for the hydroamination of ethanol, the production of methyl ethylamine, methyl diethylamine, ethyl-n-propylamine and ethyl-sec-butylamine is less; and when the catalyst is used for the hydroamination of 1,6-hexanediol, the production of heavy components and other impurities is less. After a long-period of life evaluation, it is found that the catalyst of the first type of embodiments of the present application has a stable catalytic performance, and by controlling the acidity of the catalyst within an appropriate range, the adsorption-desorption performance of the catalyst is also improved, the diffusion of the reaction system is further promoted, the reaction rate is accelerated, the carbon deposition is reduced, and the blockage of pore channel is retarded.

According to the first type of embodiments of the present application, the carrier is mainly composed of (doped) alumina, and may further comprise (doped) silica or the like, thereby further improving the properties of the catalyst, such as the type of pore channel structure and the stability of the pore structure. Preferably, the matrix in the carrier is selected from alumina doped with at least one of silica, molecular sieve and diatomite and non-doped alumina, and the content of the alumina carrier in the matrix is 65 wt% or more, preferably 75 wt% or more, based on the total amount of the alumina carrier and said additional carrier.

Preferably, the doping element is present in the carrier in an amount of 0.05 to 3 wt%, more preferably 0.08 to 2 wt%, and still more preferably 0.1 to 1.5 wt%, relative to the total weight of the matrix.

Preferably, the doping element in the carrier is derived from acid radical ions, excluding chloride ion. Since the doping element is introduced during the production of the carrier, the doping element is mainly present in the bulk phase of the carrier. The acid radical ion may be at least one selected from non-metallic acid radical ions, and more preferably at least one selected from the group consisting of borate ion, fluoride ion, phosphate ion, sulfate ion, and selenate ion. The doping element is preferably at least one selected from the group consisting of boron, fluorine, phosphorus, sulfur and selenium.

Preferably, the proportion of the pore volume of pores having a pore diameter of less than 7.5nm to the pore volume of the carrier is 5-17%, more preferably 5-10%, the proportion of the pore volume of pores having a pore diameter of more than or equal to 7.5nm and less than 9nm to the pore volume of the carrier is 5-17%, the proportion of the pore volume of pores having a pore diameter of more than or equal to 9nm and less than 27nm to the pore volume of the carrier is 61-89.5%, and the proportion of the pore volume of pores having a pore diameter of greater than 27nm to the pore volume of the carrier is 0.5-5%, more preferably 0.5-3%. The inventors of the present application have found that a catalyst having a pore channel structure satisfying the above requirements has better catalytic performance.

Preferably, the carrier has an ammonia adsorption capacity of 0.3 to 0.5 mmol/g.

Preferably, the carrier has an L acid content of 92 to 100%, preferably 96 to 100%, relative to the total of the L acid and B acid contents.

Preferably, the carrier has a specific surface area of 105-220 m$^2$/g.

Preferably, the carrier has a pore volume of 0.4 to 1.1 ml/g.

According to the first type of embodiments of the present application, the active metal component may be present in an amount of 5 to 42g, preferably 10 to 35 g, per 100g of the matrix.

According to the first type of embodiments of the present application, to better exert the performance of the catalyst, optimize the proportion of reaction products, and reduce unwanted side reactions, the catalyst may further comprise a metal promoter, which may be at least one selected from the group consisting of Group VIB, Group VIIB, Group IB, Group IIB, and lanthanide series elements, preferably at least one of Cr, Mo, W, Mn, Re, Cu, Ag, Au, Zn, La, and Ce. Preferably, the metal promoter may be present in an amount of 0 to 10g, preferably 0.5 to 6g, per 100 g of the matrix.

In the first type of embodiments of the present application, there is also provided a process for producing organic amines, comprising: contacting an amination raw material and an amination reagent with the catalyst as described above in the presence of hydrogen for amination reaction.

According to the first type of embodiments of the present application, the amination raw material or amination reagent can be selected as described above, of which the detailed description is omitted herein for brevity.

According to the first type of embodiments of the present application, the amination conditions may include: a mole ratio of hydrogen to the amination reagent and to the amination raw material of 1-5 : 2-35 : 1, a temperature of 110-220 °C, a pressure of 1-25 MPa, and a liquid phase volume space velocity of the amination raw material of 0.06-1 $m^3/(m^3 \cdot h)$.

Preferably, the amination raw material is a monohydric alcohol, and the amination conditions include: a mole ratio of hydrogen to the amination reagent and to the amination raw material of 1-4 : 2-8 : 1, a temperature of 130-200 °C, a pressure of 1-3.5 MPa, and a liquid phase volume space velocity of the amination raw material of 0.1-0.8 $m^3/(m^3 \cdot h)$.

Preferably, the amination raw material is a ketone or an aldehyde, and the amination conditions include: a mole ratio of hydrogen to the amination reagent and to the amination raw material of 1-4 : 2-5 : 1, a temperature of 110-180 °C, a pressure of 1-2.5 MPa, and a liquid phase volume space velocity of the amination raw material of 0.1-0.8 $m^3/(m^3 \cdot h)$.

Preferably, the amination raw material is an alcohol amine, and the amination conditions include: a mole ratio of hydrogen to the amination reagent and to the amination raw material of 1-4 : 3-20 : 1, a temperature of 130-200 °C, a pressure of 1-11 MPa, and a liquid phase volume space velocity of the amination raw material of 0.06-0.8 $m^3/(m^3 \cdot h)$.

Preferably, the amination raw material is a dihydric alcohol, and the amination conditions include: a mole ratio of hydrogen to the amination reagent and to the amination raw material of 1-5 : 2-35 : 1, a temperature of 130-220 °C, a pressure of 1-25 MPa, and a liquid phase volume space velocity of the amination raw material of 0.1-0.8 $m^3/(m^3 \cdot h)$.

Preferably, the amination raw material is a mixture of 1,6-hexanediol, hexamethyleneimine and 6-amino-1-hexanol (referred to as aminohexanol for short), and the amination conditions include: a mole ratio of hydrogen to the amination reagent and to the amination raw material of 1-4 : 3-35 : 1, a temperature of 130-200 °C, a pressure of 1-22 MPa, and a liquid phase volume space velocity of the amination raw material of 0.1-0.8 $m^3/(m^3 \cdot h)$.

Second type of embodiments

In the second type of embodiments of the present application, there is provided a catalyst having a function of catalyzing the production of amines from alcohols, the catalyst comprising a carrier and an active metal component and optionally a metal promoter supported on the carrier, the carrier being at least one selected from the group consisting of doped alumina, doped silica, doped molecular sieve and doped diatomite; the carrier has an ammonia adsorption capacity of 0.25-0.65 mmol/g; the carrier has an L acid content of 88% or more, relative to the total of the L acid and B acid contents; the active metal component is cobalt and/or nickel and the grain size of the active metal component in the catalyst is less than 10 nm.

The catalyst of the second type of embodiments of the present application has an improved carrier acidity, highly dispersed active metal components, and a grain size of less than 10 nm, and shows a higher catalytic activity and, at the same time, shows a higher selectivity, when used for the hydroamination of alcohols. When the catalyst is used in the hydroamination of ethanolamine, the production of components other than ethylenediamine, such as piperazine and the like, is less; and when the catalyst is used for the hydroamination of 1,6-hexanediol, the production of heavy components and other impurities is less. After a long-period of life evaluation, it is found that the catalyst of the second type of embodiments of the present application has a stable catalytic performance, and by controlling the acidity of the catalyst within an appropriate range, the adsorption-desorption performance of the catalyst is improved, so that the diffusion of the reaction system is promoted, the reaction rate is accelerated, the carbon deposition is reduced, and the blockage of pore channel is retarded.

According to the second type of embodiments of the present application, the carrier comprises a matrix selected from the group consisting of alumina, silica, molecular sieves, diatomite and the like, and a doping element. Preferably, the doping element is present in the carrier in an amount of 0.05 to 5 wt%, more preferably 0.08 to 4 wt%, relative to the total weight of the matrix.

Preferably, the doping element in the carrier is derived from acid radical ions, excluding chloride ion. The doping element is preferably at least one selected from the group consisting of boron, fluorine, phosphorus, sulfur and selenium. Since the doping element is introduced during the production of the carrier, said doping element is present in the bulk phase of the carrier. Further preferably, the acid radical ion may be at least one selected from the group consisting of non-metallic acid radical ions, such as at least one selected from the group consisting of borate ion, fluoride ion, phosphate ion, sulfate ion, and selenate ion.

Preferably, the carrier has an ammonia adsorption capacity of 0.3 to 0.5 mmol/g (e.g., 0.3 mmol/g, 0.32 mmol/g, 0.35 mmol/g, 0.38 mmol/g, 0.4 mmol/g, 0.45 mmol/g, 0.48 mmol/g, 0.5 mmol/g, or a value between any two of them).

Preferably, the carrier has an L acid content of 90-100%, relative to the total of the L acid and B acid contents.

Preferably, the active metal component of the catalyst has a grain size of from 3 to 8 nm.

Preferably, the carrier has a specific surface area of 100-200 $m^2$/g.

Preferably, the carrier has a pore volume of 0.45 to 1 ml/g.

Preferably, the carrier has an isoelectric point of 3 to 6, preferably 3.5 to 5.5.

Preferably, the content of the active metal component in the carrier may be 7 to 45 g, preferably 12 to 38 g, per 100g of the matrix.

According to the second type of embodiments of the present application, the catalyst may further comprise a metal promoter to better exert the performance of the catalyst, to optimize the proportion of reaction products, and to reduce unwanted side reactions. The metal promoter can be at least one selected from the group consisting of Group VIB, Group VIIB, Group IB, Group IIB and lanthanide series elements, preferably at least one of Cr, Mo, W, Mn, Re, Cu, Ag, Au, Zn, La and Ce. Preferably, the metal promoter may be present in the carrier in an amount of from 0 to 10g, preferably from 0.5 to 6g, per 100 g of the matrix. Further preferably, the grain size of the metal promoter is less than 10 nm.

In the second type of embodiments of the present application, there is also provided a process for producing organic amines, comprising: contacting an amination raw material and an amination reagent with the catalyst as described above in the presence of hydrogen for amination reaction.

According to the second type of embodiments of the present application, the amination raw material or amination reagent can be selected as described above, of which the detailed description is omitted herein for brevity.

According to the second type of embodiments of the present application, the amination conditions may include: a mole ratio of hydrogen to the amination reagent and to the amination raw material of 1-5 : 3-33 : 1, a temperature of 110-210 °C, a pressure of 1-22 MPa, and a liquid phase volume space velocity of the amination raw material of 0.06-1 $m^3$/(m$^3$·h).

Preferably, the amination raw material is a monohydric alcohol, and the amination conditions include: a mole ratio of hydrogen to the amination reagent and to the amination raw material of 1-4 : 2-8 : 1, a temperature of 130-200 °C, a pressure of 0.8-2.5 MPa, and a liquid phase volume space velocity of the amination raw material of 0.1-0.8 $m^3$/(m$^3$·h).

Preferably, the amination raw material is a ketone or an aldehyde, and the amination conditions include: a mole ratio of hydrogen to the amination reagent and to the amination raw material of 1-4 : 2-6 : 1, a temperature of 110-170 °C, a pressure of 1-2.5 MPa, and a liquid phase volume space velocity of the amination raw material of 0.1-0.8 $m^3$/(m$^3$·h).

Preferably, the amination raw material is an alcohol amine, and the amination conditions include: a mole ratio of hydrogen to the amination reagent and to the amination raw material of 1-4 : 3-10 : 1, a temperature of 130-200°C, a pressure of 1-11 MPa, and a liquid phase volume space velocity of the amination raw material of 0.06-0.8 $m^3$/(m$^3$·h).

Preferably, the amination raw material is a mixture of 1,6-hexanediol, hexamethyleneimine and 6-amino-1-hexanol (referred to as aminohexanol for short) or a dihydric alcohol, and the amination conditions include: a mole ratio of hydrogen to the amination reagent and to the amination raw material of 1-4 : 3-33 : 1, a temperature of 130-210°C, a pressure of 1-22 MPa, and a liquid phase volume space velocity of the amination raw material of 0.1-0.8 $m^3$/(m$^3$·h).

Third type of embodiments

In the third type of embodiments of the present application, there is provided a catalyst having a function of producing amines by hydroamination of alcohols, wherein the catalyst comprises a carrier and an active metal component and optionally a metal promoter supported on the carrier, the carrier comprises a matrix and a doping element, the matrix comprises alumina and optionally an additional carrier selected from silica and/or molecular sieves, the doping element is at least one selected from the group consisting of boron, fluorine, phosphorus, sulfur and selenium; the proportion of the pore volume of pores having a pore diameter in a range of 7-27 nm to the pore volume of the carrier is greater than 65%; the carrier has an L acid content of 85% or more, relative to the total of the L acid and B acid contents; the active metal component is cobalt and/or nickel.

By the doping of boron, fluorine, phosphorus, sulfur and selenium in the carrier, the catalyst according to the third type of embodiments of the present application shows an improved catalytic performance, for example, for the hydroamination of ethanol, and the production of methylethylamine, methyldiethylamine, ethyl-n-propylamine, ethyl-sec-butylamine is less. When the catalyst is used for the hydroamination of 1,6-hexanediol, the production of heavy components and other impurities is less, and the service life of the catalyst is prolonged.

In addition, the catalyst of the third type of embodiments of the present application has a specific pore channel structure, and when used for the hydroamination of alcohols, the catalyst exhibits a high catalytic activity, and has an excellent selectivity and stability, thereby reducing the carbon deposition in the pore channel and effectively preventing the blockage of the pore channel of the catalyst.

According to the third type of embodiments of the present application, the carrier is mainly composed of doped alumina, and may further comprise (doped) silica or the like, thereby further improving the pore channel structure and the acid-base properties of the carrier of the catalyst. Preferably, the matrix in the carrier is alumina doped with silica and/or molecular sieve, and the content of the alumina carrier in the matrix is not less than 70 wt%, preferably 75-100 wt%.

Preferably, the doping element is present in the carrier in an amount of 0.05 to 6 wt%, more preferably 0.08 to 4 wt%, relative to the total weight of the matrix.

Preferably, the doping element is incorporated in the carrier in the form of at least one selected from the group consisting of borate ion, fluoride ion, phosphate ion, sulfate ion and selenate ion. Since the doping element is introduced during the production of the carrier, the doping element is mainly present in the bulk phase of the carrier.

Preferably, the proportion of the pore volume of pores having a pore diameter in a range of 7-27 nm to the pore volume of the carrier is greater than 65%. More preferably, the proportion of the pore volume of pores having a pore diameter in a range of 7-27 nm to the pore volume of the carrier is 70 to 90%. Further preferably, the proportion of the pore volume of pores having a pore diameter of less than 7 nm to the pore volume of the carrier is 0-10%; the proportion of the pore volume of pores having a pore diameter of more than 27 nm to the pore volume of the carrier is 18-32%.

Preferably, the carrier has an L acid content of 85-98%, relative to the total of the L acid and B acid contents.

Preferably, the carrier has a specific surface area of 120-210 $m^2$/g.

Preferably, the carrier has a pore volume of 0.43 to 1.1 ml/g.

Preferably, the active metal component may be present in an amount of 8 to 44 g, preferably 12 to 37 g, per 100g of the matrix.

Preferably, the metal promoter may be present in an amount of 0 to 10g, preferably 0.5 to 6g, per 100 g of the matrix.

According to the third type of embodiments of the present application, the catalyst may further comprise a metal promoter to better exert the performance of the catalyst, to optimize the proportion of reaction products, and to reduce unwanted side reactions. The metal promoter can be at least one selected from the group consisting of Group VIB, Group VIIB, Group IB, Group IIB and lanthanide series elements, preferably at least one of Cr, Mo, W, Mn, Re, Cu, Ag, Au, Zn, La and Ce.

In the third type of embodiments of the present application, there is also provided a process for producing organic amines, comprising: contacting an amination raw material and an amination reagent with the catalyst as described above in the presence of hydrogen for amination reaction.

According to the third type of embodiments of the present application, the amination raw material and amination reagent can be selected as described above, of which the detailed description is omitted herein for brevity.

According to the third type of embodiments of the present application, the amination conditions may include: a mole ratio of hydrogen to the amination reagent and to the amination raw material of 1-6 : 2-32 : 1, a temperature of 105-210 °C, a pressure of 1-17 MPa, and a liquid phase volume space velocity of the amination raw material of 0.06-1 $m^3$/($m^3$·h).

Preferably, the amination raw material is a monohydric alcohol, and the amination conditions include: a mole ratio of hydrogen to the amination reagent and to the amination raw material of 1-4 : 2-9 : 1, a temperature of 130-208 °C, a pressure of 1-2.5 MPa, and a liquid phase volume space velocity of the amination raw material of 0.1-0.8 $m^3$/($m^3$·h).

Preferably, the amination raw material is a ketone or an aldehyde, and the amination conditions include: a mole ratio of hydrogen to the amination reagent and to the amination raw material of 1-4 : 2-5 : 1, a temperature of 105-160 °C, a pressure of 1-2 MPa, and a liquid phase volume space velocity of the amination raw material of 0.1-1 $m^3$/($m^3$·h).

Preferably, the amination raw material is an alcohol amine, and the amination conditions include: a mole ratio of hydrogen to the amination reagent and to the amination raw material of 1-4 : 3-20 : 1, a temperature of 130-200 °C, a pressure of 1-13 MPa, and a liquid phase volume space velocity of the amination raw material of 0.06-0.8 $m^3$/($m^3$·h).

Preferably, the amination raw material is a mixture of 1,6-hexanediol, hexamethyleneimine and 6-amino-1-hexanol (referred to as aminohexanol for short) or a dihydric alcohol, and the amination conditions include: a mole ratio of hydrogen to the amination reagent and to the amination raw material of 1-4 : 3-32 : 1, a temperature of 130-210 °C, a pressure of 1-17 MPa, and a liquid phase volume space velocity of the amination raw material of 0.1-0.9 $m^3$/($m^3$·h).

Fourth type of embodiments

In the fourth type of embodiments of the present application, there is provided a catalyst having a function of catalyzing the production of amines from alcohols, the catalyst comprising a carrier, and an active metal component and a metal promoter supported on the carrier, wherein the carrier has an L acid content of 85% or more, relative to the total of the L acid and B acid contents, and the active metal component is cobalt and/or nickel; the metal promoter is a combination of at least one of Group IIA metal, at least one Group IIB metal and at least one Group VA metal.

The catalyst of the fourth type of embodiments of the present application comprises a specific metal promoter, has a high catalytic activity, and at the same time, has a high selectivity and produces few by-products.

Preferably, the carrier has an L acid content of 88% or more, more preferably 90% or more, and particularly preferably 92% or more, relative to the total of the L acid and B acid contents.

Preferably, the carrier comprises a matrix and a doping element, the matrix comprises alumina and optionally an additional carrier comprising silica and/or molecular sieves; the doping element is at least one selected from the group consisting of boron, fluorine, phosphorus, sulfur and selenium; the proportion of the pore volume of pores having a pore diameter in a range of 7-27 nm to the pore volume of the carrier is greater than 65%.

According to the fourth type of embodiments of the present application, the carrier is mainly composed of doped alumina, and may further comprise (doped) silica and the like, thereby further improving the pore channel structure of the catalyst, thereby allowing an easy diffusion of reactants and products in the pore channel, and providing a more stable pore structure. Preferably, the alumina content of the matrix in the carrier is 70 wt% or more, preferably 75-100 wt%, based on the total amount of alumina and the additional carrier.

Preferably, the doping element is present in the carrier in an amount of 0.05 to 4.5 wt%, more preferably 0.07 to 2.8 wt%, relative to the total weight of the matrix.

Preferably, the doping element is incorporated in the carrier in the form of at least one selected from the group consisting of borate ion, fluoride ion, phosphate ion, sulfate ion and selenate ion. The doping element is present in the alumina precursor. Where the doping element is introduced during the preparation of the precursor, it will be wrapped in the crystal phase of the precursor, and where the doping element is introduced after the preparation of the carrier, it will be mainly present in the bulk phase of the carrier.

Preferably, the proportion of the pore volume of pores having a pore diameter in a range of 7-27 nm to the pore volume of the carrier is 70 to 90%. Preferably, the proportion of the pore volume of pores having a pore diameter less than 7 nm to the pore volume of the carrier is 0-8%. Preferably, the proportion of the pore volume of pores having a pore diameter of more than 27 nm to the pore volume of the carrier is 15-35%, more preferably 20-29%.

Preferably, the carrier has a specific surface area of 110-210 $m^2/g$.

Preferably, the carrier has a pore volume of 0.45 to 1.1 ml/g.

Preferably, the active metal component may be present in an amount of 8-45g, preferably 15-38g (e.g. may be any of 15, 20, 25, 28, 30, 32, 35, 37, 38, or a value between any two of them), per 100g of the matrix.

Preferably, the metal promoter may be present in an amount of 0.1-10g, preferably 0.5-6g (e.g. may be any of 0.5, 1, 2, 3, 3.5, 3.8, 4, 4.2, 4.5, 4.8, 5, 5.2, 5.5, 6, or a value between any two of them), per 100 g of the matrix.

According to the fourth type of embodiments of the present application, the catalyst may comprise a metal promoter as described above to better exert the performance of the catalyst, to optimize the proportion of reaction products, and to reduce unwanted side reactions. The weight ratio of the Group IIA metal to the Group IIB metal and to the Group VA group metal in the metal promoter is preferably 0.1-10 : 0.1-10 : 1, more preferably 0.2-8 : 0.2-8 : 1. Preferably, the Group IIA metal is at least one selected from the group consisting of magnesium, calcium and barium. Preferably, the Group IIB metal is selected from zinc. Preferably, the Group VA metal is selected from bismuth.

In the fourth type of embodiments of the present application, the use of a carrier having a specific pore structure and doping element allows the catalyst to show a high catalytic activity and, at the same time, show a higher selectivity, when used for the hydroamination of alcohols. When the catalyst is used for the hydroamination of ethanol, the production of methyl ethylamine, methyl diethylamine, ethyl-n-propylamine, ethyl-sec-butylamine is less. When the catalyst is used for the hydroamination of 1,6-hexanediol, the production of heavy components and other impurities is less. After a long-period of life evaluation, it is found that the catalyst has a stable catalytic performance, the diffusion of the reaction system is promoted, the reaction rate is accelerated, the carbon deposition is reduced and the blockage of the pore channel is retarded.

According to the fourth type of embodiments of the present application, the carrier can be prepared using known methods capable of providing the doping element, the pore structure satisfying the above ranges and the like, and the obtaining of the carrier with the doping element and the pore structure satisfying the above ranges can be achieved by one skilled in the art. Preferably, the carrier is prepared by a method comprising the steps of:

(1) subjecting a mixture comprising a doping element, an alumina precursor and optionally an additional carrier precursor sequentially to shaping, first drying and first calcining, wherein said additional carrier precursor comprises a silica precursor (e.g. silica sol) and/or a molecular sieve precursor (e.g. ZSM-5);
(2) mixing the product of the first calcining with a solution of a Group IIA metal precursor, and then carrying out second drying and second calcining. The method for shaping may include kneading, rolling, or flaking, etc.

In the above method for producing the carrier, it can be appreciated by one skilled in the art that: where the raw material providing the precursor of the carrier already contains a desired amount of the doping element, the shaping may be simply performed using such raw material, and where the raw material providing the precursor of the carrier does not contain the doping element or the content of the doping element is low (insufficient), an introduction of additional doping element may be performed.

In the above method for producing the carrier, the doping element may be incorporated in the raw material providing the alumina precursor, or alumina precursor and/or additional carrier precursor modified by the doping element may be directly used, and such alumina precursor or additional carrier precursor modified by the doping element may be obtained commercially or by a conventional method, of which the detailed description is omitted herein for brevity.

In the above method for producing the carrier, one skilled in the art can determine the amount of the raw material (e.g., the carrier modifier) for a component (e.g. the doping element) based on the amount of said component in the final carrier, and therefore, the amounts of some raw materials are not given herein.

In the above method for producing the carrier, the alumina precursor is preferably pseudo-boehmite. The specific surface area of the pseudo-boehmite is preferably 250-330 $m^2/g$. The pore volume of the pseudo-boehmite is preferably 0.5 to 1.1. The pseudo-boehmite can be produced by at least one of carbonization method, organoaluminum hydrolysis method, aluminum sulfate method and nitric acid method, and particularly preferably produced by aluminum sulfate method. A catalyst with better performance can be obtained by using the pseudo-boehmite with the specific pore structure.

In the above method for producing the carrier, the conditions of the first drying and the second drying may each independently include: a temperature of 80-150 °C and a drying time of 6-20 h, preferably a temperature of 100-120 °C and a drying time of 8-15 h.

In the above method for producing the carrier, the conditions of the first calcining may include: a temperature of 500-650 °C, and a calcining time of 2-20 h, preferably a temperature of 520-620 °C, and a calcining time of 4-8 h.

In the above method for producing the carrier, the conditions of the second calcining may include: a temperature of 800-1100 °C, and a calcining time of 2-20 h, preferably a temperature of 800-980 °C, and a calcining time of 5-10 h.

In the fourth type of embodiments of the present application, there is also provided a process for producing organic amines, comprising: contacting an amination raw material and an amination reagent with the catalyst as described above in the presence of hydrogen for amination reaction.

According to the fourth type of embodiments of the present application, the amination raw material or amination reagent can be selected as described above, of which the detailed description is omitted herein for brevity.

According to the fourth type of embodiments of the present application, the amination conditions may include: a mole ratio of hydrogen to the amination reagent and to the amination raw material of 1-5 : 2-35 : 1, a temperature of 110-230°C, a pressure of 0.7-22 MPa, and a liquid phase volume space velocity of the amination raw material of 0.06-1 $m^3/(m^3 \cdot h)$.

Preferably, the amination raw material is a monohydric alcohol, and the amination conditions include: a mole ratio of hydrogen to the amination reagent and to the amination raw material of 1-4 : 2-8 : 1, a temperature of 130-210°C, a pressure of 1-2.5 MPa, and a liquid phase volume space velocity of the amination raw material of 0.1-0.8 $m^3/(m^3 \cdot h)$.

Preferably, the amination raw material is a ketone or an aldehyde, and the amination conditions include: a mole ratio of hydrogen to the amination reagent and to the amination raw material of 1-4 : 2-5 : 1, a temperature of 110-180°C, a pressure of 0.7-2.5 MPa, and a liquid phase volume space velocity of the amination raw material of 0.1-0.8 $m^3/(m^3 \cdot h)$.

Preferably, the amination raw material is an alcohol amine, and the amination conditions include: a mole ratio of hydrogen to the amination reagent and to the amination raw material of 1-4 : 3-23 : 1, a temperature of 130-200°C, a pressure of 1-16 MPa, and a liquid phase volume space velocity of the amination raw material of 0.06-0.8 $m^3/(m^3 \cdot h)$.

Preferably, the amination raw material is a mixture of 1,6-hexanediol, cycloheximide and 6-amino-1-hexanol or a dihydric alcohol, and the amination conditions include: a mole ratio of hydrogen to the amination reagent and to the amination raw material of 1-4 : 3-35 : 1, a temperature of 130-230°C, a pressure of 1-22 MPa, and a liquid phase volume space velocity of the amination raw material of 0.1-0.8 $m^3/(m^3 \cdot h)$.

In some preferred embodiments, the present application provides the following technical solutions:

A1, a catalyst having a function of catalyzing hydroamination of alcohols to produce organic amine, comprising a carrier, and an active metal component and optionally a metal promoter supported on the carrier, wherein the carrier comprises a matrix and a doping element, the matrix comprises an alumina carrier and optionally an additional carrier, and said additional carrier is at least one selected from silica, molecular sieve and diatomite; the proportion of the pore volume of pores having a pore diameter of less than 7.5 nm to the pore volume of the carrier is less than 20%, the proportion of the pore volume of pores having a pore diameter of less than 9 nm to the pore volume of the carrier is less than 40%, and the proportion of the pore volume of pores having a pore diameter of greater than 27nm to the pore volume of the carrier is less than 5%; the carrier has an ammonia adsorption capacity of 0.3-0.6 mmol/g; the carrier has an L acid content of 90% or more relative to the total of the L acid and B acid contents; the active metal component is cobalt and/or nickel.

A2, the catalyst according to Item A1, wherein the content of the alumina carrier in the matrix is 65 wt% or more, preferably 75 wt% or more, relative to the total amount of the alumina carrier and said additional carrier;

and/or the content of the doping element is 0.05-3 wt%, preferably 0.08-2 wt%, relative to the total weight of the matrix;

and/or the doping element is derived from acid radical ions other than chloride ion; the acid radical ion is at least one selected from the group consisting of non-metallic acid radical ions, preferably at least one selected from the group consisting of borate ion, fluoride ion, phosphate ion, sulfate ion and selenate ion;

and/or, the proportion of the pore volume of pores having a pore diameter of less than 7.5nm to the pore volume of the carrier is 5-17%, the proportion of the pore volume of pores having a pore diameter of more than or equal to 7.5nm and less than 9nm to the pore volume of the carrier is 5-17%, the proportion of the pore volume of pores having a pore diameter of more than or equal to 9nm and less than or equal to 27nm to the pore volume of the carrier is 61-89.5%, and the proportion of the pore volume of pores having a pore diameter of greater than 27nm to the pore volume of the carrier is 0.5-5%;

and/or the carrier has an ammonia adsorption capacity of 0.3-0.5 mmol/g;

and/or the carrier has an L acid content of 92-100%, relative to the total of the L acid and B acid contents;

and/or the carrier has a specific surface area of 105-220 $m^2$/g;

and/or the carrier has a pore volume of 0.4-1.1 ml/g;

and/or the content of the active metal component is 5-42g, preferably 10-35 g, per 100g of the matrix.

A3, the catalyst according to Item A1 or A2, wherein the carrier is prepared by a method comprising the steps of: sequentially shaping, drying and calcining a mixture of a carrier modifier, pseudo-boehmite and optionally an additional carrier source, wherein said additional carrier source is at least one of silica precursor, molecular sieve precursor and diatomite precursor, and the calcining temperature is 800-1050 °C.

A4, the catalyst according to Item A3, wherein the carrier modifier is at least one selected from the group consisting of non-metallic acid radical ions, preferably at least one selected from borate ion, fluoride ion, phosphate ion, sulfate ion and selenate ion.

A5, the catalyst according to Item A3 or A4, wherein the carrier modifier is at least one selected from the group consisting of boric acid, nickel borate, cobalt borate, potassium borate, ammonium borate, potassium fluoride, cobalt fluoride, nickel fluoride, hydrofluoric acid, ammonium fluoride, phosphoric acid, aluminum phosphate, tripotassium phosphate, potassium dihydrogen phosphate, potassium hydrogen phosphate, magnesium phosphate, calcium phosphate, ammonium phosphate, sulfuric acid, cobalt sulfate, nickel sulfate, aluminum sulfate, calcium sulfate, potassium sulfate, magnesium sulfate, strontium phosphate, strontium sulfate, and selenic acid;

and/or the pseudo-boehmite has a specific surface area of 255-360 $m^2$/g, and a pore volume of 0.75-1.3 ml/g.

A6, the catalyst according to any one of Items A3-A5, wherein the drying conditions include: a temperature of 80-150 °C, and a drying time of 6-20 h;

and/or the calcining conditions include: a temperature of 800-1050 °C, and a calcining time of 2-20 h.

A7, a method for producing the catalyst according to any one of Items A1-A6, comprising: loading the active metal component and optionally the metal promoter on the carrier.

A8, a carrier as defined in any one of Items A1-A6.

A9, Use of the catalyst according to any one of Items A1-A6, or the method according to Item A7, or the carrier according to Item A8, in the production of organic amines by amination.

A10, a process for producing an organic amine, comprising: contacting an amination raw material and an amination reagent with the catalyst according to any one of Items A1-A6 in the presence of hydrogen for amination reaction; or alternatively, the method comprises: screening a catalyst comprising a carrier as defined in according to any one of Items A1 to A6, and contacting an amination raw material and an amination reagent with the screened catalyst in the presence of hydrogen for amination reaction.

A11, the method according to Item A10, wherein the amination conditions include: a mole ratio of hydrogen to the amination reagent and to the amination raw material of 1-5 : 2-35 : 1, a temperature of 110-220 °C, a pressure of 1-25 MPa, and a liquid phase volume space velocity of the amination raw material of 0.06-1 $m^3/(m^3 \cdot h)$;

and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, ethanolamine, propanolamine, isopropanolamine, 6-aminohexanol, diethanolamine, acetone, ethylene glycol, 1,3-propanediol, and 1,12-dodecanediol;

and/or the amination reagent is at least one selected from the group consisting of ammonia, C1-12 primary amines and C1-12 secondary amines, preferably at least one of ammonia, monomethylamine, dimethylamine, methyl ethyl amine, monoethylamine and diethylamine.

A12, the method according to Item A11, wherein, where the amination raw material is a monohydric alcohol, the amination conditions include: a mole ratio of hydrogen to the amination reagent and to the amination raw material of 1-4 : 2-8 : 1, a temperature of 130-200 °C, a pressure of 1-3.5 MPa, and a liquid phase volume space velocity of the amination raw material of 0.1-0.8 $m^3/(m^3 \cdot h)$;

or, where the amination raw material is a ketone or an aldehyde, the amination conditions include: a mole ratio of hydrogen to the amination reagent and to the amination raw material of 1-4 : 2-5 : 1, a temperature of 110-180 °C, a pressure of 1-2.5 MPa, and a liquid phase volume space velocity of the amination raw material of 0.1-0.8 $m^3/(m^3 \cdot h)$;

or, where the amination raw material is an alcohol amine, the amination conditions include: a mole ratio of hydrogen to the amination reagent and to the amination raw material of 1-4 : 3-20 : 1, a temperature of 130-200 °C, a pressure of 1-11 MPa, and a liquid phase volume space velocity of the amination raw material of 0.06-0.8 $m^3/(m^3 \cdot h)$;

or, where the amination raw material is a dihydric alcohol, the amination conditions include: a mole ratio of hydrogen to the amination reagent and to the amination raw material of 1-5 : 2-35 : 1, a temperature of 130-220 °C, a pressure of 1-25 MPa, and a liquid phase volume space velocity of the amination raw material of 0.1-0.8 $m^3/(m^3 \cdot h)$;

or, where the amination raw material is a mixture of 1,6-hexanediol, hexamethyleneimine and 6-amino-1-hexanol, the amination conditions include: a mole ratio of hydrogen to the amination reagent and to the amination raw material of 1-4 : 3-35 : 1, a temperature of 130-200 °C, a pressure of 1-22 MPa, and a liquid phase volume space velocity of the amination raw material of 0.1-0.8 $m^3/(m^3 \cdot h)$.

B1, a catalyst having a function of catalyzing the production of amins from alcohols, comprising a carrier, and an active metal component and optionally a metal promoter supported on the carrier, wherein the carrier is at least one selected from the group consisting of doped alumina, doped silica, doped molecular sieves and doped diatomite; the carrier has an ammonia adsorption capacity of 0.25-0.65 mmol/g; the carrier has an L acid content of 88% or more, relative to the total of the L acid and B acid contents; the active metal component is cobalt and/or nickel and the grain size of the active metal component in the catalyst is less than 10 nm.

B2. the catalyst according to Item B1, wherein the carrier comprises a matrix selected from the group consisting of alumina, silica, molecular sieves and diatomite and a doping element present in an amount of 0.05 to 5 wt%, preferably 0.08 to 4 wt%, relative to the total weight of the matrix;

and/or the doping element in the carrier is derived from acid radical ions, excluding chloride ion; the acid radical ion is at least one selected from the group consisting of non-metallic acid radical ions, preferably at least one selected from the group consisting of borate ion, fluoride ion, phosphate ion, sulfate ion and selenate ion;

and/or the carrier has an ammonia adsorption capacity of 0.3-0.5 mmol/g;

and/or the carrier has an L acid content of 90-100%, relative to the total of the L acid and B acid contents;

and/or the grain size of the active metal component in the catalyst is 3-8 nm;

and/or the carrier has a specific surface area of 100-200 $m^2/g$;

and/or the carrier has a pore volume of 0.45-1 ml/g;

and/or the carrier has an isoelectric point of 3-6, preferably 3.5-5.5;

and/or the content of the active metal component is 7 to 45 g, preferably 12 to 38 g, per 100g of the matrix.

B3, the catalyst according to Item B1 or B2, wherein the carrier is prepared by a method comprising the steps of: sequentially shaping, drying and calcining a mixture comprising a doping ion and a precursor of the matrix, wherein the doping ion is provided by a carrier modifier, the carrier modifier is at least one of inorganic acids containing the doping ion and inorganic salts containing the doping ion, and the calcining temperature is 800 °C or higher.

B4. the catalyst according to Item B3, wherein the inorganic acid is at least one selected from inorganic acids containing a non-metallic acid radical ion, the inorganic salt is at least one selected from inorganic salts containing a non-metallic acid radical ion, and the carrier modifier is preferably at least one selected from boric acid, potassium borate, magnesium borate, hydrofluoric acid, potassium fluoride, magnesium fluoride, phosphoric acid, potassium phosphate, magnesium phosphate, sulfuric acid, potassium sulfate, magnesium sulfate, and selenic acid.

B5, the catalyst according to Item B3 or B4, wherein the precursor of the matrix is pseudo-boehmite, the pseudo-boehmite has a specific surface area of 260-400 $m^2/g$, and a pore volume of 0.75-1.2 ml/g.

B6, the catalyst according to any one of Items B3-B5, wherein the drying conditions include: a temperature of 80-150 °C, and a drying time of 6-20 h;

and/or, the calcining conditions include: a temperature of 820-1120 °C, and a calcining time of 2-20 h.

B7, a method for producing the catalyst according to any one of Items B1-B6, comprising: impregnating the carrier with an impregnation solution containing a precursor of an active metal component and optionally a precursor of a metal promotor, so that the active metal component and the optional metal promotor are supported on the carrier, the impregnation solution having a pH value in a range of 3.5 to 5.5.

B8, a carrier as defined in any one of Items B1-B6.

B9, Use of the catalyst according to any one of Items B1-B6, or the method according to Item B7, or the carrier according to Item B8, for the production of organic amines by amination.

B10, a process for producing an organic amine, comprising: contacting an amination raw material and an amination reagent with the catalyst according to any one of Items B1-B6 in the presence of hydrogen for amination reaction; or alternatively, screening a catalyst comprising the carrier as defined in any one of Items B1-B6, and contacting an amination raw material and an amination reagent with the screened catalyst in the presence of hydrogen for amination reaction.

B11, the method according to Item B10, wherein the amination conditions include: a mole ratio of hydrogen to the amination reagent and to the amination raw material of 1-5 : 3-33 : 1, a temperature of 110-210 °C, a pressure of 1-22 MPa, and a liquid phase volume space velocity of the amination raw material of 0.06-1 $m^3/(m^3 \cdot h)$;

> and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, ethanolamine, propanolamine, isopropanolamine, 6-aminohexanol, diethanolamine, diisopropanolamine, dimethylethanolamine, acetone, ethylene glycol, 1,3-propanediol, and 1,12-dodecanediol;
>
> and/or the amination reagent is at least one selected from the group consisting of ammonia, C1-12 primary amines and C1-12 secondary amines, preferably at least one of ammonia, monomethylamine, dimethylamine, methyl ethyl amine, monoethylamine and diethylamine.

B12, the method according to Item B11, wherein, where the amination raw material is a monohydric alcohol, the amination conditions include: a mole ratio of hydrogen to the amination reagent and to the amination raw material of 1-4 : 2-8 : 1, a temperature of 130-200 °C, a pressure of 0.8-2.5 MPa, and a liquid phase volume space velocity of the amination raw material of 0.1-0.8 $m^3/(m^3 \cdot h)$;

> or, where the amination raw material is a ketone or an aldehyde, the amination conditions include: a mole ratio of hydrogen to the amination reagent and to the amination raw material of 1-4 : 2-6 : 1, a temperature of 110-170 °C, a pressure of 1-2.5 MPa, and a liquid phase volume space velocity of the amination raw material of 0.1-0.8 $m^3/(m^3 \cdot h)$;
>
> or, where the amination raw material is an alcohol amine, the amination conditions include: a mole ratio of hydrogen to the amination reagent and to the amination raw material of 1-4 : 3-10 : 1, a temperature of 130-200 °C, a pressure of 1-11 MPa, and a liquid phase volume space velocity of the amination raw material of 0.06-0.8 $m^3/(m^3 \cdot h)$;
>
> or, where the amination raw material is a mixture of 1,6-hexanediol, hexamethyleneimine and 6-amino-1-hexanol or a dihydric alcohol, the amination conditions include: a mole ratio of hydrogen to the amination reagent and to the amination raw material of 1-4 : 3-33 : 1, a temperature of 130-210 °C, a pressure of 1-22 MPa, and a liquid phase volume space velocity of the amination raw material of 0.1-0.8 $m^3/(m^3 \cdot h)$.

C1, a catalyst having a function of producing amines by the hydroamination of alcohols, comprising a carrier, and an active metal component and optionally a metal promoter supported on the carrier, wherein the carrier comprises a matrix and a doping element, the matrix comprises an alumina carrier and optionally an additional carrier, and said additional carrier is selected from silica and/or molecular sieve; the doping element is at least one selected from the group consisting of boron, fluorine, phosphorus, sulfur and selenium; the proportion of the pore volume of pores having a pore diameter in a range of 7-27 nm to the pore volume of the carrier is greater than 65%; the active metal component is cobalt and/or nickel.

C2. the catalyst according to Item C1, wherein the content of the alumina carrier in the carrier matrix is not less than 70 wt%, preferably 75 to 100 wt%;

and/or, the content of the doping element in the carrier is 0.05 to 6 wt%, preferably 0.08 to 4 wt%, relative to the total weight of the matrix;

and/or, the doping element is incorporated in the form of at least one selected from the group consisting of borate ion, fluoride ion, phosphate ion, sulfate ion and selenate ion;

and/or, the proportion of the pore volume of pores having a pore diameter in a range of 7-27 nm to the pore volume of the carrier is greater than 65%; preferably, the proportion of the pore volume of pores having a pore diameter of 7-27 nm to the pore volume of the carrier is 70-90%, and the proportion of the pore volume of pores having a pore diameter of less than 7 nm to the pore volume of the carrier is 0-10%;

and/or, the carrier has an L acid content of 85% or more, preferably 85-98%, relative to the total of the L acid and B acid contents;

and/or, the carrier has a specific surface area of 120-210 $m^2/g$;

and/or, the carrier has a pore volume of 0.43-1.1 ml/g;

and/or, the content of the active metal component is 8-44 g, preferably 12-37 g, per 100g of the matrix.

C3, the catalyst according to Item C1 or C2, wherein the carrier is prepared by a method comprising: sequentially shaping, drying and calcining a mixture comprising the doping element and a precursor of the carrier, wherein the precursor of the carrier is selected from precursors of alumina and optionally precursors of additional matrix, and the precursor of additional matrix is selected from precursors of silica and/or precursors of molecular sieves.

C4, the catalyst according to Item C3, wherein the doping element is provided by at least one selected from compounds containing non-metallic acid radical ions, preferably at least one selected from compounds containing borate ion, compounds containing fluoride ion, compounds containing phosphate ion, compounds containing sulfate ion, and compounds containing selenate ion.

C5, the catalyst according to Item C3 or C4, wherein the alumina precursor is pseudo-boehmite, the pseudo-boehmite has a specific surface area of 255-340 $m^2/g$, and a pore volume of 0.78-1.25 ml/g.

C6, the catalyst according to any one of Items C3-C5, wherein the drying conditions include: a temperature of 80-150 °C, and a drying time of 6-20 h;

and/or, the calcining conditions include: a temperature of 700-1100 °C, and a calcining time of 2-20 h.

C7, a method for producing the catalyst according to any one of Items C1-C6, comprising: loading the active metal component and optionally the metal promoter on the carrier.

C8, a carrier as defined in any one of Items C1 to C6.

C9, Use of the catalyst according to any one of Items C1-C6, or the method according to Item C7, or the carrier according to Item C8, for producing organic amines by amination.

C10, a process for producing an organic amine, comprising: contacting an amination raw material and an amination reagent with the catalyst according to any one of Items C1-C6 in the presence of hydrogen for amination reaction; or alternatively, screening a catalyst comprising the carrier as defined in any one of Items C1-C6 and contacting an amination raw material and an amination reagent with the screened catalyst in the presence of hydrogen for amination reaction.

C11, the process according to Item C10, wherein the amination conditions include: a mole ratio of hydrogen to the amination reagent and to the amination raw material of 1-6: 2-32 : 1, a temperature of 105-210 °C, a pressure of 1-17 MPa, and a liquid phase volume space velocity of the amination raw material of 0.06-1 $m^3/(m^3 \cdot h)$;

and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, ethanolamine, propanolamine, isopropanolamine, 6-aminohexanol, diethanolamine, diisopropanolamine, dimethylethanolamine, acetone, ethylene glycol, 1,3-propanediol, and 1,12-dodecanediol;

and/or, the amination reagent is at least one selected from the group consisting of ammonia, C1-12 primary amines and C1-12 secondary amines, preferably at least one of ammonia, monomethylamine, dimethylamine, methyl ethyl amine, monoethylamine and diethylamine.

C12, the process according to Item C11, wherein, where the amination raw material is a monohydric alcohol, the amination conditions include: a mole ratio of hydrogen to the amination reagent and to the amination raw material of 1-4 : 2-9 : 1, a temperature of 130-208 °C, a pressure of 1-2.5 MPa, and a liquid phase volume space velocity of the amination raw material of 0.1-0.8 $m^3/(m^3 \cdot h)$;

or, where the amination raw material is a ketone or an aldehyde, the amination conditions include: a mole ratio of hydrogen to the amination reagent and to the amination raw material of 1-4 : 2-5 : 1, a temperature of 105-160 °C, a pressure of 1-2 MPa, and a liquid phase volume space velocity of the amination raw material of 0.1-1 $m^3/(m^3 \cdot h)$;

or, where the amination raw material is an alcohol amine, the amination conditions include: a mole ratio of hydrogen to the amination reagent and to the amination raw material of 1-4 : 3-20 : 1, a temperature of 130-200 °C, a pressure of 1-13 MPa, and a liquid phase volume space velocity of the amination raw material of 0.06-0.8 $m^3/(m^3 \cdot h)$;

or, where the amination raw material is a mixture of 1,6-hexanediol, hexamethyleneimine and 6-amino-1-hexanol or a dihydric alcohol, the amination conditions include: a mole ratio of hydrogen to the amination reagent and to the amination raw material of 1-4 : 3-32 : 1, a temperature of 130-210 °C, a pressure of 1-17 MPa, and a liquid phase volume space velocity of the amination raw material of 0.1-0.9 $m^3/(m^3 \cdot h)$.

D1, a catalyst having a function of catalyzing the production of amines from alcohols, comprising a carrier, and an active metal component and a metal promoter supported on the carrier, wherein the active metal component is cobalt and/or nickel; the metal promoter is a combination of at least one Group IIA metal, at least one Group IIB metal, and at least one Group VA metal.

D2, The catalyst according to Item D1, wherein the carrier comprises a matrix and a doping element, the matrix comprises an alumina carrier and optionally an additional carrier, the additional carrier comprises silica and/or molecular sieves; the doping element is at least one selected from the group consisting of boron, fluorine, phosphorus, sulfur and selenium; the proportion of the pore volume of pores having a pore diameter in a range of 7-27 nm to the pore volume of the carrier is greater than 65%.

D3, the catalyst according to Item D1 or D2, wherein the content of the alumina carrier in the carrier matrix is 70 wt% or more, preferably 75-100 wt%, relative to the total amount of the alumina carrier and said additional carrier;

and/or the content of the doping element in the carrier is 0.05 to 4.5 wt%, preferably 0.07 to 2.8 wt%, relative to the total weight of the matrix;

and/or the doping element in the carrier is incorporated in the form of at least one selected from the group consisting of borate ion, fluoride ion, phosphate ion, sulfate ion and selenate ion;

and/or the proportion of the pore volume of pores having a pore diameter in a range of 7-27 nm to the pore volume of the carrier is 70-90%, and the proportion of the pore volume of pores having a pore diameter of less than 7 nm to the pore volume of the carrier is 0-8%;

and/or the carrier has a specific surface area of 110-210 $m^2/g$;

and/or the carrier has a pore volume of 0.45-1.1 ml/g;

and/or the content of said active metal component is 8-45 g, preferably 15-38 g, per 100g of the matrix;

and/or the metal promoter is present in an amount of 0.1 to 10g, preferably 0.5 to 6g, per 100 g of the matrix;

and/or the weight ratio of the Group IIA metal, the Group IIB metal and the Group VA metal in the metal promoter is 0.1-10 : 0.1-10 : 1, preferably 0.2-8 : 0.2-8 : 1;

and/or, the Group IIA metal is at least one selected from the group consisting of magnesium, calcium and barium;

and/or, the Group IIB metal is selected from zinc;

and/or, the Group VA metal is selected from bismuth.

D4, the catalyst according to Item D2 or D3, wherein the carrier is prepared by a method comprising the steps of:

(1) subjecting a mixture comprising a doping element, an alumina precursor and optionally an additional carrier precursor sequentially to shaping, first drying and first calcining, wherein said additional carrier precursor comprises a silica precursor and/or a molecular sieve precursor;

(2) mixing the product of the first calcining with a solution of a Group IIA metal precursor, and then carrying out second drying and second calcining.

D5, the catalyst according to Item D4, wherein the alumina precursor is pseudo-boehmite, the pseudo-boehmite has a specific surface area of 250-330 $m^2/g$, and a pore volume of 0.5-1.1 ml/g;

and/or the conditions of the first calcining include: a temperature of 500-650 °C, and a calcining time of 2-20 h;

and/or the conditions of the second calcining include: a temperature of 800-1100 °C, and a calcining time of 2-20 h.

D6, a method for producing the catalyst according to any one of Items D1-D5, comprising:

(1) subjecting a mixture comprising a doping element, an alumina precursor and optionally an additional carrier precursor sequentially to shaping, first drying and first calcining, wherein said additional carrier precursor comprises a silica precursor and/or a molecular sieve precursor;

(2) mixing the product of the first calcining with a solution of a Group IIA metal precursor, and then carrying out second drying and second calcining;

(3) loading at least one Group IIB metal, at least one Group VA metal, and an active metal component on the product of the second calcining.

D7, a carrier as defined in any one of Items D1 to D5.

D8, Use of the catalyst according to any one of Items D1-D5, or the method according to Item D6, or the carrier according to Item D7 for producing organic amines by amination.

D9, a method for producing organic amines, comprising the following steps: contacting an amination raw material and an amination reagent with the catalyst according to any one of Items D1-D5 in the presence of hydrogen for amination reaction;

or alternatively, the method comprising: screening a catalyst comprising a carrier as defined in any one of Items D1-D5, and contacting an amination raw material and an amination reagent with the screened catalyst in the presence of hydrogen for amination reaction.

D10, the method according to Item D9, wherein the amination conditions include: a mole ratio of hydrogen to the amination reagent and to the amination raw material of 1-5 : 2-35 : 1, a temperature of 110-230°C, a pressure of 0.7-22 MPa, and a liquid phase volume space velocity of the amination raw material of 0.06-1 $m^3/(m^3 \cdot h)$;

and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, ethanolamine, propanolamine, isopropanolamine, 6-aminohexanol, diethanolamine, diisopropanolamine, dimethylethanolamine, acetone, ethylene glycol, 1,3-propanediol, and 1,12-dodecanediol;

and/or the amination reagent is at least one selected from the group consisting of ammonia, C1-12 primary amines and C1-12 secondary amines, preferably at least one of ammonia, monomethylamine, dimethylamine, methyl ethyl amine, monoethylamine and diethylamine.

D11, the method according to Item D10, wherein, where the amination raw material is a monohydric alcohol, the amination conditions include: a mole ratio of hydrogen to the amination reagent and to the amination raw material of 1-4 : 2-8 : 1, a temperature of 130-210°C, a pressure of 1-2.5 MPa, and a liquid phase volume space velocity of the amination raw material of 0.1-0.8 $m^3/(m^3 \cdot h)$;

or, where the amination raw material is a ketone or an aldehyde, the amination conditions include: a mole ratio of hydrogen to the amination reagent and to the amination raw material of 1-4 : 2-5 : 1, a temperature of 110-180°C, a pressure of 0.7-2.5 MPa, and a liquid phase volume space velocity of the amination raw material of 0.1-0.8 $m^3/(m^3 \cdot h)$;

or, where the amination raw material is an alcohol amine, the amination conditions include: a mole ratio of hydrogen to the amination reagent and to the amination raw material of 1-4 : 3-23 : 1, a temperature of 130-200°C, a pressure of 1-16 MPa, and a liquid phase volume space velocity of the amination raw material of 0.06-0.8 $m^3/(m^3 \cdot h)$;

or, where the amination raw material is a mixture of 1,6-hexanediol, hexamethyleneimine and 6-amino-1-hexanol or a dihydric alcohol, the amination conditions include: a mole ratio of hydrogen to the amination reagent and to the amination raw material of 1-4 : 3-35 : 1, a temperature of 130-230°C, a pressure of 1-22 MPa, and a liquid phase volume space velocity of the amination raw material of 0.1-0.8 $m^3/(m^3 \cdot h)$.

## Examples

The present application will be further illustrated with reference to the following examples, but the present application is not limited thereto.

The test instrument and methods used in the following examples are as follows:

1) NH$_3$-TPD test

Test instrument: Autochem 2920 Full-automatic Chemical Adsorption Instrument (Automated Catalyst Characterization System), manufactured by MICROMERITICS, USA;

Test conditions: about 0.1g of sample was accurately weighed, put into a sample tube, heated to a temperature of 600 °C at a rate of 10 °C/min while purging with helium gas, stayed for 1h, and cooled to a temperature of 120 °C; the gas was changed to 10% NH$_3$-He mixed gas, adsorbed for 60min, then the gas was changed again to helium gas purging for 1h, counting was started after the baseline became stable; and the resultant was heated to a temperature of 600 °C at a rate of 10 °C/min, kept for 30min, the recording was stopped, and the experiment was completed. An integral computation was conducted on the peak area, obtaining an NH$_3$ desorption amount, and the desorption amount was used for characterizing the ammonia adsorption capacity of the sample.

2) BET test

Test instrument: ASAP2420 Full-automatic Physicochemical Adsorption Analyzer (Automatic Micropore & Chemisorption Analyzer), manufactured by MICROMERITICS, USA;

Test conditions: test gas: N$_2$ (99.999% pure); degassing conditions: heating to 350 °C at a rate of 10 °C/min, and vacuumizing for 4 h; analysis conditions: conducting a full analysis on the mesopore isotherm to obtain the specific surface area and the pore volume.

3) Probe adsorption spectrometry

Test instrument: NICOLET 6700 Infrared Spectrometer available from Thermo Scientific, with in-situ transmission cell;

Test conditions: the sample was accurately weighed and its mass was recorded, heated to 500 °C at a heating rate of 10 °C/min under a vacuum condition, the carrier is pretreated at the temperature for 2 hours, and then cooled to room temperature. The pretreated carrier was allowed to adsorb pyridine vapor to saturation at room temperature. Then, the carrier was statically desorbed to an equilibrium state under a vacuum condition at the temperature points of room temperature, 100 °C, 150 °C, 200 °C, 300 °C and 400 °C, respectively, with a heating rate between every two temperature points being 10 °C/min.

4) XRD analysis

Test instrument: Empyrean X-ray diffractometer manufactured by PANalytical B.V., with an anode target of Cu target, and a Pixcel 3D detector;

Test conditions: tube voltage 40KV, tube current 40mA, divergence slit 1/4°, anti-divergence slit 1/2°, receiving slit height 7.5mm, scanning speed 0.013°/step, scanning range 5°-90°.

The grain size of the active metal component and the metal promoter, if present, was obtained by calculation using the Scherrer equation.

5) Isoelectric point test

Test instrument: Zetasizer Nano ZSP particle size potentiometer available from Malvern Panalytical Company;

Test method: the sample was ground into a powder and dispersed in a low-concentration NaCl solution, the Zeta potential of the sample was measured using a particle-size potentiometer at different pH values, and then plotted against pH. The pH value at a Zeta potential of 0 is the isoelectric point of the sample.

In the following examples and comparative examples, unless otherwise specified, reagents and starting materials used are commercially available products, which are analytically pure.

Example series I

The first type of embodiments of the present application are further illustrated in detail hereinbelow with reference to the examples of Example series I. In the following examples of Example series I, the pseudo-boehmite powder had a (Al$_2$O$_3$) content on a dry basis of 72 wt%, and the silica sol was purchased from Qingdao Ocean Chemical Co., Ltd., under a trade name of JN-40.

Example I-1

Pseudo-boehmite powder (with a specific surface area of 315 m$^2$/g and a pore volume of 0.91 ml/g) was kneaded with a dilute acid aqueous solution containing nitric acid and boric acid, extruded into strips with a diameter of 5 mm, cut to a length of 4 mm, dried at 100 °C for 10 h, calcined at 850 °C for 4h to obtain a desired carrier, and the amount of boric acid was adjusted to obtain a boron content in the carrier as shown in Table I-1.
151.2 g of cobalt nitrate hexahydrate (industrial grade, with a purity of 98%) was dissolved in water to obtain a 184mL solution, and the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst C-I-1. The grain size of the supported component was 20 nm as determined by XRD (of which the detailed description can be found in Test Example I-1).

Example I-2

Silica sol was added into pseudo-boehmite powder (with a specific surface area of 322 m$^2$/g, and a pore volume of 0.93 ml/g) in a kneader, mixed uniformly, kneaded with a dilute acid aqueous solution containing nitric acid and hydrofluoric acid, extruded into granules of clover shape with a thickness of 3 mm, dried at 120 °C for 6h, then calcined at 820 °C for 3.5h to obtain a desired carrier, and the amount of hydrofluoric acid was adjusted to obtain a content of the F element in the carrier as shown in Table I-1. The amount of silica sol was adjusted to obtain a mass ratio of Al$_2$O$_3$ to SiO$_2$ in the carrier of 9 : 1.
177 g of nickel nitrate hexahydrate (industrial grade, with a purity of 98%) was dissolved in water to obtain a 172mL solution, and 3.7 g of ammonium molybdate tetrahydrate (analytically pure) was dissolved in water to obtain a 86mL solution; the nickel nitrate solution was loaded onto 100 g of the carrier obtained by spray impregnation in two times; and the ammonium molybdate solution was loaded onto the carrier by spray impregnation in one time, dried at 120 °C for 4 hours after each time of spray impregnation, calcined at 390 °C for 4 hours, then reduced with hydrogen while gradually increasing the temperature at a rate of 20 °C/h, and finally reduced at 440 °C for 3 hours to obtain a catalyst C-I-2. The grain size of the supported component was 22 nm as determined by XRD.

Example I-3

Silica sol was added into pseudo-boehmite powder (with a specific surface area of 345m$^2$/g, a pore volume of 1.12 ml/g) in a kneader, mixed uniformly, kneaded with a dilute acid aqueous solution containing nitric acid and phosphoric acid, extruded into dentate spheres with a diameter of 4 mm, dried at 80 °C for 20 h, then calcined at 800 °C for 4h to obtain a desired carrier, and adjusting the amount of the phosphoric acid to obtain a content of the P element in the carrier as shown in Table I-1. The amount of silica sol was adjusted to obtain a mass ratio of Al$_2$O$_3$ to SiO$_2$ in the carrier of 3 : 1.
50.4 g of cobalt nitrate hexahydrate (industrial grade, with a purity of 98%) and 19.5g of a 50wt% manganese nitrate solution and 8.5 g of copper nitrate trihydrate (analytically pure) were dissolved in water to obtain a 158 mL solution, and the mixed solution was loaded onto 100 g of the carrier obtained by spray impregnation in two times, dried at 120 °C for 4 hours after each time of spray impregnation, then calcined at 395 °C for 4 hours, then reduced with hydrogen while gradually increasing the temperature at a rate of 20 °C/h, and finally reduced at 430 °C for 3 hours to obtain a catalyst C-I-3. The grain size of the supported component was 15nm as determined by XRD.

Example I-4

Pseudo-boehmite powder (with a specific surface area of 350 m$^2$/g and a pore volume of 1.13 ml/g) was mixed with diatomite powder (with a specific surface area of 57 m$^2$/g), kneaded with a dilute acid aqueous solution containing nitric acid and sulfuric acid, extruded into strips with a diameter of 5 mm, cut to a length of 4 mm, dried at 150 °C for 6 hours, calcined at 880 °C for 4 hours to obtain a desired carrier, and the amount of sulfuric acid was adjusted to obtain a content of the S element in the carrier as shown in Table I-1. The amount of diatomite was adjusted to obtain a mass ratio of Al$_2$O$_3$ to SiO$_2$ in the carrier of 19 : 1.
126.4 g of nickel nitrate hexahydrate (industrial grade, with a purity of 98%) is dissolved in water to obtain a 176 mL solution, the mixed solution was loaded onto 100 g of the carrier obtained by spray impregnation in two times, dried at 120 °C for 4 hours after each time of spray impregnation, then calcined at 380 °C for 4.5 hours, then reduced with hydrogen while gradually increasing the temperature at a rate of 20 °C/h, and finally reduced at 430 °C for 3 hours to obtain a catalyst C-I-4. The grain size of the supported component was 22 nm as determined by XRD.

Example I-5

Pseudo-boehmite powder (with a specific surface area of 320 $m^2$/g, a pore volume of 0.9 ml/g) was kneaded with a dilute acid aqueous solution containing nitric acid and sulfuric acid, extruded into granules of clover shape with a thickness of 3 mm, dried at 120 °C for 8 h, and then calcined at 890 °C for 4.5h to obtain a desired carrier, wherein the amount of sulfuric acid was adjusted to obtain a content of the S element in the carrier as shown in Table I-1.
40.4 g of nickel nitrate hexahydrate (industrial grade, with a purity of 98%) and 60.5 g of cobalt nitrate hexahydrate (industrial grade, with a purity of 98%) and 2.9g of ammonium perrhenate (with a purity of 99%) were dissolved in 186 mL of water, and the mixed solution was loaded onto 100 g of the carrier obtained by spray impregnation in two times, dried at 100 °C for 6 hours after each time of spray impregnation, then calcined at 390 °C for 4 hours, then reduced with hydrogen while gradually increasing the temperature at a rate of 20 °C/h, and finally reduced at 440 °C for 3 hours to obtain a catalyst C-I-5. The grain size of the supported component was 16 nm as determined by XRD.

Example I-6

Pseudo-boehmite powder (with a specific surface area of 312m $^2$/g, a pore volume of 0.88 ml/g) was mixed with molecular sieve powder (model ZSM-5, available from Catalyst Plant of Nankai University, $SiO_2$/$Al_2O_3$ =45 (molar ratio)), kneaded with a dilute acid aqueous solution containing nitric acid and selenic acid, extruded into dentate spheres with a diameter of 4 mm, dried at 120 °C for 8 h, then calcined at 810 °C for 6h to obtain a desired carrier, and adjusting the amount of the selenic acid to obtain a content of the Se element in the carrier as shown in Table I-1. The amount of molecular sieve was adjusted to obtain a mass ratio of $Al_2O_3$ to $SiO_2$ in the carrier of 94 : 6.
126 g of cobalt nitrate hexahydrate (industrial grade, with a purity of 98%) was dissolved in water to obtain a 152 mL solution, the mixed solution was loaded onto 100 g of the carrier obtained by spray impregnation in two times, dried at 120 °C for 5 hours after each time of spray impregnation, then calcined at 400 °C for 3.5 hours, then reduced with hydrogen while gradually increasing the temperature at a rate of 20 °C/h, and finally reduced at 430 °C for 3 hours to obtain a catalyst C-I-6. The grain size of the supported component was 25 nm as determined by XRD.

Example 1-7

Pseudo-boehmite powder (with a specific surface area of 348 $m^2$/g, a pore volume of 1.13 ml/g) was kneaded with a dilute acid aqueous solution containing nitric acid and boric acid, extruded into dentate spheres with a diameter of 4 mm, dried at 100 °C for 8h, and then calcined at 950 °C for 6.5h to obtain a desired carrier, wherein the amount of boric acid used was adjusted to obtain a content of the B element in the carrier as shown in Table 1-1.
100.8 g of cobalt nitrate hexahydrate (industrial grade, with a purity of 98%) and 1.3g of silver nitrate (analytically pure) were dissolved in water to obtain a 170mL solution, and the mixed solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 410 °C for 3 hours to obtain a catalyst C-I-7. The grain size of the supported component was 11nm as determined by XRD.

Example 1-8

Pseudo-boehmite powder (with a specific surface area of 356m $^2$/g, and a pore volume of 1.2 ml/g) was kneaded with a dilute acid aqueous solution containing nitric acid, sulfuric acid and phosphoric acid, extruded into dentate spheres with a diameter of 3mm, dried at 100 °C for 8h, then calcined at 860 °C for 4h to obtain a desired carrier, and the amounts of phosphoric acid and sulfuric acid used were adjusted to obtain a content of the P element and a content of the S element in the carrier as shown in Table 1-1.
141.6 g of nickel nitrate hexahydrate (industrial grade, with a purity of 98%) and 3.1g of cerium nitrate hexahydrate (analytically pure) were dissolved in water to obtain a 184mL solution, and the mixed solution was loaded onto 100 g of the carrier obtained by spray impregnation in two times, dried at 120 °C for 5 hours after each time of spray impregnation, then calcined at 410 °C for 4 hours, then reduced with hydrogen while gradually increasing the temperature at a rate of 20 °C/h, and finally reduced at 400 °C for 3 hours to obtain a catalyst C-I-8. The grain size of the supported component was 12nm as determined by XRD.

Example 1-9

Silica sol was added into pseudo-boehmite powder (with a specific surface area of 315 $m^2$/g and a pore volume of 0.88 ml/g) in a kneader, mixed uniformly, kneaded with a dilute acid aqueous solution containing nitric acid and sulfuric acid, extruded into dentate spheres with a diameter of 3mm, dried at 100 °C for 8h, then calcined at 900 °C for 6h to obtain a desired carrier, and the amount of sulfuric acid was adjusted to obtain a content of the S element in the carrier as shown in Table I-1. The amount of silica sol was adjusted to obtain a mass ratio of $Al_2O_3$ to $SiO_2$ in the carrier of 72: 28.

100.8 g of cobalt nitrate hexahydrate (industrial grade, with a purity of 98%) and 50.6 g of nickel nitrate hexahydrate (industrial grade, with a purity of 98%) were dissolved in water to obtain a 146mL solution, and the mixed solution was loaded onto 100 g of the carrier obtained by spray impregnation in two times, dried at 100 °C for 8 hours after each time of spray impregnation, then calcined at 420 °C for 4 hours, then reduced with hydrogen while gradually increasing the temperature at a rate of 20 °C/h, and finally reduced at 410 °C for 3 hours, to obtain a catalyst C-I-9. The grain size of the supported component was 18nm as determined by XRD.

Example I-10

Silica sol was added into pseudo-boehmite powder (with a specific surface area of 292 $m^2/g$, a pore volume of 0.82 ml/g) in a kneader, mixed uniformly, kneaded with a dilute acid aqueous solution containing nitric acid, phosphoric acid and hydrofluoric acid, extruded into dentate spheres with a diameter of 3mm, dried for 7h at 110 °C, then calcined for 7h at 970 °C, to obtain a desired carrier, and the amounts of phosphoric acid and hydrofluoric acid were adjusted to obtain a content of P element and a content of F element in the carrier as shown in Table I-1. The amount of silica sol was adjusted to obtain a mass ratio of $Al_2O_3$ to $SiO_2$ in the carrier of 66: 34.

201.6g of cobalt nitrate hexahydrate (industrial grade, with a purity of 98%) and 6.2 g of lanthanum nitrate hexahydrate (analytically pure) were dissolved in water to obtain a 165mL solution, and the mixed solution was loaded onto 100 g of the carrier obtained by spray impregnation in three times, dried at 120 °C for 6 hours after each time of spray impregnation, then calcined at 420 °C for 4 hours, then reduced with hydrogen while gradually increasing the temperature at a rate of 20 °C/h, and finally reduced at 410 °C for 3 hours to obtain a catalyst C-I-10. The grain size of the supported component was 13nm as determined by XRD.

Example 1-11

Pseudo-boehmite powder (with a specific surface area of 276 $m^2/g$ and a pore volume of 0.79 ml/g) was kneaded with a dilute acid aqueous solution containing nitric acid and sulfuric acid, extruded into granules of clover shape having a thickness of 4 mm, dried at 110 °C for 6h, and then calcined at 930 °C for 6h to obtain a desired carrier, wherein the amount of sulfuric acid was adjusted to obtain a content of the S element in the carrier as shown in Table I-1.

15.2 g of nickel nitrate hexahydrate (industrial grade, with a purity of 98%), 25.2 g of cobalt nitrate hexahydrate (industrial grade, with a purity of 98%) and 4.4g of ammonium perrhenate (with a purity of 99%) were dissolved in water to obtain a 176mL solution, and the mixed solution was loaded onto 100 g of the carrier obtained by spray impregnation in two times, dried at 120 °C for 4 hours after each time of spray impregnation, then calcined at 390 °C for 5 hours, then reduced with hydrogen while gradually increasing the temperature at a rate of 20 °C/h, and finally reduced at 440 °C for 3 hours to obtain a catalyst C-I-11. The grain size of the supported component was 19nm as determined by XRD.

Example I-12

Pseudo-boehmite powder (with a specific surface area of 260 $m^2/g$, a pore volume of 0.77 ml/g) was kneaded with a dilute acid aqueous solution containing nitric acid and phosphoric acid, extruded into granules of clover shape having a thickness of 4 mm, dried at 100 °C for 12 h, then calcined at 860 °C for 9h to obtain a desired carrier, and the amount of phosphoric acid was adjusted to obtain a content of P element in the carrier as shown in Table I-1.

100.8g of cobalt nitrate hexahydrate (industrial grade, with a purity of 98%) and 14.1g of copper nitrate trihydrate (analytically pure) were dissolved in water to obtain a 180mL solution, and the mixed solution was loaded onto 100 g of the carrier obtained by spray impregnation in two times, dried at 120 °C for 4 hours after each time of spray impregnation, then calcined at 390 °C for 5 hours, then reduced with hydrogen while gradually increasing the temperature at a rate of 20 °C/h, and finally reduced at 440 °C for 3 hours to obtain a catalyst C-I-12. The grain size of the supported component was 21 nm as determined by XRD.

Example I-13

Pseudo-boehmite powder (with a specific surface area of 257 $m^2/g$ and a pore volume of 0.76 ml/g) was kneaded with a dilute acid aqueous solution containing nitric acid and boric acid, extruded into granules of clover shape with a thickness of 3mm, dried at 120 °C for 8 h, and then calcined at 850 °C for 6h to obtain a desired carrier, wherein the amount of boric acid was adjusted to obtain a content of the B element in the carrier as shown in Table I-1.

151.7g of nickel nitrate hexahydrate (industrial grade, with a purity of 98%) and 12.5g of lanthanum nitrate hexahydrate (analytically pure) were dissolved in water to obtain a 184mL solution, and the mixed solution was loaded onto 100 g of the carrier obtained by spray impregnation in two times, dried at 120 °C for 4 hours after each time of spray impregnation, then calcined at 370 °C for 6 hours, then reduced with hydrogen while gradually increasing the temperature at a rate of 20 °C/h, and finally reduced at 430 °C for 4 hours to obtain a catalyst C-I-13. The grain size of the supported component was 12nm as determined by XRD.

Example I-14

A catalyst C-I-14 was prepared as described in Example I-5, except that the sulfuric acid was added in such an amount that the content of the S element in the carrier was as shown in Table I-1. The grain size of the supported component was 16 nm as determined by XRD.

Example I-15

A catalyst C-I-15 was prepared as described in Example I-5, except that the temperature for calcining the carrier was 800 °C, and the calcining time was 2 hours. The grain size of the supported component was 14 nm as determined by XRD.

Example I-16

A catalyst was prepared as described in Example I-5, except that the silica sol was added while kneading in such an amount that the mass ratio of $Al_2O_3$ to $SiO_2$ in the carrier was 66: 34, obtaining a catalyst C-I-16. The grain size of the supported component was as 16 nm as determined by XRD.

Example I-17

ZSM-5 (with a specific surface area of 338 $m^2/g$, a pore volume of 0.61 ml/g, a crystallinity of 97.9%, $SiO_2/Al_2O_3$ = 60) was kneaded with a dilute acid aqueous solution containing nitric acid, sulfuric acid and phosphoric acid, extruded into dentate spheres with a diameter of 3mm, dried at 120 °C for 6 hours, and then calcined at 880 °C for 4 hours to obtain a desired carrier, and the amounts of phosphoric acid and sulfuric acid were adjusted to obtain a content of the P element and a content of the S element in the carrier as shown in Table I-1.
The impregnation of the catalyst was conducted as described in Example I-8, to obtain a catalyst C-I-17. The grain size of the supported component was 14 nm as determined by XRD.

Comparative Example I-1

A catalyst was prepared as described in Example I-12, except that in the production of the carrier, the calcining temperature was 700 °C and the calcining time was 5 hours, obtaining a catalyst D-I-1.

Comparative Example I-2

A catalyst was prepared as described in Example I-12, except that in the production of the carrier, the calcining temperature was 550 °C and the calcining time was 3 hours, and the amount of phosphoric acid used was adjusted to obtain a content of the P element in the carrier as shown in Table I-1, obtaining a catalyst D-I-2.

Test Example I-1

The elemental composition of the carriers and the catalysts were analyzed by plasma emission spectroscopy, the content of the element (ion) other than the carrier was expressed in the content of the element relative to 100g of the matrix, i.e. the carrier calculated on the basis of component(s) other than the doping element (e.g. calculated on the basis of $Al_2O_3$, when pseudo-boehmite was used as the source of the carrier); the carriers obtained above were characterized by probe adsorption spectroscopy (characterizing the proportion of L acid content to the total of L acid and B acid contents (i.e., L acid ratio)), $NH_3$-TPD, BET nitrogen adsorption-desorption, and the results are shown in Table I-1.

Table I-1 Properties of the carriers of the examples and comparative examples

| Example No. | Doping element | | Relative content, g | | Ammonia adsorption capacity of carrier, mmol/g | L acid ratio of carrier | Specific surface area of carrier, m$^2$/g | Pore volume of carrier, ml/g | Proportion of pore volume of pores with different pore diameters, % | | |
|---|---|---|---|---|---|---|---|---|---|---|---|
| | Species | Relative content, g | Active metal component | Metal promoter | | | | | < 7.5 nm | ≥ 7.5 nm and < 9 nm | > 27 nm |
| Ex. I-1 | B | 1 | Co 30 | 0 | 0.38 | 98 | 202 | 0.92 | 8 | 9.5 | 2 |
| Ex. I-2 | F | 1.12 | Ni 35 | Mo 2 | 0.39 | 96 | 178 | 0.86 | 10 | 14 | 1 |
| Ex. I-3 | P | 1.8 | Co 10 | Mn 3.1/Cu 2.9 | 0.45 | 95 | 163 | 0.79 | 13 | 15 | 1.2 |
| Ex. I-4 | S | 1.5 | Ni 25 | 0 | 0.43 | 99 | 188 | 0.88 | 7 | 10 | 2.9 |
| Ex. I-5 | S | 1.2 | Co 12/Ni 8 | Re 2 | 0.41 | 100 | 193 | 0.93 | 6 | 8.8 | 2.5 |
| Ex. I-6 | Se | 0.08 | Co 25 | 0 | 0.31 | 93 | 180 | 0.76 | 6 | 16 | 1.5 |
| Ex. I-7 | B | 0.25 | Co 20 | Ag 0.8 | 0.36 | 100 | 182 | 0.85 | 12 | 16 | 0.6 |
| Ex. I-8 | P+S | 0.3+0.2 | Ni 28 | Ce 0.84/Zn3.16 | 0.35 | 96 | 200 | 0.92 | 9 | 13 | 1.5 |
| Ex. I-9 | S | 0.8 | Co 20/Ni 10 | 0 | 0.39 | 99 | 153 | 0.73 | 13 | 19 | 4.1 |
| Ex. I-10 | P+F | 0.2+0.1 | Co 40 | La 2 | 0.33 | 100 | 129 | 0.55 | 18 | 18 | 1.8 |
| Ex. I-11 | S | 0.3 | Co5/Ni 3 | Re 3 | 0.31 | 100 | 176 | 0.87 | 8 | 15 | 2.3 |
| Ex. I-12 | P | 2.6 | Co 20 | Cu 5 | 0.56 | 98 | 192 | 0.79 | 15 | 16 | 0.5 |
| Ex. I-13 | B | 0.07 | Ni 30 | La 4/Zn 4 | 0.3 | 98 | 190 | 0.92 | 10 | 11 | 2.6 |
| Ex. I-14 | S | 2.5 | Co 12/Ni8 | Re 2 | 0.55 | 100 | 190 | 0.94 | 6 | 8.9 | 2.7 |
| Ex. I-15 | S | 1.2 | Co 12/Ni 8 | Re 2 | 0.42 | 91 | 199 | 0.96 | 7 | 10 | 2.4 |
| Ex. I-16 | S | 1.2 | Co 12/Ni 8 | Re 2 | 0.38 | 100 | 162 | 0.75 | 19 | 18 | 1.6 |
| Ex. I-17 | P+S | 0.3+0.2 | Ni 28 | Ce0.84/Zn 3.16 | 0.45 | 100 | 209 | 0.49 | 10 | 20 | 0.2 |
| Comp. Ex. I-1 | P | 2.6 | Co 20 | Cu 5 | 0.61 | 81 | 216 | 0.86 | 18 | 16 | 3.6 |

(continued)

| Example No. | Doping element | | Relative content, g | | Ammonia adsorption capacity of carrier, mmol/g | L acid ratio of carrier | Specific surface area of carrier, m²/g | Pore volume of carrier, ml/g | Proportion of pore volume of pores with different pore diameters, % | | |
|---|---|---|---|---|---|---|---|---|---|---|---|
| | Species | Relative content, g | Active metal component | Metal promoter | | | | | < 7.5 nm | ≥ 7.5 nm and < 9 nm | > 27 nm |
| Comp. Ex. I-2 | P | 0.5 | Co 20 | Cu 5 | 0.23 | 77 | 242 | 0.92 | 23 | 10 | 1.5 |

Test Example I-2

This test example illustrates a process for producing 1,6-hexanediamine by hydroamination of 1,6-hexanediol using the catalysts of the first type of embodiments of the present application.

100 mL of the catalysts obtained were respectively measured out, loaded into a fixed bed reactor, activated with hydrogen at 220 °C for 2 hours, then cooled to 168 °C, the pressure of the system was raised to 9.5 MPa using hydrogen, then ammonia was metered into the reaction system via a metering pump, preheated to 150 °C and then sent to the upper end of the reactor, heated and melted 1,6-hexanediol was fed into the upper end of the reactor via a metering pump, hydrogen was stably introduced via a gas mass flowmeter, wherein the molar ratio of hydrogen to ammonia and to 1,6-hexanediol was 3 : 12: 1, the liquid phase volume space velocity of 1,6-hexanediol was 0.45 $h^{-1}$, a catalytic amination reaction was conducted in the reactor, at a reaction temperature of 198 °C, and a reaction pressure of 9.5 MPa, the reaction solution was sampled and analyzed at a reaction time of 200 hours. The analysis results are shown in Table I-2.

The analysis of the sample was conducted by gas chromatography and was calibrated using a correction factor of a standard sample formulated.

The conversion and selectivity were calculated based on the molar content of each component in the reaction solution.

$$\text{Conversion of hexanediol} = 100\% - \frac{\text{Molar content of hexanediol}}{\text{Molar content of (hexanediol + hexanediamine + hexamethyleneimine + aminohexanol)} + \text{Molar content of dimers of amine} \times 2} \times 100\%$$

$$\text{Selectivity of hexanediamine} = \frac{\text{Molar content of hexanediamine}}{\text{Molar content of (hexanediamine + hexamethyleneimine + aminohexanol)} + \text{Molar content of dimers of amine} \times 2} \times 100\%$$

The selectivity of hexamethyleneimine was calculated by changing the numerator in the equation above for calculating the selectivity of hexanediamine to the molar content of hexamethyleneimine, the selectivity of aminohexanol was calculated by changing the numerator in the equation above for calculating the selectivity of hexanediamine to the molar content of aminohexanol, and so on, and the selectivity to "others" was calculated by changing the numerator in the equation above for calculating the selectivity of hexanediamine to the molar content of dimers of amine $\times 2$, the dimer of amine refers to the dimer of 1,6-hexanediamine (i.e. bis(hexamethylene) triamine, also known as N-(6-aminohexyl)-1,6-hexanediamine) and the dimer of 1,6-hexanediamine with hexamethyleneimine (i.e. N-(6-aminohexyl) hexamethyleneimine).

Table I-2 Test results for the catalysts of the examples and comparative examples

| Catalyst | Conversion, % | Selectively, % | | | |
| --- | --- | --- | --- | --- | --- |
| | | Hexanediamine | Hexamethyleneimine | Aminohexanol | Others |
| C-I-1 | 91 | 49.2 | 20.3 | 27.5 | 3 |
| C-I-2 | 92 | 50.1 | 19.2 | 27.8 | 2.9 |
| C-I-3 | 93 | 51 | 19.2 | 26.7 | 3.1 |
| C-I-4 | 91 | 48.9 | 20.1 | 27.8 | 3.2 |
| C-I-5 | 94 | 51.6 | 20.5 | 24.9 | 3 |
| C-I-6 | 91 | 48.8 | 21.2 | 26.8 | 3.2 |
| C-I-7 | 93 | 52.4 | 18.6 | 26.2 | 2.8 |
| C-I-8 | 94 | 52.6 | 18.5 | 25.8 | 3.1 |
| C-I-9 | 89 | 47.9 | 19.9 | 28.2 | 4 |
| C-I-10 | 84 | 45.3 | 21.1 | 29.1 | 4.5 |
| C-I-11 | 83 | 48.2 | 18.2 | 29.8 | 3.8 |
| C-I-12 | 90 | 46.8 | 19.3 | 29.7 | 4.2 |
| C-I-13 | 87 | 45.9 | 18.4 | 31.8 | 3.9 |
| C-I-14 | 83 | 46.8 | 19.6 | 28.7 | 4.9 |

(continued)

| Catalyst | Conversion, % | Selectively, % | | | |
|---|---|---|---|---|---|
| | | Hexanediamine | Hexamethyleneimine | Aminohexanol | Others |
| C-I-15 | 78 | 45.7 | 18.4 | 32.2 | 3.7 |
| C-I-16 | 80 | 44.9 | 19.8 | 32 | 3.3 |
| D-I-1 | 66 | 34.9 | 20.1 | 36.5 | 8.5 |
| D-I-2 | 69 | 30.8 | 22.3 | 32.7 | 14.2 |

As can be seen from the data in Table I-2, the catalyst of the present application has higher conversion and higher activity than the comparative catalysts, indicating that the catalyst of the present application provides a faster reaction rate.

Test Example I-3

This test example illustrates a process for producing 1,3-propanediamine by the hydroamination of 1,3-propanediol using the catalysts of the first type of embodiments of the present application.

100 mL of the catalyst C-I-3 obtained in Example I-3 was measured out, loaded into a fixed bed reactor, activated for 2 hours at 220 °C with hydrogen, then cooled to 165 °C, the pressure of the system was raised to 8.8 MPa using hydrogen, then ammonia was metered into the reaction system via a metering pump, preheated to 120 °C and then sent to the upper end of the reactor, 1,3-propanediol was fed into the upper end of the reactor via a metering pump, hydrogen was stably introduced with a gas mass flowmeter, wherein the molar ratio of hydrogen to ammonia and to 1,3-propanediol was 3 : 9 : 1, the liquid phase volume space velocity of 1,3-propanediol was 0.4 $h^{-1}$, a catalytic amination reaction was carried out in the reactor, and after the reaction became stable, the reaction solution was sampled and analyzed (the analysis conditions, and the methods for calculating the conversion and the selectivity are similar to those of Test Example I-2). The analysis results are shown in Table I-3.

Table I-3 Results of Test Example I-3

| Continuous reaction time | Conversion of 1,3-propanediol, % | Selectively, % | | |
|---|---|---|---|---|
| | | 1,3-propanediamine | 3-aminopropanol | Others |
| 200h | 75.0 | 84.1 | 14.0 | 1.9 |
| 1000h | 75.3 | 84.8 | 13.3 | 1.8 |

Test Example I-4

This test example illustrates a process for producing ethylamine by hydroamination of ethanol using a catalyst according to the first type of embodiments of the present application.

100 mL of the catalyst C-I-3 obtained in Example I-3 was measured out, loaded into a fixed bed reactor, activated with hydrogen at 220 °C for 2 hours, then cooled to 170 °C, the pressure of the system was raised to 1.8 MPa using hydrogen, then ammonia was metered into the reaction system via a metering pump, preheated to 125 °C, then sent to the upper end of the reactor, ethanol was fed into the upper end of the reactor via a metering pump, hydrogen was stably introduced via a gas mass flowmeter, wherein the molar ratio of hydrogen to ammonia and to ethanol was 3 : 5 : 1, the liquid phase volume space velocity of ethanol was 0.6$h^{-1}$, a catalytic amination reaction was conducted in the reactor, at a reaction temperature of 180 °C, and a reaction pressure of 1.8 MPa, and after the reaction became stable, the reaction solution was sampled and analyzed (the analysis conditions, and the methods for calculating the conversion and the selectivity are similar to those of Test Example I-2). The analysis results are shown in Table I-4.

Table I-4 Results of Test Example I-4

| Continuous reaction time | Conversion of Ethanol, % | Selectively, % | | | |
|---|---|---|---|---|---|
| | | Monoethylamine | Diethylamine | Triethylamine | Others |
| 200h | 98.95 | 27.4 | 47.3 | 24.8 | 0.5 |

(continued)

| Continuous reaction time | Conversion of Ethanol, % | Selectively, % | | | |
|---|---|---|---|---|---|
| | | Monoethylamine | Diethylamine | Triethylamine | Others |
| 1000h | 98.78 | 27.9 | 47.1 | 24.4 | 0.5 |

The catalysts D-I-1 and D-I-2 were tested under the same process conditions, it was found from the analysis results that more component of "others" was produced using the comparative catalysts D-I-1 and D-I-2, of which the selectivity was 0.8% and 1.2%, respectively, and it was found in a long-period evaluation (for a period 200 h) that the deactivation rate of the catalysts D-I-1 and D-I-2 was relatively high. After 200 hours of evaluation, the carbon deposition of the catalyst C-I-3 was different from that of the catalysts D-I-1 and D-I-2, with the carbon deposition of the latter two being obviously more than that of the catalyst C-I-3, the reduction of the specific surface area and the pore volume of the catalyst C-I-3 was not obvious (less than 2%), while the reduction of the specific surface area of the catalysts D-I-1 and D-I-2 were respectively 7% and 9%, and the reduction of the pore volume of the catalysts D-I-1 and D-I-2 were respectively 9% and 10%, indicating that the pore channel was blocked by the carbon deposition.

Example series II

The second type of embodiments of the present application are further illustrated hereinbelow in detail with reference to the examples of Example series II. In the following examples of Example series II, the pseudo-boehmite powder had a (Al$_2$O$_3$) content on a dry basis of 70 wt%, and the silica sol was purchased from Qingdao Ocean Chemical Co., Ltd., under a trade name of JN-40.

Example II-1

Pseudo-boehmite powder (with a specific surface area of 380 m$^2$/g, a pore volume of 1.09 ml/g) was kneaded with a dilute acid aqueous solution containing 5 vol% of nitric acid and 3.5 vol% of phosphoric acid, and extruded into strips, dried at 120 °C for 10 h, and then calcined at 850 °C for 4h to obtain a desired carrier, of which the detailed parameters are shown in Table II-1.
141.56 g of nickel nitrate hexahydrate (industrial grade, with a purity of 98%) was dissolved in water to obtain a 166mL solution, the pH of the solution was adjusted to 4.3, the solution was loaded onto 100 g of the alumina carrier obtained by spray impregnation in two times, dried at 120 °C for 8 hours after each time of spray impregnation, then calcined for 4 hours at 400 °C, then reduced with hydrogen while gradually increasing the temperature at a rate of 20 °C/h, and finally reduced for 3 hours at 440 °C, to obtain a catalyst C-II-1.

Example II-2

Pseudo-boehmite powder (with a specific surface area of 400 m$^2$/g, and a pore volume of 1.15 ml/g) was kneaded with a dilute acid aqueous solution containing 5 vol% of nitric acid and 3 vol% of boric acid, silica sol was added during the kneading process, extruded into strips, dried at 120 °C for 12 hours, and then calcined at 900 °C for 4 hours to obtain a desired carrier, of which the detailed parameters are shown in Table II-1, and the pseudo-boehmite powder and the silica sol were used in such an amount that the weight ratio of Al$_2$O$_3$ to SiO$_2$ in the carrier was 4:1.
176.38 g of cobalt nitrate hexahydrate (industrial grade, with a purity of 98%) was dissolved in water to obtain a 160 mL solution, the pH of the solution was adjusted to 3.9, the solution was loaded onto 100 g of the carrier obtained by wet impregnation in two steps, dried at 120 °C for 6 hours after each wet impregnation, then calcined at 390 °C for 4 hours, then reduced with hydrogen while gradually increasing the temperature at a rate of 20 °C/h, and finally reduced at 460 °C for 5 hours to obtain a catalyst C-II-2.

Example II-3

Pseudo-boehmite powder (with a specific surface area of 395 m$^2$/g, a pore volume of 1.19 ml/g) was kneaded with a dilute nitric acid of 2 vol% and a dilute acid aqueous solution containing 2 vol% of sulfuric acid, extruded into dentate spheres with a diameter of 4mm, dried at 150 °C for 8h, and then calcined at 950 °C for 3.5h to obtain a desired carrier, of which the detailed parameters are shown in Table II-1.
75.59 g of cobalt nitrate hexahydrate (industrial grade, with a purity of 98%) and 27.36 g of a 50wt% manganese nitrate solution were dissolved in water to obtain a 138 mL solution, the pH of the solution was adjusted to 4.4, the solution was loaded onto 100 g of the carrier obtained by spray impregnation in two times, dried at 120 °C for 2 hours after each time of spray impregnation, then calcined at 400 °C for 4 hours, then reduced with hydrogen while gradually increasing the temperature at a rate of 20 °C/h, and finally reduced at 400 °C for 8 hours, to obtain a catalyst C-II-3.

Example II-4

Pseudo-boehmite powder (with a specific surface area of 395 m$^2$/g, a pore volume of 1.05 ml/g) was kneaded with a dilute acid aqueous solution containing 5 vol% of nitric acid and 3 vol% of boric acid, and extruded into strips, dried at 120 °C for 18 h, and then calcined at 1010 °C for 4.5h to obtain a desired carrier, of which the detailed parameters are shown in Table II-1.
60.47 g of cobalt nitrate hexahydrate (industrial grade, with a purity of 98%), 50.56 g of nickel nitrate hexahydrate (industrial grade, with a purity of 98%) and 0.73g of ammonium perrhenate (with a purity of 99%) were dissolved in water to obtain a 120mL solution, the pH of the solution was adjusted to 4.3, and the solution was loaded onto 100 g of the carrier obtained by isovolumetric impregnation in two times, dried at 120 °C for 5 hours after each time of impregnation, then calcined at 390 °C for 4 hours, then reduced with hydrogen while gradually increasing the temperature at a rate of 20 °C/h, and finally reduced at 450 °C for 6 hours to obtain a catalyst C-II-4.

Example II-5

Pseudo-boehmite powder (with a specific surface area of 382 m$^2$/g, a pore volume of 1.09 ml/g) was kneaded with a dilute acid aqueous solution containing 5 vol% of nitric acid and 2 vol% of sulfuric acid, extruded into dentate spheres with a diameter of 4 mm, dried at 120 °C for 10h, and then calcined at 820 °C for 10h to obtain a desired carrier, of which the detailed parameters are shown in Table II-1.
192.12 g of nickel nitrate hexahydrate (industrial grade, with a purity of 98%) and 3.94 g of silver nitrate were dissolved in water to obtain a 182 mL solution, the pH of the solution was adjusted to 4.6, the solution was loaded onto 100 g of the carrier obtained by spray impregnation in two times, dried at 120 °C for 4 hours after each time of spray impregnation, then calcined for 4 hours at 400 °C, then reduced with hydrogen while gradually increasing the temperature at a rate of 20 °C/h, and finally reduced for 3 hours at 480 °C, to obtain a catalyst C-II-5.

Example II-6

Pseudo-boehmite powder (with a specific surface area of 375 m$^2$/g, a pore volume of 1.19 ml/g) was kneaded with a dilute acid aqueous solution containing 5 vol% of nitric acid and 3.5 vol% of phosphoric acid, extruded into dentate spheres, dried for 15 h at 120 °C, and then calcined for 5.5h at 880 °C to obtain a desired carrier, of which the detailed parameters are shown in Table II-1.
100.79 g of cobalt nitrate hexahydrate (industrial grade, with a purity of 98%) and 14.56 g of zinc nitrate (analytically pure) were dissolved in water to obtain a 154mL solution, the pH of the solution was adjusted to 5.1, and the solution was loaded onto 100 g of the carrier obtained by spray impregnation in two times, dried at 120 °C for 6 hours after each time of spray impregnation, then calcined at 400 °C for 4 hours, then reduced with hydrogen while gradually increasing the temperature at a rate of 20 °C/h, and finally reduced at 450 °C for 3 hours to obtain a catalyst C-II-6.

Example II-7

Pseudo-boehmite powder (with a specific surface area of 342 m$^2$/g, a pore volume of 0.78 ml/g) was kneaded with a dilute acid aqueous solution containing 5 vol% of nitric acid and 3.5 vol% of phosphoric acid, and extruded into dentate spheres, dried at 120 °C for 10 h, and then calcined at 930 °C for 4.5h to obtain a desired carrier, of which the detailed parameters are shown in Table II-1.
120.94 g of cobalt nitrate hexahydrate (industrial grade, with a purity of 98%) and 9.28g of bismuth nitrate pentahydrate (analytically pure) were dissolved in water to obtain a 102mL solution, the pH of the solution was adjusted to 4.9, and the solution was loaded onto 100 g of the carrier obtained by wet impregnation in two times, dried at 120 °C for 4 hours after each time of wet impregnation, then calcined at 400 °C for 4 hours, then reduced with hydrogen while gradually increasing the temperature at a rate of 20 °C/h, and finally reduced at 430 °C for 4 hours to obtain a catalyst C-II-7.

Example II-8

Pseudo-boehmite powder (with a specific surface area of 280 m$^2$/g, a pore volume of 0.89 ml/g) was kneaded with a dilute acid aqueous solution containing 5 vol% of nitric acid, 2 vol% of hydrofluoric acid and 0.5 wt% of selenium nitrate, extruded into dentate spheres, dried at 120 °C for 20 h, and then calcined at 980 °C for 12h to obtain a desired carrier, of which the detailed parameters are shown in Table II-1.

202.23 g of nickel nitrate hexahydrate (industrial grade, with a purity of 98%) and 13.65g of zinc nitrate hexahydrate (analytically pure) were dissolved in water to obtain a 128 mL solution, the pH of the solution was adjusted to 3.7, and the solution was loaded onto 100 g of the carrier obtained by spray impregnation in two times, dried at 120 °C for 10 hours after each time of spray impregnation, then calcined at 400 °C for 4 hours, then reduced with hydrogen while gradually increasing the temperature at a rate of 20 °C/h, and finally reduced at 410 °C for 6 hours to obtain a catalyst C-II-8.

Example II-9

Pseudo-boehmite powder (with a specific surface area of 380 m$^2$/g, a pore volume of 1.09 ml/g) was kneaded with a dilute acid aqueous solution containing 5 vol% of nitric acid and 3.5 vol% of phosphoric acid, silica sol was added during the kneading process, extruded into strips, dried at 120 °C for 10 hours, and then calcined at 850 °C to obtain a desired carrier, of which the detailed parameters are shown in Table II-1, and the pseudo-boehmite powder and the silica sol were used in such an amount that the weight ratio of $Al_2O_3$ to $SiO_2$ in the carrier was 9:1.

75.59 g of cobalt nitrate hexahydrate (industrial grade, with a purity of 98%), 50.56 g of nickel nitrate hexahydrate (industrial grade, with a purity of 98%) and 29.31 g of a 50wt% manganese nitrate solution were dissolved in water to obtain a 106 mL solution, the pH of the solution was adjusted to 4.0, the solution was loaded onto 100 g of the alumina carrier obtained by spray impregnation in two times, dried at 120 °C for 8 hours after each time of spray impregnation, then calcined at 400 °C for 4 hours, then reduced with hydrogen while gradually increasing the temperature at a rate of 20 °C/h, and finally reduced at 440 °C for 5 hours, to obtain a catalyst C-II-9.

Example II-10

Pseudo-boehmite powder (with a specific surface area of 369 m$^2$/g, a pore volume of 1.15 ml/g) was kneaded with a dilute acid aqueous solution containing 5 vol% of nitric acid and 3 vol% of sulfuric acid, and extruded into strips, dried at 120 °C for 15 h, and then calcined at 1010 °C for 6.5h to obtain a desired carrier, of which the detailed parameters are shown in Table II-1.

35.39 g of nickel nitrate hexahydrate (industrial grade, with a purity of 98%) and 2.18 g of ammonium perrhenate (with a purity of 99%) were dissolved in water to obtain a 164 mL solution, the pH of the solution was adjusted to 5.3, and the solution was loaded onto 100 g of the carrier obtained by spray impregnation in two times, dried at 120 °C for 6 hours after each time of spray impregnation, calcined at 350 °C for 4 hours, then reduced with hydrogen while gradually increasing the temperature at a rate of 20 °C/h, and finally reduced at 420 °C for 5 hours to obtain a catalyst C-II-10.

Comparative Example II-1

A catalyst D-II-1 was prepared as described in Example II-3, except that sulfuric acid was not added during the production of the carrier and the pH of the impregnation solution was not adjusted (with an autogenous pH of about 7).

Comparative Example II-2

A catalyst D-II-2 was prepared as described in Example II-3, except that sulfuric acid was used in such an amount that the S content in the carrier was as shown in Table II-1, and the pH of the impregnation solution was not adjusted (with an autogenous pH of about 7).

Comparative Example II-3

A catalyst was prepared as described in Example II-3, except that sulfuric acid was used in such an amount that the S content in the carrier was as shown in Table II-1, in the production of the carrier the calcining temperature was 650 °C and the calcining time was 4 hours, and the pH of the impregnation solution was not adjusted (with an autogenous pH of about 7), to obtain a catalyst D-II-3.

Test Example II-1

The elemental composition of the carriers and the catalysts were analyzed by plasma emission spectroscopy, the content of the element (ion) other than the carrier was expressed in the content of the element relative to 100g of the matrix; the carriers obtained above were characterized by probe adsorption spectroscopy, NH$_3$-TPD, BET nitrogen adsorption-desorption, and the grain size of the active metal component in the catalyst was measured by XRD, and the results are shown in Table II-1.

Table II-1 Properties of the carriers of the examples and comparative examples

| Example No. | Doping element | | Relative content, g | | Grain size, nm | Ammonia adsorption capacity, mmol/g | L acid ratio | Specific surface area, m$^2$/g | Pore volume, ml/g | Isoelectric point |
|---|---|---|---|---|---|---|---|---|---|---|
| | Species | Relative content, g | Active metal component | Metal promoter | | | | | | |
| Ex. II-1 | P | 0.88 | Ni 28 | / | 8 | 0.35 | 92 | 189 | 0.88 | 4.2 |
| Ex. II-2 | B | 1.58 | Co 35 | / | 6.9 | 0.4 | 95 | 168 | 0.85 | 3.8 |
| Ex. II-3 | S | 0.4 | Co 15 | Mn 4.2 | 6.3 | 0.35 | 90 | 155 | 0.74 | 4.3 |
| Ex. II-4 | B | 3.41 | Co 12, Ni 10 | Re 0.5 | 5.5 | 0.42 | 100 | 138 | 0.65 | 4.2 |
| Ex. II-5 | S | 0.97 | Ni 38 | Ag 2.5 | 7.2 | 0.35 | 97 | 185 | 0.96 | 4.5 |
| Ex. II-6 | S | 4 | Co 20 | Zn 3.2 | 7 | 0.46 | 92 | 176 | 0.82 | 5.0 |
| Ex. II-7 | P | 0.08 | Co 24 | Bi 4 | 5.2 | 0.3 | 96 | 169 | 0.56 | 4.8 |
| Ex. II-8 | F + Se | 0.05 | Ni 40 | Zn 3 | 8.3 | 0.25 | 90 | 150 | 0.69 | 3.6 |
| Ex. II-9 | S | 5 | Ni 10, Co15 | Mn 4.5 | 9.6 | 0.64 | 88 | 125 | 0.58 | 3.9 |
| Ex. II-10 | P | 4.21 | Ni 7 | Re 1.5 | 8.8 | 0.56 | 95 | 116 | 0.87 | 5.2 |
| Comp. Ex. II-1 | / | 0 | Co 15 | Mn 4.2 | 20.2 | 0.72 | 80 | 212 | 0.91 | 4.0 |
| Comp. Ex. II-2 | S | 5.5 | Co 15 | Mn 4.2 | 11.4 | 0.75 | 65 | 227 | 0.99 | 4.5 |
| Comp. Ex. II-3 | S | 0.2 | Co 15 | Mn 4.2 | 9.6 | 0.23 | 72 | 246 | 1.03 | 4.2 |

33

Test Example II-2

This test example illustrates a process for producing 1,6-hexanediamine by hydroamination of 1,6-hexanediol using the catalyst of the second type of embodiments of the present application.

100 mL of the catalysts obtained in the examples were respectively measured out, loaded into a fixed bed reactor, activated with hydrogen at 250 °C for 4 hours, then cooled to 160 °C, the pressure of the system was raised to 11 MPa using hydrogen, then ammonia was metered into the reaction system via a metering pump, preheated to 170 °C, then sent to the upper end of the reactor, heated and melted 1,6-hexanediol was fed into the upper end of the reactor via a metering pump, and hydrogen was stably introduced via a gas mass flowmeter, wherein the molar ratio of hydrogen to ammonia and to 1,6-hexanediol was 4 : 10 : 1, the liquid phase volume space velocity of 1,6-hexanediol was 0.4 $h^{-1}$, a catalytic amination reaction was conducted in the reactor, at a reaction temperature of 185 °C, and a reaction pressure of 11 MPa, the reaction solution was sampled and analyzed at a reaction time of 20 hours, and the analysis results are listed in the Table II-2.

The analysis of the sample was conducted by gas chromatography and was calibrated using a correction factor of a standard sample formulated.

The conversion and selectivity were calculated based on the molar content of each component in the reaction solution, and the calculation methods were the same as described in Test Example I-2.

Table II-2 Test results for the catalysts of the examples and comparative examples

| Catalyst | Composition of catalyst | Conversion , % | Selectively, % | | | |
|---|---|---|---|---|---|---|
| | | | Hexanediamine | Hexamethyleneimine | Aminohexanol | Others |
| C-II-1 | $Co/Al_2O_3$ | 90.7 | 50.4 | 20.5 | 26.0 | 3.1 |
| C-II-2 | $Ni/Al_2O_3\text{-}SiO_2$ | 91.2 | 46.9 | 21.1 | 28.6 | 3.4 |
| C-II-3 | $Co\text{-}Mn/Al_2O_3$ | 92.3 | 51.2 | 19.3 | 26.3 | 3.2 |
| C-II-4 | $Co\text{-}Ni\text{-}Re/Al_2O_3$ | 90.9 | 50.8 | 25.5 | 20.8 | 2.9 |
| C-II-5 | $Ni\text{-}Ag/Al_2O_3$ | 91.5 | 52.8 | 16.8 | 27.6 | 2.8 |
| C-II-6 | $Co\text{-}Zn/Al_2O_3$ | 91.9 | 52.1 | 16.9 | 28.2 | 2.8 |
| C-II-7 | $Co\text{-}Bi/Al_2O_3$ | 90.6 | 49.7 | 19.8 | 27.5 | 3.0 |
| C-II-8 | $Ni\text{-}Zn/Al_2O_3$ | 89.4 | 47.7 | 20.3 | 28.3 | 3.7 |
| C-II-9 | $Co\text{-}Ni\text{-}Mn/Al_2O_3\text{-}SiO_2$ | 88.9 | 48.2 | 18.9 | 29.4 | 3.5 |
| C-II-10 | $Co\text{-}Re/Al_2O_3$ | 88.5 | 47.9 | 24.6 | 23.6 | 3.9 |
| D-II-1 | $Co\text{-}Mn/Al_2O_3$ | 72.6 | 44.3 | 28.9 | 21.0 | 5.8 |
| D-II-2 | $Co\text{-}Mn/Al_2O_3$ | 85.2 | 40.6 | 29.7 | 21.9 | 7.8 |
| D-II-3 | $Co\text{-}Mn/Al_2O_3$ | 82.1 | 37.6 | 30.2 | 22.8 | 9.4 |

As can be seen from the data in Table II-2, the catalyst of the present application has higher conversion and higher activity than the comparative catalysts, indicating that the catalyst of the present application provides a faster reaction rate.

Test Example II-3

This test example illustrates a process for producing ethylenediamine by hydroamination of ethanolamine using the catalyst of the second type of embodiments of the present application.

100 mL of the catalyst C-II-3 obtained in Example II-3 was measured out, loaded into a fixed bed reactor, activated for 2 hours at 250 °C with hydrogen, then cooled to 168 °C, the pressure of the system was raised to 8 MPa using hydrogen, then ammonia was metered into the reaction system via a metering pump, preheated to 165 °C and then sent to the upper end of the reactor, ethanolamine was fed into the upper end of the reactor via a metering pump, hydrogen was stably introduced via a gas mass flowmeter, wherein the molar ratio of hydrogen to ammonia and to ethanolamine was 3 : 10 : 1, the liquid phase volume space velocity of ethanolamine was 0.75 h$^{-1}$, a catalytic amination was conducted in the reactor, at a reaction temperature of 205 °C and a reaction pressure of 8 MPa, and after the reaction became stable, the reaction solution was sampled and analyzed (the analysis conditions, and the methods for calculating the conversion and the selectivity are similar to those of Test Example II-2). The analysis results are shown in Table II-3.

Table II-3 Results of Test Example II-3

| Continuous reaction time | Conversion of ethanolamine, % | Selectively, % | | | | | | |
|---|---|---|---|---|---|---|---|---|
| | | Ethylenediamine | Piperazine | Diethylenetriamine | Hydroxyethyl ethylenediamine | N-aminoethylpiperazine | N-hydroxyethyl piperazine | Others |
| 20 | 90.7 | 58.20 | 23.70 | 7.45 | 9.55 | 0.50 | 0.40 | 0.20 |
| 500 | 91.0 | 58.30 | 23.80 | 7.35 | 9.40 | 0.55 | 0.40 | 0.20 |

The catalysts D-II-1 to D-II-4 were tested under the same process conditions, it was found from the analysis results that more component of "others" was produced using the comparative catalysts D-II-1 to D-II-4, and it was found in a long-period evaluation that the catalysts D-II-1 to D-II-4 had a reduced selectivity and conversion and a relatively high deactivation rate. After 500 hours of evaluation, the carbon deposition of the catalyst C-II-3 was different from that of the catalysts D-II-1 to D-II-4, with the carbon deposition of the latter being obviously more than that of the catalyst C-II-3, the reduction of the specific surface area and the pore volume of the catalyst C-II-3 was not obvious (less than 2%), while the reduction of the specific surface area of the catalysts D-II-1 to D-II-4 was respectively 19%, 16%, 18% and 16%, and the reduction of the pore volume of the catalysts D-II-1 to D-II-4 was respectively 20%, 18%, 19% and 18%, indicating that the carbon deposition of those catalyst was relatively large, causing a blockage of the pore channel.

Example series III

The third type of embodiments of the present application are further illustrated in detail with reference to the examples of Example series III. In the following examples of Example series III, the pseudo-boehmite powder used had a ($Al_2O_3$) content on a dry basis of 72 wt%; the silica sol was purchased from Qingdao Ocean Chemical Co., Ltd, under a trade name of JN-40.

Example III-1

Pseudo-boehmite powder (with a specific surface area of 315 $m^2$/g, a pore volume of 0.96 ml/g, and a content of the P element in the powder being 3.6 g per 100 g of alumina) prepared by aluminum sulfate method was kneaded with a dilute acid aqueous solution containing 5 vol% of nitric acid, extruded into strips with a diameter of 5 mm, cut to a length of 4 mm, dried at 120 °C for 8 hours, and calcined at 760 °C for 5 hours to obtain a carrier.
186.5 g of cobalt nitrate hexahydrate (industrial grade, with a purity of 98%) was dissolved in water to obtain a 142mL solution, the cobalt nitrate solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst C-III-1.

Example III-2

Pseudo-boehmite powder (with a specific surface area of 295 $m^2$/g, a pore volume of 0.93 ml/g, and a content of the B element in the powder being 0.8 g per 100 g of alumina) prepared by aluminum sulfate method was kneaded with a dilute acid aqueous solution containing 5 vol% of nitric acid, mixed uniformly, extruded into granules of clover shape with a thickness of 3 mm, dried at 100 °C for 15 h, and calcined at 930 °C for 4h to obtain a carrier.
151.7 g of nickel nitrate hexahydrate (industrial grade, with a purity of 98%) was dissolved in water to obtain a 158mL solution, the nickel nitrate solution was loaded onto 100 g of the carrier obtained by spray impregnation in two times, dried at 100 °C for 8 hours after each time of spray impregnation, then calcined at 390 °C for 4 hours, then reduced with hydrogen while gradually increasing the temperature at a rate of 20 °C/h, and finally reduced at 440 °C for 3 hours, to obtain a catalyst C-III-2.

Example III-3

Pseudo-boehmite powder (with a specific surface area of 278 $m^2$/g, a pore volume of 0.85 ml/g, and a content of the S element in the powder being 0.4 g per 100 g of alumina) prepared by aluminum sulfate method was kneaded with a dilute acid aqueous solution containing 3.5 vol% of nitric acid, silica sol was added during the kneading process, mixed uniformly, extruded into dentate spheres with a diameter of 4 mm, dried at 150 °C for 6h, calcined at 980 °C for 3h to obtain a carrier, and the amount of silica sol used was adjusted to obtain a mass ratio of alumina to silica in the carrier of 4 : 1.
68.92 g of cobalt nitrate hexahydrate (industrial grade, with a purity of 98%) was dissolved into water to obtain a 112mL solution, the cobalt nitrate solution was loaded onto 100 g of the alumina carrier obtained by spray impregnation in two times, dried at 110 °C for 8 hours after each time of spray impregnation, then calcined for 4 hours at 390 °C, then reduced with hydrogen while gradually increasing the temperature at a rate of 20 °C/h, and finally reduced for 2 hours at 460 °C, to obtain a catalyst C-III-3.

Example III-4

Pseudo-boehmite powder (with a specific surface area of 325 $m^2$/g, a pore volume of 1.12 ml/g, and a content of the F element in the powder being 2.2 g per 100 g of alumina) prepared by aluminum sulfate method was uniformly mixed with molecular sieve powder (ZSM-5, Catalyst Plant of Nankai University, $SiO_2/Al_2O_3 = 45$ (molar ratio)), kneaded with a dilute acid aqueous solution containing 5vol% of nitric acid, extruded into strips with a diameter of 5 mm, cut to a length of 4 mm, dried at 80 °C for 20 h, and calcined at 730 °C for 8h to obtain a desired carrier, wherein the amount of the ZSM-5 molecular sieve powder was adjusted so that the content of alumina derived from the pseudo-boehmite in the carrier accounted for 90% of the total mass of the carrier.

126 g of cobalt nitrate hexahydrate (industrial grade, with a purity of 98%) was dissolved in water to obtain a 202mL solution, the cobalt nitrate solution was loaded onto 100 g of the alumina carrier obtained by spray impregnation in two times, dried at 90 °C for 20 hours after each time of spray impregnation, then calcined at 400 °C for 4 hours, then reduced with hydrogen while gradually increasing the temperature at a rate of 20 °C/h, and finally reduced at 430 °C for 3 hours to obtain a catalyst C-III-4.

Example III-5

Pseudo-boehmite powder (with a specific surface area of 260 m$^2$/g, a pore volume of 0.82 ml/g, and a content of the P element in the powder being 0.6 g per 100 g of alumina) prepared by aluminum sulfate method was kneaded with a dilute acid aqueous solution containing nitric acid and boric acid, extruded into granules of clover shape with a thickness of 3 mm, dried for 8 hours at 100 °C, and then calcined for 3 hours at 1005 °C to obtain a desired carrier, wherein the amount of boric acid used was 2.29g relative to 100g of the pseudo-boehmite powder used calculated as $Al_2O_3$, and the nitric acid concentration of the dilute acid aqueous solution was 5 vol%.

75.6 g of cobalt nitrate hexahydrate (industrial grade, with a purity of 98%) was dissolved into water to obtain a 122 mL solution, the cobalt nitrate solution was loaded onto 100 g of the carrier obtained by spray impregnation in two times, dried at 120 °C for 4 hours after each time of spray impregnation, calcined at 390 °C for 4 hours, then reduced with hydrogen while gradually increasing the temperature at a rate of 20 °C/h, and finally reduced at 440 °C for 3 hours to obtain a catalyst C-III-5.

Example III-6

Pseudo-boehmite powder (with a specific surface area of 345 m$^2$/g, a pore volume of 1.09 ml/g, and a content of the B element in the powder being 0.02 g per 100 g of alumina) prepared by aluminum sulfate method was kneaded with a dilute acid aqueous solution containing nitric acid and sulfuric acid, extruded into dentate spheres with a diameter of 4 mm, dried at 120 °C for 10 h, and calcined at 750 °C for 12 h to obtain a desired carrier, wherein the amount of sulfuric acid used was 0.24g, per 100g of the pseudo-boehmite powder used, calculated as $Al_2O_3$, and the nitric acid concentration in the dilute acid aqueous solution was 5 vol%.

126.4 g of nickel nitrate hexahydrate (industrial grade, with a purity of 98%) was dissolved into water to obtain a 184 mL solution, the nickel nitrate solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst C-III-6.

Example III-7

Pseudo-boehmite powder (with a specific surface area of 320 m$^2$/g, a pore volume of 0.93 ml/g, and a content of the S element in the powder being 0.1 g per 100 g of alumina) prepared by aluminum sulfate method was kneaded with a dilute acid aqueous solution containing nitric acid and phosphoric acid, extruded into dentate spheres with a diameter of 4 mm, dried at 100 °C for 15 h, and calcined at 780 °C for 10 h to obtain a desired carrier, wherein the amount of phosphoric acid used was 6.01g per 100g of the pseudo-boehmite powder used, calculated as $Al_2O_3$. The concentration of nitric acid in the dilute acid aqueous solution was 5 vol%.

296.85 g of cobalt nitrate hexahydrate (industrial grade, with a purity of 98%) was dissolved into water to obtain a 138 mL solution, the cobalt nitrate solution was loaded onto 100 g of the alumina carrier obtained by spray impregnation in two times, dried for 5 hours at 120 °C after each time of spray impregnation, then calcined for 4 hours at 400 °C, then reduced with hydrogen while gradually increasing the temperature at a rate of 20 °C/h, and finally reduced for 3 hours at 450 °C, to obtain a catalyst C-III-7.

Example III-8

Pseudo-boehmite powder (with a specific surface area of 295 m$^2$/g, a pore volume of 0.88 ml/g, and a content of the S element in the powder being 0.2 g per 100 g of alumina) prepared by aluminum sulfate method was kneaded with a dilute acid aqueous solution containing 5 vol% of nitric acid, extruded into dentate spheres with a diameter of 4 mm, dried at 90 °C for 18 h, and calcined at 810 °C for 8h to obtain a desired carrier.

100.8 g of cobalt nitrate hexahydrate (industrial grade, with a purity of 98%) was dissolved in water to obtain a 134mL solution, the solution was loaded onto 100 g of the carrier obtained by spray impregnation in two times, dried at 100 °C for 8 hours after each time of spray impregnation, then calcined for 4 hours at 400 °C, then reduced with hydrogen while gradually increasing the temperature at a rate of 20 °C/h, and finally reduced for 3 hours at 420 °C, to obtain a catalyst C-III-8.

Example III-9

Pseudo-boehmite powder (with a specific surface area of 340 $m^2$/g, a pore volume of 1.18 ml/g, and a content of the F element in the powder being 0.12 g per 100 g of alumina) prepared by aluminum sulfate method was kneaded with a dilute acid aqueous solution containing 5 vol% of nitric acid, silica sol was added during the kneading process, extruded into cylindrical bars with a diameter of 3 mm, dried at 100 °C for 16 h, calcined at 850 °C for 6h to obtain a desired carrier, and the amount of silica sol used was adjusted so that the molar ratio of alumina to silica in the carrier was 73: 27.

176.4 g of cobalt nitrate hexahydrate (industrial grade, with a purity of 98%) and 1.3 g of silver nitrate (analytically pure) were dissolved in water to obtain a 178 mL solution, and the solution was loaded onto 100 g of the carrier obtained above by spray impregnation in two times, dried at 120 °C for 4 hours after each time of spray impregnation, then calcined at 360 °C for 4 hours, then reduced with hydrogen while gradually increasing the temperature at a rate of 20 °C/h, and finally reduced at 450 °C for 3 hours, to obtain a catalyst C-III-9.

Example III-10

Pseudo-boehmite powder (with a specific surface area of 310 $m^2$/g, and a pore volume of 0.92 ml/g, and a content of the S element in the powder being 1.4 g per 100 g of alumina) prepared by aluminum sulfate method was kneaded with a dilute acid aqueous solution containing 5 vol% of nitric acid, extruded into strips with a diameter of 5 mm, dried at 100 °C for 12 h, and calcined at 760 °C for 10h to obtain a desired carrier.

126 g of cobalt nitrate hexahydrate (industrial grade, with a purity of 98%) and 13.7 g of zinc nitrate hexahydrate (analytically pure) were dissolved in water to obtain a 172 mL solution, the solution was loaded onto 100 g of the carrier obtained above by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 420 °C for 3 hours to obtain a catalyst C-III-10.

Example III-11

Pseudo-boehmite powder (with a specific surface area of 305m $^2$/g, a pore volume of 0.94 ml/g, and a content of the P element in the powder being 1.55 g per 100 g of alumina) prepared by aluminum sulfate method was kneaded with a dilute acid aqueous solution containing 5 vol% of nitric acid, extruded into strips with a diameter of 5 mm, dried at 120 °C for 6h, and calcined at 860 °C for 5h to obtain a desired carrier.

141.1 g of cobalt nitrate hexahydrate (industrial grade, with a purity of 98%) and 19.5 g of a 50% manganese nitrate aqueous solution were dissolved in water to obtain a 168mL solution, the solution was loaded onto 100 g of the carrier obtained above by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 420 °C for 3 hours to obtain a catalyst C-III-11.

Comparative Example III-1

A catalyst D-III-1 was prepared as described in Example III-8, except that pseudo-boehmite powders having different contents of S element were used so that the content of S element was as shown in Table III-1, and in the production of the carrier the calcining temperature was 700 °C and the calcining time was 4 hours.

Comparative Example III-2

A D-III-2 catalyst was prepared as described in Example III-8, except that a pseudo-boehmite powder containing no doping element (with a specific surface area of 224 $m^2$/g, a pore volume of 0.95 ml/g) was used.

Comparative Example III-3

A catalyst D-III-3 was prepared as described in Example III-8, except that pseudo-boehmite powders having different contents of S element were used so that the content of S element was as shown in Table III-1, and in the production of the carrier the calcining temperature was 500 °C and the calcining time was 4 hours.

Test Example III-1

The elemental composition of the carriers and the catalysts was analyzed by plasma emission spectroscopy, wherein the contents of the doping element, the active metal component and the metal promoter were expressed by weight relative to 100g of the matrix; the carriers obtained above were characterized by probe adsorption spectroscopy and BET nitrogen adsorption-desorption, and the results are shown in Table III-1.

Table III-1 Properties of the carriers of the examples and comparative examples

| Example No. | Doping element | | Relative content, g | | Proportion of pores having a pore diameter < 7nm, (%) | Proportion of pores having a pore diameter of 7-27 nm (%) | Specific surface area, m$^2$/g | Pore volume, ml/g | L acid ratio, % |
|---|---|---|---|---|---|---|---|---|---|
| | Species | Relative content, g | Active metal component | Metal promoter | | | | | |
| Ex. III-1 | P | 3.6 | Co 37 | - | 7 | 68 | 150 | 0.71 | 85 |
| Ex. III-2 | B | 0.8 | Ni 30 | - | 5 | 66 | 158 | 0.79 | 92 |
| Ex. III-3 | S | 0.32 | Co 12 | - | 8 | 70 | 152 | 0.58 | 95 |
| Ex. III-4 | F | 1.98 | Co 25 | - | 2 | 66 | 180 | 1.01 | 86 |
| Ex. III-5 | P+B | 0.6+0.4 | Co 15 | - | 9 | 73 | 132 | 0.61 | 98 |
| Ex. III-6 | B+S | 0.02+0.08 | Ni 25 | - | 3 | 65 | 195 | 0.92 | 88 |
| Ex. III-7 | S+P | 0.1+1.9 | Co 37 | - | 3 | 74 | 169 | 0.69 | 88 |
| Ex. III-8 | S | 0.2 | Co 20 | - | 4 | 76 | 161 | 0.62 | 91 |
| Ex. III-9 | F | 0.09 | Co 35 | Ag 0.8 | 4 | 66 | 188 | 0.89 | 92 |
| Ex. III-10 | S | 1.4 | Co 25 | Zn 3 | 2 | 69 | 178 | 0.86 | 87 |
| Ex. III-11 | P | 1.55 | Co 28 | Mn 3 | 4 | 68 | 172 | 0.84 | 92 |
| Comp. Ex. III-1 | S | 0.04 | Co 20 | - | 21 | 48.5 | 202 | 0.79 | 82 |
| Comp. Ex. III-2 | - | 0 | Co 20 | - | 15 | 56 | 142 | 0.65 | 79 |
| Comp. Ex. III-3 | S | 4.5 | Co 20 | - | 8 | 41 | 215 | 0.76 | 71 |

Test Example III-2

This test example illustrates a process for producing 1,6-hexanediamine by hydroamination of 1,6-hexanediol using the catalyst of the third type of embodiments of the present application.

100 mL of the catalysts obtained in the examples were respectively measured out, loaded into a fixed bed reactor, activated with hydrogen at 220 °C for 2 hours, then cooled to 170 °C, the pressure of the system was raised to 10 MPa using hydrogen, then ammonia was metered into the reaction system via a metering pump, preheated to 150 °C, then sent into the upper end of the reactor, heated and melted 1,6-hexanediol was fed into the upper end of the reactor via a metering pump, hydrogen was stably introduced via a gas mass flowmeter, wherein the molar ratio of hydrogen to ammonia and to 1,6-hexanediol was 3 : 10 : 1, the liquid phase volume space velocity of 1,6-hexanediol was 0.45 $h^{-1}$, a catalytic amination reaction was conducted in the reactor, at a reaction temperature of 190 °C, and a reaction pressure of 10 MPa, the reaction solution was sampled and analyzed at a reaction time of 240 hours, and the analysis results are shown in Table III-2.

The analysis of the sample was conducted by gas chromatography and was calibrated using a correction factor of a standard sample formulated.

The conversion and selectivity were calculated based on the molar content of each component in the reaction solution, and the calculation methods were the same as described in Test Example I-2.

Table III-2 Test results for the catalysts of the examples and comparative examples

| Catalyst | Composition of catalyst | Conversion , % | Product selectivity% | | | |
|---|---|---|---|---|---|---|
| | | | Hexanediamine | Hexamethyleneimine | Aminohexanol | Others |
| C-III-1 | $Co/Al_2O_3$ | 90.2 | 48.5 | 20.9 | 27.4 | 3.2 |
| C-III-2 | $Ni/Al_2O_3$ | 91.1 | 50.2 | 21.5 | 24.9 | 3.4 |
| C-III-3 | $Co/Al_2O_3-SiO_2$ | 89.3 | 46.1 | 18.9 | 32.2 | 2.8 |
| C-III-4 | $Co/Al_2O_3-ZSM-5$ | 87.9 | 47.2 | 21.5 | 27.9 | 3.4 |
| C-III-5 | $Co/ Al_2O_3$ | 92.2 | 46.3 | 20.8 | 29.8 | 3.1 |
| C-III-6 | $Ni/Al_2O_3$ | 88.4 | 48.7 | 19.3 | 28.6 | 3.4 |
| C-III-7 | $Co/Al_2O_3$ | 93.4 | 52.5 | 22.6 | 21.6 | 3.3 |
| C-III-8 | $Co/Al_2O_3$ | 93.1 | 49.2 | 20.5 | 27.1 | 3.2 |
| C-III-9 | $Co-Ag/Al_2O_3-SiO_2$ | 93.6 | 49.6 | 21.8 | 26 | 2.6 |
| C-III-10 | $Co-Zn/Al_2O_3$ | 90.5 | 47.8 | 20.6 | 28.7 | 2.9 |
| C-III-11 | $Co-Mn/Al_2O_3$ | 91.6 | 48.9 | 21.5 | 26.6 | 3 |
| D-III-1 | Co/Al2O3 | 66.3 | 32.6 | 25.2 | 31.0 | 11.2 |
| D-III-2 | Co/Al2O3 | 62.5 | 37.6 | 24.6 | 28.3 | 9.5 |
| D-III-3 | $Co/Al_2O_3$ | 74.8 | 34.9 | 28.1 | 24.7 | 12.3 |

The "others" in the table refers to amines containing 12 carbon atoms, which is difficult to diffuse out of the pore channel where a relative amount of said amine is produced, thereby causing a blocking of the pore channel, and in turn an increase of the amount of carbon deposition, so that the activity of the catalyst will be quickly reduced. After 240 hours of evaluation, the catalysts were discharged and subjected to thermogravimetric analysis, and the results show that the carbon deposition of the catalysts D-III-1, D-III-2 and D-III-3 is at least two times that of the catalysts C-III-1 to C-III-11.

After 1000 hours of evaluation, the results show that the reduction of the catalytic activity of the catalysts C-III-1 to C-III-11 is not obvious, namely the conversion and the selectivity of the catalysts determined after 1000 hours of continuous usage are not substantially reduced as compared those determined after 240 hours of usage. While the conversion of the catalysts D-III-1, D-III-2 and D-III-3 is respectively reduced by 29%, 32% and 35%, and the selectivity of the hexanediamine of those catalysts is respectively reduced by 15.1%, 12.8% and 16.3%.

Test Example III-3

This test example illustrates a process for producing ethylamine by hydroamination of ethanol using the catalyst of the third type of embodiments of the present application.

100 mL of the catalyst C-III-8 obtained in Example III-8 was measured out, loaded into a fixed bed reactor, activated with hydrogen at 220 °C for 2 hours, then cooled to 160 °C, the pressure of the system was raised to 1.6 MPa using hydrogen, then ammonia was metered into the reaction system via a metering pump, preheated to 135 °C, then sent to the upper end of the reactor, ethanol was fed into the upper end of the reactor via a metering pump, hydrogen was stably introduced via a gas mass flowmeter, wherein the molar ratio of hydrogen to ammonia and to ethanol was 3 : 8 : 1, the liquid phase volume space velocity of ethanol was 0.45 h$^{-1}$, a catalytic amination reaction was conducted in the reactor at a reaction temperature of 170 °C, and a reaction pressure of 1.6 MPa, and after the reaction became stable, the reaction solution was sampled and analyzed (the analysis conditions, and the methods for calculating the conversion and the selectivity are similar to those of Test Example III-2). The analysis results are shown in Table III-3.

Table III-3 Results of Test Example III-3

| Evaluation time | Conversion of ethanol, % | Selectively, % | | | |
|---|---|---|---|---|---|
| | | Monoethylamine | Diethylamine | Triethylamine | Others |
| 240h | 98.7 | 24.9 | 51.2 | 23.7 | 0.2 |
| 1000h | 98.5 | 25.1 | 51.1 | 23.6 | 0.2 |

Test Example III-4

This test example illustrates a process for producing isopropylamine by hydroamination of acetone using the catalyst of the third type of embodiments of the present application.

100 mL of the catalyst C-III-10 obtained in Example III-10 was measured out, loaded into a fixed bed reactor, activated with hydrogen at 200 °C for 2 hours, then cooled to 145 °C, the pressure of the system was raised to 1.5 MPa using hydrogen, then ammonia was metered into the reaction system via a metering pump, preheated to 110 °C and then sent into the upper end of the reactor, acetone was fed into the upper end of the reactor via a metering pump, hydrogen was stably introduced via a gas mass flowmeter, wherein the molar ratio of hydrogen to ammonia and to acetone was 3 : 6 : 1, the liquid phase volume space velocity of acetone was 0.4 h$^{-1}$, a catalytic amination reaction was conducted in the reactor, and after the reaction became stable, the reaction solution was sampled and analyzed (the analysis conditions, and the methods for calculating the conversion and the selectivity are similar to those of Test Example III-2). The analysis results are shown in Table III-4.

Table 4 Results of Test Examples III-4

| Evaluation time | Conversion of acetone, % | Selectively, % | | | |
|---|---|---|---|---|---|
| | | Monoisopropylamine | Diisopropylamine | Isopropyl alcohol | Others |
| 240h | 99.65 | 80.3 | 1.56 | 18.1 | 0.02 |
| 1000h | 99.59 | 80.6 | 1.35 | 18.0 | 0.02 |

Example series IV

The fourth type of embodiments of the present application are further illustrated in detail hereinbelow with reference to the examples of Example series IV. In the following example of Example series IV, the pseudo-boehmite powder had a ($Al_2O_3$) content on a dry basis of 70 wt%; the silica sol was purchased from Qingdao Ocean Chemical Co., Ltd, under a trade name of JN-40.

Example IV-1

Pseudo-boehmite powder (with a specific surface area of 288 $m^2/g$, a pore volume of 0.91 ml/g, and a doping element P present in the pseudo-boehmite powder in an amount of 0.22 g relative to 100g of the pseudo-boehmite powder used, calculated as $Al_2O_3$) prepared by aluminum sulfate method was kneaded with a dilute acid aqueous solution containing 5 vol% of nitric acid, extruded into strips with a diameter of 5 mm, cut to a length of 4 mm, dried at 120 °C for 8h, and then calcined at 550 °C for 6 h. 32.1 g of magnesium nitrate hexahydrate (analytically pure) was dissolved in water to obtain a 85 mL solution, and the aqueous magnesium nitrate solution was loaded onto 100g of the calcined product by spray impregnation, dried at 100 °C for 10 hours and then calcined at 820 °C for 5 hours to obtain a desired carrier.
186.5 g of cobalt nitrate hexahydrate (industrial grade, with a purity of 98%), 6.83g of zinc nitrate hexahydrate (analytically pure) and 1.16g of bismuth nitrate pentahydrate (analytically pure) were dissolved in water to obtain a 148 mL solution, and the solution was loaded onto the carrier obtained above by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3 h at 430 °C to obtain a catalyst C-IV-1.

Example IV-2

Pseudo-boehmite powder (with a specific surface area of 285 $m^2/g$, a pore volume of 0.93 ml/g, and a doping element B present in the pseudo-boehmite powder in an amount of 0.53 g relative to 100g of the pseudo-boehmite powder used, calculated as $Al_2O_3$) prepared by aluminum sulfate method was kneaded with a dilute acid aqueous solution containing 5vol% of nitric acid, extruded into granules of clover shape with a thickness of 3 mm, dried at 120 °C for 15 hours, and then calcined at 520 °C for 8 hours. 11.8g of calcium nitrate tetrahydrate (analytically pure) was dissolved in water to obtain a 91ml solution, and the aqueous calcium nitrate solution was loaded onto 100g of the calcined product by spray impregnation, dried at 110 °C for 10 hours and further calcined at 800 °C for 6 hours to obtain a desired carrier.
151.7 g of nickel nitrate hexahydrate (industrial grade, with a purity of 98%), 6.83g of zinc nitrate hexahydrate (analytically pure) and 1.16g of bismuth nitrate pentahydrate (analytically pure) were dissolved in water to obtain a 156 mL solution, the solution was loaded onto the carrier obtained above by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3 h at 430 °C to obtain a catalyst C-IV-2.

Example IV-3

Pseudo-boehmite (with a specific surface area of 285 $m^2/g$, a pore volume of 0.9 ml/g, and a doping element P present in the pseudo-boehmite powder in an amount of 0.23 g relative to 100g of the pseudo-boehmite powder used, calculated as $Al_2O_3$) prepared by aluminum sulfate method with a dilute acid aqueous solution containing 5 vol% of nitric acid, mixed uniformly, extruded into strips with a diameter of 5 mm, cut to a length of 4 mm, dried at 120 °C for 8h, and then calcined at 550 °C for 6 h. 32.1 g of magnesium nitrate hexahydrate (analytically pure) was dissolved in water to obtain a 85ml solution, and the aqueous magnesium nitrate solution was loaded onto 100g of the calcined product by spray impregnation, dried at 100 °C for 10 hours and then calcined at 820 °C for 5 hours to obtain a desired carrier.
45.4 g of cobalt nitrate hexahydrate (industrial grade, with a purity of 98%), 6.83g of zinc nitrate hexahydrate (analytically pure) and 1.16g of bismuth nitrate pentahydrate (analytically pure) were dissolved in water to obtain a 146mL solution, the solution was loaded onto the carrier obtained above by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3 h at 430 °C to obtain a catalyst C-IV-3.

Example IV-4

A carrier was prepared as described in Example IV-1, except that the pseudo-boehmite powder was uniformly mixed with a molecular sieve powder (ZSM-5, Catalyst Plant of Nankai University, $SiO_2/Al_2O_3$ =45 (molar ratio)), and the amount of the ZSM-5 molecular sieve powder was adjusted so that the content of alumina derived from the pseudo-boehmite in the carrier accounted for 85% of the total mass of the carrier.

176.4 g of cobalt nitrate hexahydrate (industrial grade, with a purity of 98%), 4.55g of zinc nitrate hexahydrate (analytically pure) and 2.32g of bismuth nitrate pentahydrate (analytically pure) were dissolved in water to obtain a 178mL solution, and the solution was loaded onto the carrier obtained above by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3 h at 430 °C to obtain a catalyst C-IV-4.

Example IV-5

Pseudo-boehmite powder (with a specific surface area of 258 m²/g, a pore volume of 0.65 ml/g, and a doping element P present in the pseudo-boehmite powder in an amount of 0.18 g relative to 100g of the pseudo-boehmite powder used, calculated as $Al_2O_3$) prepared by aluminum sulfate method was kneaded with a dilute acid aqueous solution containing 4.5 vol% of nitric acid, extruded into granules of clover shape with a thickness of 4 mm, dried at 100 °C for 18 hours, and then calcined at 560 °C for 5 hours. 11.8g of calcium nitrate tetrahydrate (analytically pure) was dissolved in water to obtain a 73mL solution, and the aqueous calcium nitrate solution was loaded onto 100g of the calcined product by spray impregnation, dried at 120 °C for 10 hours and further calcined at 970 °C for 6 hours to obtain a desired carrier.

75.6 g of cobalt nitrate hexahydrate (industrial grade, with a purity of 98%), 4.55g of zinc nitrate hexahydrate (analytically pure) and 2.32g of bismuth nitrate pentahydrate (analytically pure) were dissolved in water to obtain a 124 mL solution, and the solution was loaded onto the carrier obtained above by spray impregnation in two times, dried at 100 °C for 10 hours after each time of spray impregnation, then calcined at 388 °C for 4 hours, and then reduced with hydrogen while gradually increasing the temperature at a rate of 20 °C/h, and finally reduced at 420 °C for 4 h to obtain a catalyst C-IV-5.

Example IV-6

Pseudo-boehmite (with a specific surface area of 318 m²/g, a pore volume of 0.93 ml/g, and a doping element B present in the pseudo-boehmite powder in an amount of 3.66 g relative to 100g of the pseudo-boehmite powder used, calculated as $Al_2O_3$) prepared by aluminum sulfate method was kneaded with a dilute acid aqueous solution containing 5 vol% of nitric acid, silica sol (JN-40, available from Qingdao Ocean Chemical Co., Ltd.) was added during the kneading process, mixed uniformly, extruded into dentate spheres with a diameter of 3.5mm, dried for 4 h at 120 °C, and then calcined for 5h at 630 °C. 7.6g of barium nitrate (analytically pure) was dissolved in water to obtain a 88ml solution, and the aqueous barium nitrate solution was loaded onto 100g of the calcined product by spray impregnation, and then dried at 100 °C for 12 hours, and further calcined at 880 °C for 3 hours to obtain a desired carrier. The amount of silica sol used was adjusted so that the mass ratio of alumina to silica in the carrier was 4:1.

126.4 g of nickel nitrate hexahydrate (industrial grade, with a purity of 98%), 4.55g of zinc nitrate hexahydrate (analytically pure) and 2.32g of bismuth nitrate pentahydrate (analytically pure) were dissolved in water to obtain 160mL of a solution, and the solution was loaded onto the carrier obtained above by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3.5 h at 420 °C to obtain a catalyst C-IV-6.

Example IV-7

The carrier obtained in Example IV-1 was used.

201.6 g of cobalt nitrate hexahydrate (industrial grade, with a purity of 98%), 6.83g of zinc nitrate hexahydrate (analytically pure) and 1.16g of bismuth nitrate pentahydrate (analytically pure) were dissolved in water to obtain a 150mL solution, the solution was loaded onto the carrier obtained above by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3 h at 430 °C to obtain a catalyst C-IV-7.

Example IV-8

Pseudo-boehmite (with a specific surface area of 290 m$^2$/g, a pore volume of 0.78 ml/g, and a doping element S present in the pseudo-boehmite powder in an amount of 0.88 g relative to 100g of the pseudo-boehmite powder used, calculated as Al$_2$O$_3$) prepared by aluminum sulfate method was kneaded with a dilute acid aqueous solution containing 4.5 vol% of nitric acid, silica sol was added during the kneading process, mixed uniformly, extruded into granules of clover shape with a thickness of 4mm, drying for 20h at 80 °C, and then calcined for 7h at 550 °C. 17.7g of calcium nitrate tetrahydrate (analytically pure) was dissolved in water to obtain a 84ml solution, and the aqueous calcium nitrate solution was loaded onto 100g of the calcined product by spray impregnation, dried at 120 °C for 10 hours and further calcined at 900 °C for 4 hours to obtain a desired carrier. The amount of silica sol used was adjusted so that the molar ratio of alumina to silica in the carrier of 86 : 14.
100.8 g of cobalt nitrate hexahydrate (industrial grade, with a purity of 98%), 2.88g of zinc nitrate hexahydrate (analytically pure) and 3.48g of bismuth nitrate pentahydrate (analytically pure) were dissolved in water to obtain a 152 mL solution, and the solution was loaded onto the carrier obtained above by spray impregnation in two times, dried at 100 °C for 10 hours after each time of spray impregnation, then calcined at 400 °C for 4 hours, and then reduced with hydrogen while gradually increasing the temperature at a rate of 20 °C/h, and finally reduced at 430 °C for 4 h to obtain a catalyst C-IV-8.

Example IV-9

Pseudo-boehmite (with a specific surface area of 320 m$^2$/g, and a pore volume of 0.95 ml/g, and a doping element F present in the pseudo-boehmite powder in an amount of 0.82 g relative to 100g of the pseudo-boehmite powder used, calculated as Al$_2$O$_3$) prepared by aluminum sulfate method was kneaded with a dilute acid aqueous solution containing 5.2 vol% of nitric acid, silica sol was added during the kneading process, mixed uniformly, extruded into granules of clover shape with a thickness of 3mm, dried at 100 °C for 8h, and then calcined for 4h at 620 °C. 3.8g of barium nitrate (analytically pure) was dissolved in water to obtain a 83ml solution, and the aqueous barium nitrate solution was loaded onto 100g of the calcined product by spray impregnation, and then dried at 120 °C for 8 hours and calcined at 950 °C for 5 hours to obtain a desired carrier. The amount of silica sol used was adjusted so that the molar ratio of alumina to silica in the carrier of 89 : 11.
176.4 g of cobalt nitrate hexahydrate (industrial grade, with a purity of 98%), 2.88g of zinc nitrate hexahydrate (analytically pure) and 3.48g of bismuth nitrate pentahydrate (analytically pure) were dissolved in water to obtain 160mL of a solution, and the solution was loaded onto the carrier obtained above by spray impregnation in two times, dried at 110 °C for 8 hours after each time of spray impregnation, then calcined at 400 °C for 4 hours, and then reduced with hydrogen while gradually increasing the temperature at a rate of 20 °C/h, and finally reduced for 4.5 h at 420 °C to obtain a catalyst C-IV-9.

Example IV-10

Pseudo-boehmite powder (with a specific surface area of 291 m$^2$/g, a pore volume of 0.93 ml/g, and a doping element S present in the pseudo-boehmite powder in an amount of 0.95 g relative to 100g of the pseudo-boehmite powder used, calculated as Al$_2$O$_3$) prepared by aluminum sulfate method was kneaded with a dilute acid aqueous solution containing 4 vol% of nitric acid, then extruded into granules of clover shape with a thickness of 3.5 mm, dried at 100 °C for 8 hours, and then calcined at 600 °C for 5 hours. 42.7g of magnesium nitrate hexahydrate (analytically pure) was dissolved in 92ml of water, and the aqueous magnesium nitrate solution was loaded onto 100g of the calcined product by spray impregnation, dried at 120 °C for 8 hours and further calcined at 830 °C for 8 hours to obtain a desired carrier.
226.8 g of cobalt nitrate hexahydrate (industrial grade, with a purity of 98%), 2.88g of zinc nitrate hexahydrate (analytically pure) and 4.64g of bismuth nitrate pentahydrate (analytically pure) were dissolved in water to obtain a 172mL solution, and the solution was loaded onto the carrier obtained above by spray impregnation in two times, dried at 120 °C for 6 hours after each time of spray impregnation, then calcined at 400 °C for 4 hours, and then reduced with hydrogen while gradually increasing the temperature at a rate of 20 °C/h, and finally reduced for 4.5 h at 420 °C to obtain a catalyst C-IV-10.

Example IV-11

A catalyst C-IV-11 was prepared as described in Example IV-1, except that the pseudo-boehmite powder used was doped with element P, and the element P was present in the pseudo-boehmite powder in an amount of 4.3 g relative to 100g of the pseudo-boehmite powder used, calculated as Al$_2$O$_3$.

Example IV-12

A catalyst C-IV-12 was prepared as described in Example IV-2, except that the pseudo-boehmite powder used was free of the doping element, and had a specific surface area of 286 $m^2/g$ and a pore volume of 0.93 ml/g.

Example IV-13

A catalyst C-IV-13 was prepared as described in Example IV-2, except that the second calcining temperature was 1200 °C.

Comparative Example IV-1

A catalyst D-IV-1 was prepared as described in Example IV-5, except that the aqueous calcium nitrate solution was replaced with an equal volume of water and the solution used for the spray impregnation of the carrier was prepared by dissolving 151.7 g of nickel nitrate hexahydrate (industrial grade, with a purity of 98%) in water to obtain a 158mL solution, so that only nickel was loaded as an active metal component on the catalyst.

Comparative Example IV-2

A catalyst D-IV-2 was prepared as described in Example IV-3, except that the solution used for spray impregnation of the carrier was prepared by dissolving 45.4 g of cobalt nitrate hexahydrate (industrial grade, with a purity of 98%) and 1.16 g of bismuth nitrate pentahydrate (analytically pure) in water to obtain a 148 mL solution.

Comparative Example IV-3

A catalyst D-IV-3 was prepared as described in Example IV-1, except that the zinc nitrate was replaced with 8.5 g of copper nitrate trihydrate.

Test Example IV-1

The elemental composition of the carriers and the catalysts were analyzed by plasma emission spectroscopy, the content of the element (ion) other than the carrier was expressed by weight relative to 100g of the matrix; the carriers obtained above were characterized by probe adsorption spectrometry, BET nitrogen adsorption-desorption, and the results are shown in Table IV-1.

Table IV-1 Properties of the carriers of the examples and comparative examples

| Example No. | Doping element | | Relative content, g | | Proportion of pores having a pore diameter < 7 nm, % | Proportion of pores having a pore diameter of 7-27 nm, % | Specific surface area, m²/g | Pore volume, ml/g | L acid ratio, % |
|---|---|---|---|---|---|---|---|---|---|
| | Species | Relative content, g | Active metal component | Metal promoter | | | | | |
| Ex. IV-1 | P | 0.22 | Co 37 | Mg 3/Zn 1.5/Bi 0.5 | 2 | 75 | 165 | 0.74 | 87 |
| Ex. IV-2 | B | 0.53 | Ni 30 | Ca 2/Zn 1.5/Bi 0.5 | 3 | 72 | 163 | 0.78 | 89 |
| Ex. IV-3 | P | 0.23 | Co 9 | Mg 3/Zn 1.5/Bi 0.5 | 2 | 73 | 164 | 0.73 | 88 |
| Ex. IV-4 | P | 0.19 | Co 35 | Mg 6/Zn 1/Bi 1 | 4 | 76 | 172 | 0.79 | 87 |
| Ex. IV-5 | P | 0.18 | Co 15 | Ca 2/Zn 1/Bi 1 | 5 | 73 | 139 | 0.62 | 85 |
| Ex. IV-6 | B | 2.93 | Ni 25 | Ba 4/Zn 1/Bi 1 | 1 | 70 | 190 | 0.8 | 94 |
| Ex. IV-7 | P | 0.22 | Co 40 | Mg 3/Zn 1.5/Bi 0.5 | 2 | 74 | 165 | 0.75 | 87 |
| Ex. IV-8 | S | 0.76 | Co 20 | Ca 3/Zn 0.5/Bi 1.5 | 4 | 76 | 168 | 0.66 | 94 |
| Ex. IV-9 | F | 0.73 | Co 35 | Ba 2/Zn 0.5/Bi 1.5 | 2 | 74 | 191 | 0.81 | 93 |
| Ex. IV-10 | S | 0.95 | Co 45 | Mg 4/Zn 0.5/Bi 2 | 3 | 71 | 182 | 0.86 | 95 |
| Ex. IV-11 | P | 4.30 | Co 37 | Mg 3/Zn 1.5/Bi 0.5 | 3 | 73 | 163 | 0.73 | 92 |
| Ex. IV-12 | None | None | Ni 30 | Ca 2/Zn 1.5/Bi 0.5 | 4 | 72 | 166 | 0.79 | 85 |
| Ex. IV-13 | B | 0.53 | Ni 30 | Ca 2/Zn 1.5/Bi 0.5 | 10 | 63 | 128 | 0.54 | 87 |
| Comp. Ex. IV-1 | P | 0.18 | Ni 15 | None | 6 | 70 | 145 | 0.61 | 78 |

(continued)

| Example No. | Doping element | | Relative content, g | | Proportion of pores having a pore diameter < 7 nm, % | Proportion of pores having a pore diameter of 7-27 nm, % | Specific surface area, m$^2$/g | Pore volume, ml/g | L acid ratio, % |
|---|---|---|---|---|---|---|---|---|---|
| | Species | Relative content, g | Active metal component | Metal promoter | | | | | |
| Comp. Ex. IV-2 | P | 0.23 | Co 9 | Mg 3/Bi 0.5 | 3 | 73 | 151 | 0.70 | 65 |
| Comp. Ex. IV-3 | P | 0.22 | Co 37 | Mg 3/Cu 3/Bi 0.5 | 4 | 80 | 165 | 0.74 | 74 |

Test Example IV-2

This test example illustrates a process for producing 1,6-hexanediamine by hydroamination of 1,6-hexanediol using the catalyst of the fourth type of embodiments of the present application.

100 mL of the catalysts obtained in the examples and comparative examples were respectively measured out, loaded into a fixed bed reactor, activated with hydrogen at 220 °C for 2 hours, then cooled to 172 °C, the pressure of the system was raised to 12.5 MPa using hydrogen, then ammonia was metered into the reaction system via a metering pump, preheated to 150 °C and sent to the upper end of the reactor, then heated and melted 1,6-hexanediol was fed into the upper end of the reactor via a metering pump, hydrogen was stably introduced via a gas mass flowmeter, wherein the molar ratio of hydrogen to ammonia and to 1,6-hexanediol was 3 : 18 : 1, the liquid phase volume space velocity of 1,6-hexanediol was 0.5 h$^{-1}$, a catalytic amination reaction was conducted in the reactor at a reaction temperature of 195 °C, and a reaction pressure of 12.5 MPa, and after the reaction became stable, the reaction solution was sampled and analyzed. The analysis results are shown in Table IV-2.

The analysis of the sample was conducted by gas chromatography and was calibrated using a correction factor of a standard sample formulated.

The conversion and selectivity were calculated based on the molar content of each component in the reaction solution, and the calculation methods were the same as described in Test Example I-2.

Table IV-2 Test results for the catalysts of the examples and comparative examples

| Catalyst | Composition of catalyst | Conversion, % | Selectively, % | | | |
|---|---|---|---|---|---|---|
| | | | Hexanediamine | Hexamethyleneimine | Aminohexanol | Others |
| C-IV-1 | Co-Mg-Zn-Bi/Al$_2$O$_3$ | 96 | 68.8 | 15.2 | 13.4 | 2.6 |
| C-IV-2 | Ni-Ca-Zn-Bi/Al$_2$O$_3$ | 95 | 66.2 | 17 | 14 | 2.8 |
| C-IV-3 | Co-Mg-Zn-Bi/Al$_2$O$_3$ | 90 | 63.5 | 18.5 | 15 | 3 |
| C-IV-4 | Co-Mg-Zn-Bi/Al$_2$O$_3$-ZSM-5 | 93 | 58.5 | 18.5 | 19.6 | 3.4 |
| C-IV-5 | Co-Ca-Zn-Bi/ Al$_2$O$_3$ | 94 | 63.9 | 17.8 | 15.2 | 3.1 |
| C-IV-6 | Ni-Ba-Zn-Bi/Al$_2$O$_3$-SiO$_2$ | 93 | 62.7 | 18.1 | 16.4 | 2.8 |
| C-IV-7 | Co-Mg-Zn-Bi/Al$_2$O$_3$ | 90 | 64.3 | 19.2 | 13.6 | 2.9 |
| C-IV-8 | Co-Ca-Zn-Bi/Al$_2$O$_3$-SiO$_2$ | 94 | 63.7 | 17.6 | 15.5 | 3.2 |
| C-IV-9 | Co-Ba-Zn-Bi/Al$_2$O$_3$-SiO$_2$ | 95 | 64.3 | 16.8 | 16.1 | 2.8 |
| C-IV-10 | Co-Mg-Zn-Bi/Al$_2$O$_3$ | 90 | 60.6 | 19.7 | 16.1 | 3.6 |
| C-IV-11 | Co-Mg-Zn-Bi/Al$_2$O$_3$ | 89 | 57.2 | 20.3 | 18 | 4.5 |
| C-IV-12 | Ni-Ca-Zn-Bi/Al$_2$O$_3$ | 88 | 56.1 | 21.3 | 18.8 | 3.8 |
| C-IV-13 | Ni-Ca-Zn-Bi/Al$_2$O$_3$ | 88 | 55.7 | 21.9 | 18.3 | 4.1 |
| D-IV-1 | Ni /Al$_2$O$_3$ | 84 | 48.2 | 26.2 | 20.5 | 5.1 |
| D-IV-2 | Co-Mg-Bi/Al$_2$O$_3$ | 82 | 49.2 | 24.8 | 21.1 | 4.9 |
| D-IV-3 | Co-Mg-Cu-Bi/Al$_2$O$_3$ | 75 | 38.9 | 27.8 | 25.5 | 7.8 |

As seen from the above Table, the catalysts D-IV-1 to D-IV-3 show a lower conversion under the same process conditions, indicating a lower activity than the catalysts C-IV-1 to C-IV-13 of the present application; and as compared to the catalysts C-IV-1 to C-IV-13, the selectivity of hexanediamine of the catalysts D-IV-1 to D-IV-3 is lower while the selectivity of other components is higher, indicating that the reaction material is not easy to be desorbed from the catalysts D-IV-1 to D-IV-3, and thus undergoes a further reaction to generate other byproducts.

Test Example IV-3

This test example illustrates a process for producing ethylamine by hydroamination of ethanol using the catalyst according to the fourth type of embodiments of the present application.

100 mL of the catalyst C-IV-3 obtained in Example IV-3 was measured out, loaded into a fixed bed reactor, activated with hydrogen at 220 °C for 2 hours, then cooled to 173 °C, the pressure of the system was raised to 1.65 MPa using hydrogen, then ammonia was metered into the reaction system via a metering pump, preheated to 110 °C, then sent to the upper end of the reactor, ethanol was fed into the upper end of the reactor via a metering pump, hydrogen was stably introduced via a gas mass flowmeter, wherein the molar ratio of hydrogen to ammonia and to ethanol was 3 : 5 : 1, the liquid phase volume space velocity of ethanol was 0.5 $h^{-1}$, a catalytic amination reaction was conducted in the reactor at a reaction temperature of 178 °C, and a reaction pressure of 1.6 MPa, and after the reaction became stable, the reaction solution was sampled and analyzed (the analysis conditions, and the methods for calculating the conversion and the selectivity are similar to those of Test Example IV-2). The analysis results are shown in Table IV-3.

Table IV-3 Results of Test Example IV-3

| Continuous reaction time | Conversion of ethanol | Selectively, % | | | |
|---|---|---|---|---|---|
| | | Monoethylamine | Diethylamine | Triethylamine | Others |
| 20h | 98.83 | 28.1 | 46.8 | 24.4 | 0.7 |
| 500h | 98.81 | 28.1 | 47.2 | 24.1 | 0.6 |

Test Example IV-4

The catalysts C-IV-2, C-IV-9, D-IV-1, D-IV-2, D-IV-3 were loaded into fixed bed reactors, respectively, under the same conditions as in Test Example IV-2, with an only change that the reaction time was prolonged, and a test was conducted for 500 hours. An analysis and comparison were conducted on the reaction solution at a reaction time of 20 hours (the analysis conditions, and the methods for calculating the conversion and the selectivity are the same as in Test Example IV-2), and the reaction solution after 500 hours of reaction (the analysis conditions, and the methods for calculating the conversion and the selectivity are the same as in Test Example IV-2). The analysis results are shown in Table IV-4.

Table IV-4 Results of Test Example IV-4

| Catalyst | | Composition of catalyst | Convers ion, % | Selectively, % | | | |
|---|---|---|---|---|---|---|---|
| | | | | Hexanediamine | Hexamethyleneimine | Aminohexanol | Others |
| 20 h of reacti on | C-IV-2 | Ni-Ca-Zn-Bi/Al$_2$O$_3$ | 95 | 66.2 | 17 | 14 | 2.8 |
| | C-IV-9 | Co-Ba-Zn-Bi/Al$_2$O$_3$-Si O$_2$ | 95 | 64.3 | 16.8 | 16.1 | 2.8 |
| | D-IV-1 | Ni/Al$_2$O$_3$ | 84 | 48.2 | 26.2 | 20.5 | 5.1 |
| | D-IV-2 | Co-Mg-Bi/Al$_2$O$_3$ | 82 | 49.2 | 24.8 | 21.1 | 4.9 |
| | D-IV-3 | Co-Mg-Cu-Bi/Al$_2$O$_3$ | 75 | 38.9 | 27.8 | 25.5 | 7.8 |
| 500h of reacti on | C-IV-2 | Ni-Ca-Zn-Bi/Al$_2$O$_3$ | 94 | 66.1 | 16.9 | 14.1 | 2.9 |
| | C-IV-9 | Co-Ba-Zn-Bi/Al$_2$O$_3$-Si O$_2$ | 94 | 64 | 16.5 | 16.5 | 3 |
| | D-IV-1 | Ni /Al$_2$O$_3$ | 71 | 41.2 | 24.1 | 27.9 | 6.8 |
| | D-IV-2 | Co-Mg-Bi/Al$_2$O$_3$ | 63 | 42.3 | 22.6 | 27.3 | 7.8 |
| | D-IV-3 | Co-Mg-Cu-Bi/Al$_2$O$_3$ | 49 | 32.6 | 20.9 | 36.2 | 10.3 |

After 500 hours of evaluation, the activity and selectivity of the catalysts C-IV-2 and C-IV-9 are not substantially changed, while the activity and selectivity of the comparative catalysts D-IV-1, D-IV-2 and D-IV-3 are obviously reduced. The specific surface area, pore volume and carbon deposition of each catalyst are characterized, and it is found that the specific surface area and pore volume of the catalysts D-IV-1, D-IV-2 and D-IV-3 are reduced greatly, while the specific surface area and pore volume of the catalysts C-IV-2 and C-IV-9 are substantially unchanged (the reduction value is less than 2 %), and the carbon deposition of the catalysts D-IV-1, D-IV-2 and D-IV-3 is more than twice that of the catalysts C-IV-2 and C-IV-9.

Test Example IV-5

This test example illustrates a process for producing hexanediamine from a mixture of 1,6-hexanediol, hexamethyleneimine and aminohexanol using the catalyst according to the fourth type of embodiments of the present application.

100 mL of the catalyst C-IV-3 obtained in Example IV-3 was weighed and loaded into a fixed bed reactor, activated with hydrogen at 220 °C for 2 hours, then cooled to 175 °C, the pressure of the system was raised to 14 MPa using hydrogen, then ammonia was metered into the reaction system via a metering pump, preheated to 150 °C and sent to the upper end of the reactor, a mixed solution comprising 53 wt% of 1,6-hexanediol, 30 wt% of hexamethyleneimine and 17 wt% of 6-amino-1-hexanol was fed into the upper end of the reactor via a metering pump, hydrogen was stably introduced via a gas mass flowmeter, wherein the molar ratio of hydrogen to ammonia and to the total amount of the three substances in the mixed solution was 3 : 10 : 1, the liquid phase volumetric space velocity of the mixed solution was 0.5 h$^{-1}$, a catalytic amination reaction was conducted in the reactor, at a reaction temperature of 180 °C, and a reaction pressure of 14 MPa, and after the reaction became stable, the reaction solution was sampled and analyzed (the analysis conditions, and the methods for calculating the conversion and the selectivity are the same as in Test Example IV-2). The analysis results are shown in Table IV-5.

Table IV-5 Results of Test Example IV-5

| Continuous reaction time | 1,6-hexane diol | Selectively, % | | | |
|---|---|---|---|---|---|
| | Conversion , % | 1,6-hexanediamine | Hexamethyleneimine | 6-amino-1-hexanol | Others |
| 20 h | 98.6 | 98.1 | 0.8 | 0.4 | 0.7 |
| 500h | 98.3 | 98.0 | 0.8 | 0.4 | 0.8 |

The present application is illustrated in detail hereinabove with reference to preferred embodiments, but is not intended to be limited to those embodiments. Various modifications may be made following the inventive concept of the present application, and these modifications shall be within the scope of the present application.

It should be noted that the various technical features described in the above embodiments may be combined in any suitable manner without contradiction, and in order to avoid unnecessary repetition, various possible combinations are not described in the present application, but such combinations shall also be within the scope of the present application.

In addition, the various embodiments of the present application can be arbitrarily combined as long as the combination does not depart from the spirit of the present application, and such combined embodiments should be considered as the disclosure of the present application.

**Claims**

1. A catalyst useful for producing organic amines by catalytic amination, comprising an inorganic porous carrier containing aluminium and/or silicon and an active metal component supported on the carrier, wherein the active metal component comprises at least one metal selected from the group consisting of Group VIII and Group IB metals, preferably selected from cobalt, nickel, palladium, copper or a combination thereof, more preferably selected from cobalt, nickel or a combination thereof, wherein the carrier has an L acid content of 85% or more, preferably 88% or more, more preferably 90% or more, especially preferably 92% or more, relative to the total of the L acid and B acid contents.

2. The catalyst according to claim 1, wherein the carrier comprises a matrix and a doping element, wherein the matrix is selected from alumina, silica, molecular sieves, diatomite, aluminosilicates, or combinations thereof, and

the doping element is a non-metallic element, preferably selected from the group consisting of Group IIIA non-metallic elements, Group VA non-metallic elements, Group VIA non-metallic elements, Group VIIA non-metallic elements and combinations thereof, excluding chlorine, more preferably selected from boron, fluorine, phosphorus, sulfur, selenium or a combination thereof;

preferably, the doping element in the carrier was derived from a non-metallic acid radical ion, the non-metallic acid radical ion is preferably selected from the group consisting of borate ion, fluoride ion, phosphate ion, sulfate ion, selenate ion and combinations thereof.

3. The catalyst according to claim 1 or 2, wherein the carrier has at least one of the following characteristics:

the proportion of the pore volume of pores having a pore diameter in a range of 7-27 nm to the pore volume of the carrier is greater than 65%, preferably 70 to 90%, and the proportion of the pore volume of pores having a pore diameter less than 7 nm to the pore volume of the carrier is 0 to 10%, preferably 0 to 8%;

the proportion of the pore volume of pores having a pore diameter of less than 7.5nm to the pore volume of the carrier is less than 20%, preferably 5-17%, the proportion of the pore volume of pores having a pore diameter of less than 9nm to the pore volume of the carrier is less than 40%, the proportion of the pore volume of pores having a pore diameter of greater than 27nm to the pore volume of the carrier is less than 5%, preferably 0.5-5%, preferably, the proportion of the pore volume of pores having a pore diameter of more than or equal to 7.5nm and less than 9nm to the pore volume of the carrier is 5-17%, and the proportion of the pore volume of pores having a pore diameter of more than or equal to 9nm and less than or equal to 27nm to the pore volume of the carrier is 61-89.5%;

the carrier has an ammonia adsorption capacity of 0.25-0.65 mmol/g, preferably 0.3-0.6 mmol/g, and more preferably 0.3-0.5 mmol/g;

the content of alumina in the carrier was 65 wt% or more, preferably 70 wt% or more, more preferably 75 wt% or more, based on the total amount of the matrix;

the content of the doping element is 0.05 to 6 wt%, preferably 0.05 to 5 wt%, more preferably 0.05 to 4.5 wt%, and particularly preferably 0.07 to 4 wt%, relative to the total amount of the matrix;

the carrier has a specific surface area of 100-220 $m^2$/g, preferably 105-210 $m^2$/g, more preferably 110-210 $m^2$/g, and particularly preferably 120-210 $m^2$/g;

the carrier has a pore volume of 0.4-1.1 ml/g, preferably 0.43-1.1 ml/g, more preferably 0.45-1.1 ml/g, and particularly preferably 0.45-1 ml/g; and

the isoelectric point of the carrier is 3-6, preferably 3.5-5.5.

4. The catalyst according to any one of claims 1-3, wherein the active metal component is present in an amount of 5 to 45 g, preferably 8 to 44 g, more preferably 10 to 38 g, particularly preferably 15 to 37 g, per 100g of the matrix, preferably the active metal component has a grain size of less than 10nm, more preferably 3 to 8 nm.

5. The catalyst according to any one of claims 1-4, further comprising a metal promoter supported on the carrier, and the metal promoter comprises at least one metal selected from the group consisting of Group VIB, Group VIIB, Group IB, Group IIB, and lanthanide series metals, preferably at least one metal selected from Cr, Mo, W, Mn, Re, Cu, Ag, Au, Zn, La, and Ce;

preferably, the metal promoter is present in an amount of 0 to 10g, preferably 0.1 to 10g, more preferably 0.5 to 8 g, per 100 g of the matrix.

6. The catalyst according to claim 5, wherein:

the metal promoter comprises a combination of at least one Group VIIB metal and at least one Group IB metal, wherein the weight ratio of the Group VIIB metal to the Group IB metal, calculated as metal element, is 0.05-15 : 1, preferably 0.1-12 : 1; or alternatively

the metal promoter comprises a combination of at least one Group VIIB metal and at least one Group IIB metal, wherein the weight ratio of the Group VIIB metal to the Group IIB metal, calculated as metal element, is 0.2-20 : 1, preferably 0.3-6 : 1; or alternatively

the metal promoter comprises a combination of at least one Group VIB metal, at least one Group IB metal and at least one Group IIB metal, wherein the weight ratio of the Group VIB metal to the Group IB metal and to the Group IIB metal, calculated as metal element, is 0.1-10 : 0.1-10 : 1, preferably 0.2-8 : 0.2-8 : 1,

preferably, the Group VIIB metal is selected from manganese, rhenium, or a combination thereof, the Group IB metal is selected from copper, silver, gold, or a combination thereof, the Group IB metal is zinc, and/or the Group VIB metal is selected from molybdenum, tungsten, or a combination thereof.

7. The catalyst according to any one of claims 1-4, further comprising a metal promoter supported on the carrier, wherein the metal promoter is a combination of at least one Group IIA metal, at least one Group IIB metal and at least one Group VA metal,

preferably, the metal promoter is present in an amount of 0.1 to 10g, preferably 0.5 to 6g, per 100 g of the matrix; preferably, the weight ratio of the Group IIA metal to the Group IIB metal and to the Group VA metal in the metal promoter is 0.1-10 : 0.1-10 : 1, preferably 0.2-8 : 0.2-8 : 1;
preferably, the Group IIA metal is selected from magnesium, calcium, barium, or a combination thereof, the Group IIB metal is zinc, and/or the Group VA metal is bismuth.

8. A method for producing the catalyst according to any one of claims 1-7, comprising the steps of:

1) providing an inorganic porous carrier containing aluminum and/or silicon, which carrier has an L acid content of 85% or more, preferably 88% or more, more preferably 90% or more, particularly preferably 92% or more, relative to the total of the L acid and B acid contents;
2) loading the active metal component and optionally the metal promoter on the carrier; and
3) carrying out a heat treatment and optionally a reduction treatment on the material obtained in step 2) to obtain the catalyst,

preferably, the heat treatment comprises calcining, or a combination of drying and calcining.

9. The method according to claim 8, wherein said "providing an inorganic porous carrier containing aluminum and/or silicon" of step 1) comprises subjecting a mixture comprising a doping element and a matrix or a precursor thereof to shaping, drying and calcining sequentially to obtain the carrier,

wherein the matrix is selected from alumina, silica, molecular sieves, diatomite, aluminosilicates or combinations thereof, preferably, the alumina precursor is pseudo-boehmite having a specific surface area of 250-400 $m^2$/g, preferably 255-360 $m^2$/g, more preferably 255-340 $m^2$/g, particularly preferably 260-330 $m^2$/g and a pore volume of 0.5-1.3 ml/g, preferably 0.75-1.25 ml/g, more preferably 0.78-1.2 ml/g, particularly preferably 0.78-1.1 ml/g; the doping element is a non-metallic element, preferably selected from the group consisting of Group IIIA non-metallic elements, Group VA non-metallic elements, Group VIA non-metallic elements, Group VIIA non-metallic elements and combinations thereof excluding chlorine, preferably selected from boron, fluorine, phosphorus, sulfur, selenium or a combination thereof,
preferably, the drying conditions of step 1) include: a temperature of 80-150 °C, and a drying time of 6-20 h; and preferably, the calcining conditions of step 1) include: a temperature of 500-1120 °C, such as 500-650 °C, preferably 700-1100 °C, more preferably 800-1050 °C, and a calcining time of 2-20 h.

10. The method according to claim 9, wherein the doping element is provided using a carrier modifier comprising at least one compound capable of providing a non-metallic acid radical ion, such as an inorganic acid and/or an inorganic salt comprising a non-metallic acid radical, preferably the non-metallic acid radical ion is selected from borate ion, fluoride ion, phosphate ion, sulfate ion, selenate ion or a combination thereof;
preferably, the carrier modifier is selected from boric acid, nickel borate, cobalt borate, potassium borate, ammonium borate, magnesium borate, potassium fluoride, magnesium fluoride, cobalt fluoride, nickel fluoride, hydrofluoric acid, ammonium fluoride, phosphoric acid, aluminum phosphate, tripotassium phosphate, potassium dihydrogen phosphate, potassium hydrogen phosphate, magnesium phosphate, calcium phosphate, ammonium phosphate, sulfuric acid, cobalt sulfate, nickel sulfate, aluminum sulfate, calcium sulfate, potassium sulfate, magnesium sulfate, strontium phosphate, strontium sulfate, selenic acid, or combinations thereof.

11. The method according to any one of claims 8-10, wherein the loading of step 2) comprises impregnating the carrier with a solution comprising a precursor of the active metal component and optionally a precursor of the metal promoter, preferably the impregnation solution has a pH in a range of 3.5-5.5.

12. A process for producing organic amines, comprising: contacting an amination raw material and an amination reagent with the catalyst according to any one of claims 1-7 in the presence of hydrogen for amination reaction to obtain an organic amine,

wherein the amination raw material is selected from alcohols, ketones, alcohol amines, aldehydes or combinations thereof, preferably selected from C2-20 alcohols, C3-20 ketones, C2-20 alcohol amines, C2-20 aldehydes

EP 4 238 647 A1

or combinations thereof, more preferably selected from ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanal, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexandial, 1,8-octanediol, 1,8-octanedial, 1,12-dodecanediol, 1,12-dodecanedialdehyde, ethanolamine, propanolamine, isopropanolamine, 6-aminohexanol, diethanolamine, diisopropanolamine, dimethylethanolamine, acetone, ethylene glycol, 1,3-propanediol, or combinations thereof;

the amination reagent is selected from ammonia, primary amines, secondary amines or a combination thereof, preferably selected from ammonia, C1-12 primary amines, C1-12 secondary amines or a combination thereof, more preferably selected from ammonia, monomethylamine, dimethylamine, methyl ethyl amine, monoethylamine, diethylamine or a combination thereof.

13. The process according to claim 12, wherein the amination conditions include: a molar ratio of hydrogen to the amination reagent and to the amination raw material of 1-6 : 2-35 : 1, preferably 1-6 : 2-33 : 1, more preferably 1-5 : 3-33 : 1, a temperature of 105-230 °C, preferably 110-220 °C, more preferably 110-210 °C, a pressure of 0.7-25 MPa, preferably 1-25 MPa, more preferably 1-22 MPa, particularly preferably 1-17 MPa, and a liquid phase volume space velocity of the amination raw material of 0.06-1 $m^3/(m^3 \cdot h)$.

14. The process according to claim 13, wherein:

where the amination raw material is a monohydric alcohol, the amination conditions include: a molar ratio of hydrogen to the amination reagent and to the amination raw material of preferably 1-4 : 2-9 : 1, more preferably 1-4 : 2-8 : 1, a temperature of 130-210 °C, preferably 130-2080 °C, more preferably 130-200 °C, a pressure of 0.8-3.5 MPa, preferably 1-2.5 MPa, and a liquid phase volume space velocity of the amination raw material of 0. 1-0.8 $m^3/(m^3 \cdot h)$;

where the amination raw material is a ketone or an aldehyde, the amination conditions include: a molar ratio of hydrogen to the amination reagent and to the amination raw material of 1-4 : 2-6 : 1, preferably 1-4 : 2-5 : 1, a temperature of 105-180 °C, preferably 110-170 °C, more preferably 110-160 °C, a pressure of 0.7-2.5 MPa, preferably 1-2.5 MPa, more preferably 1-2 MPa, and a liquid phase volume space velocity of the amination raw material of 0.1-1 $m^3/(m^3 \ h)$, preferably 0.1-0.8 $m^3/(m^3 \ h)$;

where the amination raw material is an alcohol amine, the amination conditions include: a molar ratio of hydrogen to the amination reagent and to the amination raw material of 1-4 : 3-23 : 1, preferably 1-4 : 3-20 : 1, more preferably 1-4 : 3-10 : 1, a temperature of 130-200 °C, a pressure of 1-16 MPa, preferably 1-13 MPa, more preferably 1-11 MPa, and a liquid phase volume space velocity of the amination raw material of 0.06-0.8 $m^3/(m^3 \ h)$;

where the amination raw material is a dihydric alcohol, the amination conditions include: a molar ratio of hydrogen to the amination reagent and to the amination raw material of 0.3-5 : 2-35 : 1, preferably 1-4 : 3-35 : 1, more preferably 1-4 : 3-33 : 1, particularly preferably 1-4 : 3-32 : 1, a temperature of 130-230 °C, preferably 130-220 °C, more preferably 130-210 °C, a pressure of 1-25 MPa, preferably 1-22 MPa, more preferably 1-17 MPa, and a liquid phase volume space velocity of the amination raw material of 0.1-0.9 $m^3/(m^3 \ h)$, preferably 0.1-0.8 $m^3/(m^3 \ h)$; or

where the amination raw material is a mixture of 1,6-hexanediol, hexamethyleneimine and 6-amino-1-hexanol, the amination conditions include: a molar ratio of hydrogen to the amination reagent and to the amination raw material of 0.3-4 : 3-35 : 1, preferably 1-4 : 3-33 : 1, more preferably 1-4 : 3-32 : 1, a temperature of 130-230 °C, preferably 130-220 °C, more preferably 130-210 °C, a pressure of 1-22 MPa, preferably 1-17 MPa, and a liquid phase volume space velocity of the amination raw material of 0.1-0.9 $m^3/(m^3 \ h)$, preferably 0.1-0.8 $m^3/(m^3 \ h)$.

## INTERNATIONAL SEARCH REPORT

| | International application No. |
|---|---|
| | **PCT/CN2021/126326** |

**A. CLASSIFICATION OF SUBJECT MATTER**

B01J 23/75(2006.01)i; B01J 23/755(2006.01)i; B01J 23/78(2006.01)i; B01J 23/80(2006.01)i; B01J 23/83(2006.01)i; C07C 211/03(2006.01)i; C07C 209/04(2006.01)i

According to International Patent Classification (IPC) or to both national classification and IPC

**B. FIELDS SEARCHED**

Minimum documentation searched (classification system followed by classification symbols)

B01J23/-; C07C211/-; C07C209/-

Documentation searched other than minimum documentation to the extent that such documents are included in the fields searched

Electronic data base consulted during the international search (name of data base and, where practicable, search terms used)

CNTXT, CNKI, VEN, WEB OF SCIENCE: 胺化, 氨化, 催化剂, 路易斯酸, 氧化铝, 磷酸, 硼酸, 硫酸, 氢氟酸, amination, ammonification, catalyst+, lewis acid, Al2O3, alumina, phosphoric acid, boric acid, sulphuric acid, hydrofluoric acid

**C. DOCUMENTS CONSIDERED TO BE RELEVANT**

| Category* | Citation of document, with indication, where appropriate, of the relevant passages | Relevant to claim No. |
|---|---|---|
| Y | 吕养心 等 (LYU, Yanxin et al.). "CuO/NiO/γ-Al2O3催化剂合成环己胺 (Synthesis of Cyclohexylamine with CuO/NiO/γ-Al2O3 Catalyst)" 化学反应工程与工艺 (Chemical Reaction Engineering and Technology), Vol. 20, No. 2, 30 June 2004 (2004-06-30), sections 1.1.1, 1.2 and abstract | 1-5, 8-14 |
| Y | 马骏 等 (MA, Jun et al.). "β沸石在乙醇胺化反应中的催化行为 (Catalytic Behaviour of Amination of C2H5OH with NH3 over β Zeolite)" 石油化工 (Petrochemical Technology), Vol. 22, No. 9, 30 September 1993 (1993-09-30), section 3.1 and abstract | 1-5, 8-14 |
| A | CN 101569862 A (CHINA PETROLEUM & CHEMICAL CORPORATION et al.) 04 November 2009 (2009-11-04) entire document | 1-14 |
| A | KR 20120103323 A (KOREA RESEARCH INSTITUTE OF CHEMICAL TECHNOLOGY et al.) 19 September 2012 (2012-09-19) entire document | 1-14 |

☐ Further documents are listed in the continuation of Box C.　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 January 2022** | **26 January 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

International application No.

**PCT/CN2021/126326**

| Patent document cited in search report | | | Publication date (day/month/year) | Patent family member(s) | | | Publication date (day/month/year) |
|---|---|---|---|---|---|---|---|
| CN | 101569862 | A | 04 November 2009 | CN | 101569862 | B | 30 November 2011 |
| KR | 20120103323 | A | 19 September 2012 | KR | 101336975 | B1 | 04 December 2013 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102658162 A

- GB 6609352009 T